# EUROPEAN PATENT APPLICATION

(11) **EP 2 522 216 A1**
(43) Date of publication of application: **14.11.2012**
(21) Application number: 12163044.6
(22) Date of filing: 12.02.2004
(51) Int. Cl.: A01H 5/00, A01K 1/10, A23D 9/013, C12N 15/82

(54) **Production of very long chain polyunsaturated fatty acids in oilseed plants**

(30) Priority: 12.02.2003 US 446941 P
(62) Divisional of application: 04710743.8
(71) Applicant: E. I. du Pont de Nemours and Company, Wilmington, DE 19898 (US)
(72) Inventor: Kinney, Anthony, J., Wilmington, DE 19809 (US); Cahoon, Edgar, B., Lincoln, NE 68506 (US); Damude, Howard, G., Hockessin, DE 19707 (US); Hitz, William, D., Wilmington, DE 19807 (US); Kolar, Charles, W., St. Louis, MO 63128 (US); Liu, Zhan-Bin, West Chester, PA 19382 (US)
(74) Representative: Dzieglewska, Hanna Eva

(57) **Abstract**

Oilseed plants which have been transformed to produce very long chain polyunsaturated fatty acids, recombinant constructs used in such transformations, methods for producing such fatty acids in a plant are described and uses of oils and seeds obtained from such transformed plants in a variety of food and feed applications are described.

SEQUENCE LISTING

<110>  E.I. du Pont de Nemours and Co.

<120>  Production of Very Long Chain Polyunsaturated Fatty Acids in Oilseed
Plants

<130>  27.13.88893/01

<140>
<141>  2004-02-12

<150>  US 60/446,941
<151>  2003-02-12

<160>  98

<170>  PatentIn version 3.2

<210>  1
<211>  24
<212>  DNA
<213>  Artificial Sequence

<220>
<223>  synthetic oligonucleotide

<400>  1
gccccccatc ctttgaaagc ctgt                                              24

<210>  2
<211>  24
<212>  DNA
<213>  Artificial Sequence

<220>
<223>  synthetic oligonucleotide

<400>  2
gccccccatc ctttgaaagc ctgt                                              24

<210>  3
<211>  2012
<212>  DNA
<213>  Glycine max

<400>  3
atcttaggcc cttgattata tggtgtttag atggattcac atgcaagttt ttatttcaat      60
cccttttcct ttgaataact gaccaagaac aacaagaaaa aaaaaaaaag aaaaggatca     120
ttttgaaagg atatttttcg ctcctattca aatactgtat ttttaccaaa aaaactgtat     180
ttttcctaca ctctcaagct ttgtttttcg cttcgactct catgatttcc ttcatatgcc     240
aatcactcta tttataaatg gcataaggta gtgtgaacaa ttgcaaagct tgtcatcaaa     300
agcttgcaat gtacaaatta atgtttttca tgcctttcaa aattatctgc accccctagc     360
tattaatcta acatctaagt aaggctagtg aattttttcg aatagtcatg cagtgcatta     420
atttccccgt gactattttg gctttgactc caacactggc cccgtacatc cgtccctcat     480
tacatgaaaa gaaatattgt ttatattctt aattaaaaat attgtccctt ctaaattttc     540
atatagttaa ttattatatt acttttttct ctattctatt agttctattt tcaaattatt     600
atttatgcat atgtaaagta cattatattt ttgctatata cttaaatatt tctaaattat     660
taaaaaaaga ctgatatgaa aaatttattc tttttaaagc tatatcattt tatatatact     720
ttttcttttc ttttctttca ttttctattc aatttaataa gaaataaatt ttgtaaattt     780
ttatttatca atttataaaa atattttact ttatatgttt tttcacattt ttgttaaaca     840
aatcatatca ttatgattga aagagaggaa attgacagtg agtaataagt gatgagaaaa     900
aaatgtgtta tttcctaaaa aaaacctaaa caaacatgta tctactctct atttcatcta     960
tctctcattt catttttctc tttatctctt tctttatttt tttatcatat catttcacat    1020
taattatttt tactctcttt attttttctc tctatccctc tcttatttcc actcatatat    1080

acactccaaa attggggcat gcctttatca ctactctatc tcctccacta aatcatttaa 1140
atgaaactga aaagcattgg caagtctcct cccctcctca agtgatttcc aactcagcat 1200
tggcatctga ttgattcagt atatctattg catgtgtaaa agtctttcca caatacataa 1260
ctattaatta atcttaaata aataaaggat aaaatatttt tttttcttca taaaattaaa 1320
atatgttatt ttttgtttag atgtatattc gaataaatct aaatatatga taatgatttt 1380
ttatattgat taaacatata atcaatatta aatatgatat ttttttatat aggttgtaca 1440
cataatttta taaggataaa aaatatgata aaaataaatt ttaaatattt ttatatttac 1500
gagaaaaaaa aatattttag ccataaataa atgaccagca tattttacaa ccttagtaat 1560
tcataaattc ctatatgtat atttgaaatt aaaaacagat aatcgttaag ggaaggaatc 1620
ctacgtcatc tcttgccatt tgtttttcat gcaaacagaa agggacgaaa aaccacctca 1680
ccatgaatca ctcttcacac catttttact agcaaacaag tctcaacaac tgaagccagc 1740
tctctttccg tttcttttta caacactttc tttgaaatag tagtattttt ttttcacatg 1800
atttattaac gtgccaaaag atgcttattg aatagagtgc acatttgtaa tgtactacta 1860
attagaacat gaaaaagcat tgttctaaca cgataatcct gtgaaggcgt taactccaaa 1920
gatccaattt cactatataa attgtgacga aagcaaaatg aattcacata gctgagagag 1980
aaaggaaagg ttaactaaga agcaatactt ca 2012

<210> 4
<211> 27
<212> DNA
<213> Artificial Sequence

<220>
<223> synthetic oligonucleotide

<400> 4
ggtccaatat ggaacgatga gttgata 27

<210> 5
<211> 35
<212> DNA
<213> Artificial Sequence

<220>
<223> synthetic oligonucleotide

<400> 5
cgcggatccg ctggaactag aagagagacc taaga 35

<210> 6
<211> 1408
<212> DNA
<213> Glycine max

<400> 6
aactaaaaaa agctctcaaa ttacattttg agttgtttca ggttccattg ccttattgct 60
aaaactccaa ctaaaataac aaatagcaca tgcaggtgca aacaacacgt tactctgatg 120
aaggtgatgt gcctctagca gtctagctta tgaggctcgc tgcttatcaa cgattcatca 180
ttccccaaga cgtgtacgca gattaaacaa tggacaaaac ttcaatcgat tatagaataa 240
taattttaac agtgccgact tttttctgta aacaaaaggc cagaatcata tcgcacatca 300
tcttgaatgc agtgtcgagt ttggaccatt tgagtacaaa gccaatattg aatgattttt 360
cgattttaca tgtgtgaatc agacaaaagt gcatgcaatc acttgcaagt aaattaagga 420
tactaatcta ttcctttcat tttatatgct ccacttttat ataaaaaaat atacattatt 480
atatatgcat tattaattat tgcagtatta tgctattggt tttatggccc tgctaaataa 540
cctaaatgag tctaactatt gcatatgaat caaatgaagg aagaatcatg atctaaacct 600
gagtacccaa tgcaataaaa tgcgtcctat tacctaaact tcaaacacac attgccatcg 660
gacgtataaa ttaatgcata taggttattt tgagaaaaga aaacatcaaa agctctaaaa 720
cttcttttaa ctttgaaata agctgataaa aatacgcttt aaatcaactg tgtgctgtat 780
ataagctgca atttcacatt ttaccaaacc gaaacaagaa tggtaacagt gaggcaaaaa 840
tttgaaaaat gtcctacttc acattcacat caaattaatt acaactaaat aaataaacat 900
cgtgattcaa gcagtaatga aagtcgaaat cagatagaat atacacgttt aacatcaatt 960
gaattttttt ttaaatggat atatacaagt ttactatttt atatataatg aaaattcatt 1020
ttgtgttagc acaaaactta cagaaagaga taaattttaa ataaagagaa ttatatccaa 1080
ttttataatc caaaataatc aaattaaaga atattggcta gatagaccgg ctttttcact 1140
gcccctgctg gataatgaaa attcatatca aaacaataca gaagttctag tttaataata 1200

aaaaagttgg caaactgtca ttccctgttg gtttttaagc caaatcacaa ttcaattacg 1260
tatcagaaat taatttaaac caaatatata gctacgaggg aacttcttca gtcattacta 1320
gctagctcac taatcactat atatacgaca tgctacaagt gaagtgacca tatcttaatt 1380
tcaaatcata aaattcttcc accaagtt 1408

<210> 7
<211> 898
<212> DNA
<213> Glycine max

<400> 7
tatatatgtg agggtagagg gtatcacatg agctctggat ttccataatg aaaaggaatc 60
agaaaaaaga aaagggtttg caactaaaaa cttgggaaag aacaaaggtt taatcttggg 120
atcggtgacc aaacctcttt ttgataccat cttccattta atctagaata tgaaaataag 180
tggataataa aaaagaaaaa tgatatttaa tctaagttca aacaactcga ttagtccttt 240
cctcagttat aaaaaggaaa acaaaacaac gtacaactca atcagatttc aatttgctta 300
ttttgtttca actcaatatt tagcttttaa taattaacta aggtttttat attatattta 360
gaattttttt tctcctttta ttttatttgc atgtatatta ggagttgtcc aatgataatt 420
attctttaat aatgaatcat tagtcttaca tcattacatg atacacatgt atgagatgtc 480
cactccatct cttgttaatt tgatgggcat ccattactta tcaaccatcc gccatagtta 540
tctggttgtg tattttgtta tctgttggta ctctggagta gcatgcataa cgctatattt 600
ttatttctag gatcatgcat atacgcgcaa accaaagaac agagaccgat gtaaagacaa 660
aacatagagt atcctttcca aaacaacgtc caagttcata aaatagagac gaaatgcaag 720
cacagcacac ataagtggat gatcaagatg ggctcgtcca tgccacgcac accaacacac 780
gtcaagcagc aagccctccc gtggccaaat gtgcatgcat acatgttaac aagagcttgc 840
ataactataa atagccctaa tctcactcca tgtttcatcg tccaataata tatatact 898

<210> 8
<211> 36
<212> DNA
<213> Artificial Sequence

<220>
<223> synthetic oligonucleotide

<400> 8
cgcggatcct atatatgtga gggtagaggg tatcac 36

<210> 9
<211> 44
<212> DNA
<213> Artificial Sequence

<220>
<223> synthetic oligonucleotide

<400> 9
gaattcgcgg ccgcagtata tatattattg gacgatgaaa catg 44

<210> 10
<211> 690
<212> DNA
<213> Glycine max

<400> 10
tagcctaagt acgtactcaa aatgccaaca aataaaaaaa aagttgcttt aataatgcca 60
aaacaaatta ataaaacact tacaacaccg gatttttttt aattaaaatg tgccatttag 120
gataaatagt taatattttt aataattatt taaaaagccg tatctactaa aatgattttt 180
atttggttga aaatattaat atgtttaaat caacacaatc tatcaaaatt aaactaaaaa 240
aaaaataagt gtacgtggtt aacattagta cagtaatata agaggaaaat gagaaattaa 300
gaaattgaaa gcgagtctaa tttttaaatt atgaacctgc atatataaaa ggaaagaaag 360
aatccaggaa gaaaagaaat gaaaccatgc atggtcccct cgtcatcacg agtttctgcc 420
atttgcaata gaaacactga aacacctttc tctttgtcac ttaattgaga tgccgaagcc 480
acctcacacc atgaacttca tgaggtgtag cacccaaggc ttccatagcc atgcatactg 540
aagaatgtct caagctcagc accctacttc tgtgacgttg tccctcattc accttcctct 600

cttccctata aataaccacg cctcaggttc tccgcttcac aactcaaaca ttctcctcca 660
ttggtcctta aacactcatc agtcatcacc 690

<210> 11
<211> 36
<212> DNA
<213> Artificial Sequence

<220>
<223> synthetic oligonucleotide

<400> 11
cgcggatcct agcctaagta cgtactcaaa atgcca 36

<210> 12
<211> 41
<212> DNA
<213> Artificial Sequence

<220>
<223> synthetic oligonucleotide

<400> 12
gaattcgcgg ccgcggtgat gactgatgag tgtttaagga c 41

<210> 13
<211> 32
<212> DNA
<213> Artificial Sequence

<220>
<223> synthetic oligonucleotide

<400> 13
ttgcggccgc aaaccatggc tgctgctccc ag 32

<210> 14
<211> 24
<212> DNA
<213> Artificial Sequence

<220>
<223> synthetic oligonucleotide

<400> 14
aagcggccgc ttactgcgcc ttac 24

<210> 15
<211> 34
<212> DNA
<213> Artificial Sequence

<220>
<223> synthetic oligonucleotide

<400> 15
atctagacct gcaggccaac tgcgtttggg gctc 34

<210> 16
<211> 40
<212> DNA
<213> Artificial Sequence

<220>
<223> synthetic oligonucleotide

<400>  16
cttttaactt cgcggccgct tgctattgat gggtgaagtg                              40
<210>  17
<211>  38
<212>  DNA
<213>  Artificial Sequence

<220>
<223>  synthetic oligonucleotide

<400>  17
caatagcaag cggccgcgaa gttaaaagca atgttgtc                                38

<210>  18
<211>  35
<212>  DNA
<213>  Artificial Sequence

<220>
<223>  synthetic oligonucleotide

<400>  18
aatctagacg tacgcaaagg caaagattta aactc                                   35

<210>  19
<211>  36
<212>  DNA
<213>  Artificial Sequence

<220>
<223>  synthetic oligonucleotide

<400>  19
tttctagacg tacgtccctt cttatctttg atctcc                                  36

<210>  20
<211>  34
<212>  DNA
<213>  Artificial Sequence

<220>
<223>  synthetic oligonucleotide

<400>  20
gcggccgcag ttggatagaa tatatgtttg tgac                                    34

<210>  21
<211>  41
<212>  DNA
<213>  Artificial Sequence

<220>
<223>  synthetic oligonucleotide

<400>  21
ctatccaact gcggccgcat ttcgcaccaa atcaatgaaa g                            41

<210>  22
<211>  38
<212>  DNA
<213>  Artificial Sequence

<220>
<223>  synthetic oligonucleotide

<400>  22
aatctagacg tacgtgaagg ttaaacatgg tgaatatg                              38

<210>  23
<211>  29
<212>  DNA
<213>  Artificial Sequence

<220>
<223>  synthetic oligonucleotide

<400>  23
atctagacgt acgtcctcga agagaaggg                                        29

<210>  24
<211>  22
<212>  DNA
<213>  Artificial Sequence

<220>
<223>  synthetic oligonucleotide

<400>  24
ttctagacgt acggatataa tg                                               22

<210>  25
<211>  36
<212>  DNA
<213>  Artificial Sequence

<220>
<223>  synthetic oligonucleotide

<400>  25
tttctagacg tacggtctca atagattaag aagttg                                36

<210>  26
<211>  33
<212>  DNA
<213>  Artificial Sequence

<220>
<223>  synthetic oligonucleotide

<400>  26
gcggccgcga agagagatac taagagaatg ttg                                   33

<210>  27
<211>  39
<212>  DNA
<213>  Artificial Sequence

<220>
<223>  synthetic oligonucleotide

<400>  27
gtatctctct tcgcggccgc atttggcacc aaatcaatg                             39

<210>  28
<211>  36
<212>  DNA
<213>  Artificial Sequence

<220>

<223>  synthetic oligonucleotide

<400>  28
tttctagacg tacgtcaaaa aatttcattg taactc                                36

<210>  29
<211>  37
<212>  DNA
<213>  Artificial Sequence

<220>
<223>  synthetic oligonucleotide

<400>  29
cgcggatcca tcttaggccc ttgattatat ggtgttt                               37

<210>  30
<211>  43
<212>  DNA
<213>  Artificial Sequence

<220>
<223>  synthetic oligonucleotide

<400>  30
gaattcgcgg ccgctgaagt attgcttctt agttaacctt tcc                        43

<210>  31
<211>  41
<212>  DNA
<213>  Artificial Sequence

<220>
<223>  synthetic oligonucleotide

<400>  31
cgcggatcca actaaaaaaa gctctcaaat tacattttga g                          41

<210>  32
<211>  44
<212>  DNA
<213>  Artificial Sequence

<220>
<223>  synthetic oligonucleotide

<400>  32
gaattcgcgg ccgcaacttg gtggaagaat tttatgattt gaaa                       44

<210>  33
<211>  1617
<212>  DNA
<213>  Mortierella alpina

<400>  33
cgacactcct tccttcttct cacccgtcct agtccccttc aacccccctc tttgacaaag      60
acaacaaacc atggctgctg ctcccagtgt gaggacgttt actcgggccg aggttttgaa     120
tgccgaggct ctgaatgagg gcaagaagga tgccgaggca cccttcttga tgatcatcga     180
caacaaggtg tacgatgtcc gcgagttcgt ccctgatcat cccggtggaa gtgtgattct     240
cacgcacgtt ggcaaggacg gcactgacgt ctttgacact tttcaccccg aggctgcttg     300
ggagactctt gccaactttt acgttggtga tattgacgag agcgaccgcg atatcaagaa     360
tgatgacttt gcggccgagg tccgcaagct gcgtaccttg ttccagtctc ttggttacta     420
cgattcttcc aaggcatact acgccttcaa ggtctcgttc aacctctgca tctggggttt     480
gtcgacggtc attgtggcca agtggggcca gacctcgacc ctcgccaacg tgctctcggc     540
tgcgcttttg ggtctgttct ggcagcagtg cggatggttg gctcacgact ttttgcatca     600

ccaggtcttc caggaccgtt tctggggtga tcttttcggc gccttcttgg gaggtgtctg 660
ccagggcttc tcgtcctcgt ggtggaagga caagcacaac actcaccacg ccgcccccaa 720
cgtccacggc gaggatcccg acattgacac ccaccctctg ttgacctgga gtgagcatgc 780
gttggagatg ttctcggatg tcccagatga ggagctgacc cgcatgtggt cgcgtttcat 840
ggtcctgaac cagacctggt tttacttccc cattctctcg tttgcccgtc tctcctggtg 900
cctccagtcc attctctttg tgctgcctaa cggtcaggcc cacaagccct cgggcgcgcg 960
tgtgcccatc tcgttggtcg agcagctgtc gcttgcgatg cactggacct ggtacctcgc 1020
caccatgttc ctgttcatca aggatcccgt caacatgctg gtgtactttt tggtgtcgca 1080
ggcggtgtgc ggaaacttgt tggcgatcgt gttctcgctc aaccacaacg gtatgcctgt 1140
gatctcgaag gaggaggcgg tcgatatgga tttcttcacg aagcagatca tcacgggtcg 1200
tgatgtccac ccgggtctat ttgccaactg gttcacgggt ggattgaact atcagatcga 1260
gcaccacttg ttcccttcga tgcctcgcca caacttttca aagatccagc ctgctgtcga 1320
gaccctgtgc aaaaagtaca atgtccgata ccacaccacc ggtatgatcg agggaactgc 1380
agaggtcttt agccgtctga acgaggtctc caaggctgcc tccaagatgg gtaaggcgca 1440
gtaaaaaaaa aaacaaggac gttttttttc gccagtgcct gtgcctgtgc ctgcttccct 1500
tgtcaagtcg agcgtttctg gaaaggatcg ttcagtgcag tatcatcatt ctccttttac 1560
cccccgctca tatctcattc atttctctta ttaaacaact tgttcccccc ttcaccg 1617

<210> 34
<211> 457
<212> PRT
<213> Mortierella alpina

<400> 34
Met Ala Ala Ala Pro Ser Val Arg Thr Phe Thr Arg Ala Glu Val Leu
1               5                   10                  15

Asn Ala Glu Ala Leu Asn Glu Gly Lys Lys Asp Ala Glu Ala Pro Phe
            20                  25                  30

Leu Met Ile Ile Asp Asn Lys Val Tyr Asp Val Arg Glu Phe Val Pro
        35                  40                  45

Asp His Pro Gly Gly Ser Val Ile Leu Thr His Val Gly Lys Asp Gly
    50                  55                  60

Thr Asp Val Phe Asp Thr Phe His Pro Glu Ala Ala Trp Glu Thr Leu
65                  70                  75                  80

Ala Asn Phe Tyr Val Gly Asp Ile Asp Glu Ser Asp Arg Asp Ile Lys
                85                  90                  95

Asn Asp Asp Phe Ala Ala Glu Val Arg Lys Leu Arg Thr Leu Phe Gln
            100                 105                 110

Ser Leu Gly Tyr Tyr Asp Ser Ser Lys Ala Tyr Tyr Ala Phe Lys Val
        115                 120                 125

Ser Phe Asn Leu Cys Ile Trp Gly Leu Ser Thr Val Ile Val Ala Lys
    130                 135                 140

Trp Gly Gln Thr Ser Thr Leu Ala Asn Val Leu Ser Ala Ala Leu Leu
145                 150                 155                 160

Gly Leu Phe Trp Gln Gln Cys Gly Trp Leu Ala His Asp Phe Leu His
                165                 170                 175

His Gln Val Phe Gln Asp Arg Phe Trp Gly Asp Leu Phe Gly Ala Phe
            180                 185                 190

Leu Gly Gly Val Cys Gln Gly Phe Ser Ser Ser Trp Trp Lys Asp Lys
        195                 200                 205

His Asn Thr His His Ala Ala Pro Asn Val His Gly Glu Asp Pro Asp
    210                 215                 220

Ile Asp Thr His Pro Leu Leu Thr Trp Ser Glu His Ala Leu Glu Met
225                 230                 235                 240

Phe Ser Asp Val Pro Asp Glu Glu Leu Thr Arg Met Trp Ser Arg Phe
                245                 250                 255

Met Val Leu Asn Gln Thr Trp Phe Tyr Phe Pro Ile Leu Ser Phe Ala
            260                 265                 270

Arg Leu Ser Trp Cys Leu Gln Ser Ile Leu Phe Val Leu Pro Asn Gly
        275                 280                 285

Gln Ala His Lys Pro Ser Gly Ala Arg Val Pro Ile Ser Leu Val Glu
    290                 295                 300

Gln Leu Ser Leu Ala Met His Trp Thr Trp Tyr Leu Ala Thr Met Phe
305                 310                 315                 320

Leu Phe Ile Lys Asp Pro Val Asn Met Leu Val Tyr Phe Leu Val Ser
                325                 330                 335

Gln Ala Val Cys Gly Asn Leu Leu Ala Ile Val Phe Ser Leu Asn His
            340                 345                 350

Asn Gly Met Pro Val Ile Ser Lys Glu Glu Ala Val Asp Met Asp Phe
        355                 360                 365

Phe Thr Lys Gln Ile Ile Thr Gly Arg Asp Val His Pro Gly Leu Phe
    370                 375                 380

Ala Asn Trp Phe Thr Gly Gly Leu Asn Tyr Gln Ile Glu His His Leu
385                 390                 395                 400

Phe Pro Ser Met Pro Arg His Asn Phe Ser Lys Ile Gln Pro Ala Val
                405                 410                 415

Glu Thr Leu Cys Lys Lys Tyr Asn Val Arg Tyr His Thr Thr Gly Met
            420                 425                 430

Ile Glu Gly Thr Ala Glu Val Phe Ser Arg Leu Asn Glu Val Ser Lys
        435                 440                 445

Ala Ala Ser Lys Met Gly Lys Ala Gln
    450                 455

<210>  35
<211>  1362
<212>  DNA
<213>  Saprolegnia diclina

<400>  35
atggtccagg ggcaaaaggc cgagaagatc tcgtgggcga ccatccgtga gcacaaccgc      60
caagacaacg cgtggatcgt gatccaccac aaggtgtacg acatctcggc ctttgaggac     120
cacccgggcg gcgtcgtcat gttcacgcag gccggcgaag acgcgaccga tgcgttcgct     180
gtcttccacc cgagctcggc gctcaagctc ctcgagcagt actacgtcgg cgacgtcgac     240
cagtcgacgg cggccgtcga cacgtcgatc tcggacgagg tcaagaagag ccagtcggac     300
ttcattgcgt cgtaccgcaa gctgcgcctt gaagtcaagc gcctcggctt gtacgactcg     360
agcaagctct actacctcta caagtgcgcc tcgacgctga gcattgcgct tgtgtcggcg     420
gccatttgcc tccactttga ctcgacggcc atgtacatgg tcgcggctgt catccttggc     480
ctcttttacc agcagtgcgg ctggctcgcc catgactttc tgcaccacca agtgtttgag     540
aaccacttgt ttggcgacct cgtcggcgtc atggtcggca acctctggca gggcttctcg     600
gtgcagtggt ggaagaacaa gcacaacacg caccatgcga tccccaacct ccacgcgacg     660
cccgagatcg ccttccacgg cgacccggac attgacacga tgccgattct cgcgtggtcg     720
ctcaagatgg cgcagcacgc ggtcgactcg cccgtcgggc tcttcttcat gcgctaccaa     780

gcgtacctgt actttcccat cttgctcttt gcgcgtatct cgtgggtgat ccagtcggcc 840
atgtacgcct tctacaacgt tgggcccggc ggcacctttg acaaggtcca gtacccgctg 900
ctcgagcgcg ccggcctcct cctctactac ggctggaacc tcggccttgt gtacgcagcc 960
aacatgtcgc tgctccaagc ggctgcgttc ctctttgtga gccaggcgtc gtgcggcctc 1020
ttcctcgcga tggtctttag cgtcggccac aacggcatgg aggtctttga caaggacagc 1080
aagcccgatt tttggaagct gcaagtgctc tcgacgcgca acgtgacgtc gtcgctctgg 1140
atcgactggt tcatgggcgg cctcaactac cagatcgacc accacttgtt cccgatggtg 1200
ccccggcaca acctcccggc gctcaacgtg ctcgtcaagt cgctctgcaa gcagtacgac 1260
atcccatacc acgagacggg cttcatcgcg ggcatggccg aggtcgtcgt gcacctcgag 1320
cgcatctcga tcgagttctt caaggagttt cccgccatgt aa 1362

<210> 36
<211> 453
<212> PRT
<213> Saprolegnia diclina

<400> 36
Met Val Gln Gly Gln Lys Ala Glu Lys Ile Ser Trp Ala Thr Ile Arg
1               5                   10                  15

Glu His Asn Arg Gln Asp Asn Ala Trp Ile Val Ile His His Lys Val
            20                  25                  30

Tyr Asp Ile Ser Ala Phe Glu Asp His Pro Gly Gly Val Val Met Phe
        35                  40                  45

Thr Gln Ala Gly Glu Asp Ala Thr Asp Ala Phe Ala Val Phe His Pro
    50                  55                  60

Ser Ser Ala Leu Lys Leu Leu Glu Gln Tyr Tyr Val Gly Asp Val Asp
65                  70                  75                  80

Gln Ser Thr Ala Ala Val Asp Thr Ser Ile Ser Asp Glu Val Lys Lys
                85                  90                  95

Ser Gln Ser Asp Phe Ile Ala Ser Tyr Arg Lys Leu Arg Leu Glu Val
            100                 105                 110

Lys Arg Leu Gly Leu Tyr Asp Ser Ser Lys Leu Tyr Tyr Leu Tyr Lys
        115                 120                 125

Cys Ala Ser Thr Leu Ser Ile Ala Leu Val Ser Ala Ala Ile Cys Leu
    130                 135                 140

His Phe Asp Ser Thr Ala Met Tyr Met Val Ala Ala Val Ile Leu Gly
145                 150                 155                 160

Leu Phe Tyr Gln Gln Cys Gly Trp Leu Ala His Asp Phe Leu His His
                165                 170                 175

Gln Val Phe Glu Asn His Leu Phe Gly Asp Leu Val Gly Val Met Val
            180                 185                 190

Gly Asn Leu Trp Gln Gly Phe Ser Val Gln Trp Trp Lys Asn Lys His
        195                 200                 205

Asn Thr His His Ala Ile Pro Asn Leu His Ala Thr Pro Glu Ile Ala
    210                 215                 220

Phe His Gly Asp Pro Asp Ile Asp Thr Met Pro Ile Leu Ala Trp Ser
225                 230                 235                 240

Leu Lys Met Ala Gln His Ala Val Asp Ser Pro Val Gly Leu Phe Phe
                245                 250                 255

Met Arg Tyr Gln Ala Tyr Leu Tyr Phe Pro Ile Leu Leu Phe Ala Arg
            260                 265                 270

Ile Ser Trp Val Ile Gln Ser Ala Met Tyr Ala Phe Tyr Asn Val Gly
        275                 280                 285

Pro Gly Gly Thr Phe Asp Lys Val Gln Tyr Pro Leu Leu Glu Arg Ala
    290                 295                 300

Gly Leu Leu Leu Tyr Tyr Gly Trp Asn Leu Gly Leu Val Tyr Ala Ala
305                 310                 315                 320

Asn Met Ser Leu Leu Gln Ala Ala Ala Phe Leu Phe Val Ser Gln Ala
                325                 330                 335

Ser Cys Gly Leu Phe Leu Ala Met Val Phe Ser Val Gly His Asn Gly
            340                 345                 350
Met Glu Val Phe Asp Lys Asp Ser Lys Pro Asp Phe Trp Lys Leu Gln
        355                 360                 365

Val Leu Ser Thr Arg Asn Val Thr Ser Ser Leu Trp Ile Asp Trp Phe
    370                 375                 380

Met Gly Gly Leu Asn Tyr Gln Ile Asp His His Leu Phe Pro Met Val
385                 390                 395                 400

Pro Arg His Asn Leu Pro Ala Leu Asn Val Leu Val Lys Ser Leu Cys
                405                 410                 415

Lys Gln Tyr Asp Ile Pro Tyr His Glu Thr Gly Phe Ile Ala Gly Met
            420                 425                 430

Ala Glu Val Val Val His Leu Glu Arg Ile Ser Ile Glu Phe Phe Lys
        435                 440                 445

Glu Phe Pro Ala Met
    450

<210>  37
<211>  1413
<212>  DNA
<213>  Saprolegnia diclina

<400>  37
atggccccgc agacggagct ccgccagcgc cacgccgccg tcgccgagac gccggtggcc      60
ggcaagaagg cctttacatg gcaggaggtc gcgcagcaca acacggcggc ctcggcctgg     120
atcattatcc gcggcaaggt ctacgacgtg accgagtggg ccaacaagca ccccggcggc     180
cgcgagatgg tgctgctgca cgccggtcgc gaggccaccg acacgttcga ctcgtaccac     240
ccgttcagcg acaaggccga gtcgatcttg aacaagtatg agattggcac gttcacgggc     300
ccgtccgagt ttccgacctt caagccggac acgggcttct acaaggagtg ccgcaagcgc     360
gttggcgagt acttcaagaa gaacaacctc catccgcagg acggcttccc gggcctctgg     420
cgcatgatgg tcgtgtttgc ggtcgccggc ctcgccttgt acggcatgca cttttcgact     480
atctttgcgc tgcagctcgc ggccgcggcg ctctttggcg tctgccaggc gctgccgctg     540
ctccacgtca tgcacgactc gtcgcacgcg tcgtacacca acatgccgtt cttccattac     600
gtcgtcggcc gctttgccat ggactggttt gccggcggct cgatggtgtc atggctcaac     660
cagcacgtcg tgggccacca catctacacg aacgtcgcgg gctcggaccc ggatcttccg     720
gtcaacatgg acggcgacat ccgccgcatc gtgaaccgcc aggtgttcca gcccatgtac     780
gcattccagc acatctacct tccgccgctc tatggcgtgc ttggcctcaa gttccgcatc     840
caggacttca ccgacacgtt cggctcgcac acgaacggcc cgatccgcgt caacccgcac     900
gcgctctcga cgtggatggc catgatcagc tccaagtcgt tctgggcctt ctaccgcgtg     960
taccttccgc ttgccgtgct ccagatgccc atcaagacgt accttgcgat cttcttcctc    1020
gccgagtttg tcacgggctg gtacctcgcg ttcaacttcc aagtaagcca tgtctcgacc    1080
gagtgcggct acccatgcgg cgacgaggcc aagatggcgc tccaggacga gtgggcagtc    1140
tcgcaggtca agacgtcggt cgactacgcc catggctcgt ggatgacgac gttccttgcc    1200
ggcgcgctca actaccaggt cgtgcaccac ttgttcccca gcgtgtcgca gtaccactac    1260

ccggcgatcg cgcccatcat cgtcgacgtc tgcaaggagt acaacatcaa gtacgccatc 1320
ttgccggact ttacggcggc gttcgttgcc cacttgaagc acctccgcaa catgggccag 1380
cagggcatcg ccgccacgat ccacatgggc taa 1413

<210> 38
<211> 470
<212> PRT
<213> Saprolegnia diclina

<400> 38
Met Ala Pro Gln Thr Glu Leu Arg Gln Arg His Ala Ala Val Ala Glu
1               5                   10                  15

Thr Pro Val Ala Gly Lys Lys Ala Phe Thr Trp Gln Glu Val Ala Gln
            20                  25                  30

His Asn Thr Ala Ala Ser Ala Trp Ile Ile Ile Arg Gly Lys Val Tyr
        35                  40                  45

Asp Val Thr Glu Trp Ala Asn Lys His Pro Gly Gly Arg Glu Met Val
    50                  55                  60

Leu Leu His Ala Gly Arg Glu Ala Thr Asp Thr Phe Asp Ser Tyr His
65                  70                  75                  80

Pro Phe Ser Asp Lys Ala Glu Ser Ile Leu Asn Lys Tyr Glu Ile Gly
                85                  90                  95

Thr Phe Thr Gly Pro Ser Glu Phe Pro Thr Phe Lys Pro Asp Thr Gly
            100                 105                 110

Phe Tyr Lys Glu Cys Arg Lys Arg Val Gly Glu Tyr Phe Lys Lys Asn
        115                 120                 125

Asn Leu His Pro Gln Asp Gly Phe Pro Gly Leu Trp Arg Met Met Val
    130                 135                 140

Val Phe Ala Val Ala Gly Leu Ala Leu Tyr Gly Met His Phe Ser Thr
145                 150                 155                 160

Ile Phe Ala Leu Gln Leu Ala Ala Ala Ala Leu Phe Gly Val Cys Gln
                165                 170                 175

Ala Leu Pro Leu Leu His Val Met His Asp Ser Ser His Ala Ser Tyr
            180                 185                 190

Thr Asn Met Pro Phe Phe His Tyr Val Val Gly Arg Phe Ala Met Asp
        195                 200                 205

Trp Phe Ala Gly Gly Ser Met Val Ser Trp Leu Asn Gln His Val Val
    210                 215                 220

Gly His His Ile Tyr Thr Asn Val Ala Gly Ser Asp Pro Asp Leu Pro
225                 230                 235                 240

Val Asn Met Asp Gly Asp Ile Arg Arg Ile Val Asn Arg Gln Val Phe
                245                 250                 255

Gln Pro Met Tyr Ala Phe Gln His Ile Tyr Leu Pro Pro Leu Tyr Gly
            260                 265                 270

Val Leu Gly Leu Lys Phe Arg Ile Gln Asp Phe Thr Asp Thr Phe Gly
        275                 280                 285

Ser His Thr Asn Gly Pro Ile Arg Val Asn Pro His Ala Leu Ser Thr

290                 295                 300

Trp Met Ala Met Ile Ser Ser Lys Ser Phe Trp Ala Phe Tyr Arg Val
305                 310                 315                 320

Tyr Leu Pro Leu Ala Val Leu Gln Met Pro Ile Lys Thr Tyr Leu Ala
                325                 330                 335

Ile Phe Phe Leu Ala Glu Phe Val Thr Gly Trp Tyr Leu Ala Phe Asn
            340                 345                 350

Phe Gln Val Ser His Val Ser Thr Glu Cys Gly Tyr Pro Cys Gly Asp
        355                 360                 365

Glu Ala Lys Met Ala Leu Gln Asp Glu Trp Ala Val Ser Gln Val Lys
    370                 375                 380

Thr Ser Val Asp Tyr Ala His Gly Ser Trp Met Thr Thr Phe Leu Ala
385                 390                 395                 400

Gly Ala Leu Asn Tyr Gln Val Val His His Leu Phe Pro Ser Val Ser
                405                 410                 415

Gln Tyr His Tyr Pro Ala Ile Ala Pro Ile Ile Val Asp Val Cys Lys
            420                 425                 430

Glu Tyr Asn Ile Lys Tyr Ala Ile Leu Pro Asp Phe Thr Ala Ala Phe
        435                 440                 445

Val Ala His Leu Lys His Leu Arg Asn Met Gly Gln Gln Gly Ile Ala
    450                 455                 460

Ala Thr Ile His Met Gly
465                 470

<210>  39
<211>  819
<212>  DNA
<213>  Thraustochytrium aureum

<400>  39
atggcaaaca gcagcgtgtg ggatgatgtg gtgggccgcg tggagaccgg cgtggaccag      60
tggatggatg gcgccaagcc gtacgcactc accgatgggc tcccgatgat ggacgtgtcc     120
accatgctgg cattcgaggt gggatacatg gccatgctgc tcttcggcat cccgatcatg     180
aggcagatgg agaagccttt tgagctcaag accatcaagc tcttgcacaa cttgtttctc     240
ttcggacttt ccttgtacat gtgcgtggtg accatccgcc aggctatcct tggaggctac     300
aaagtgtttg gaaacgacat ggagaagggc aacgagtctc atgctcaggg catgtctcgc     360
atcgtgtacg tgttctacgt gtccaaggca tacgagttct tggataccgc catcatgatc     420
ctttgcaaga agttcaacca ggtttccttc ttgcatgtgt accaccatgc caccattttt     480
gccatctggt gggctatcgc caagtacgct ccaggaggtg atgcgtactt ttcagtgatc     540
ctcaactctt tcgtgcacac cgtcatgtac gcatactact tcttctcctc ccaagggttc     600
gggttcgtga agccaatcaa gccgtacatc accacccttc agatgaccca gttcatggca     660
atgcttgtgc agtccttgta cgactacctc ttcccatgcg actacccaca ggctcttgtg     720
cagcttcttg gagtgtacat gatcaccttg cttgccctct tcggcaactt ttttgtgcag     780
agctatctta aaaagccaaa aaagagcaag accaactaa                            819

<210>  40
<211>  272
<212>  PRT
<213>  Thraustochytrium aureum

<400>  40
Met Ala Asn Ser Ser Val Trp Asp Asp Val Val Gly Arg Val Glu Thr
1               5                   10                  15

Gly Val Asp Gln Trp Met Asp Gly Ala Lys Pro Tyr Ala Leu Thr Asp
            20                  25                  30

Gly Leu Pro Met Met Asp Val Ser Thr Met Leu Ala Phe Glu Val Gly
        35                  40                  45

Tyr Met Ala Met Leu Leu Phe Gly Ile Pro Ile Met Arg Gln Met Glu
    50                  55                  60

Lys Pro Phe Glu Leu Lys Thr Ile Lys Leu Leu His Asn Leu Phe Leu
65                  70                  75                  80

Phe Gly Leu Ser Leu Tyr Met Cys Val Val Thr Ile Arg Gln Ala Ile
                85                  90                  95

Leu Gly Gly Tyr Lys Val Phe Gly Asn Asp Met Glu Lys Gly Asn Glu
            100                 105                 110

Ser His Ala Gln Gly Met Ser Arg Ile Val Tyr Val Phe Tyr Val Ser
        115                 120                 125

Lys Ala Tyr Glu Phe Leu Asp Thr Ala Ile Met Ile Leu Cys Lys Lys
    130                 135                 140

Phe Asn Gln Val Ser Phe Leu His Val Tyr His His Ala Thr Ile Phe
145                 150                 155                 160

Ala Ile Trp Trp Ala Ile Ala Lys Tyr Ala Pro Gly Gly Asp Ala Tyr
                165                 170                 175

Phe Ser Val Ile Leu Asn Ser Phe Val His Thr Val Met Tyr Ala Tyr
            180                 185                 190

Tyr Phe Phe Ser Ser Gln Gly Phe Gly Phe Val Lys Pro Ile Lys Pro
        195                 200                 205

Tyr Ile Thr Thr Leu Gln Met Thr Gln Phe Met Ala Met Leu Val Gln
    210                 215                 220

Ser Leu Tyr Asp Tyr Leu Phe Pro Cys Asp Tyr Pro Gln Ala Leu Val
225                 230                 235                 240

Gln Leu Leu Gly Val Tyr Met Ile Thr Leu Leu Ala Leu Phe Gly Asn
                245                 250                 255

Phe Phe Val Gln Ser Tyr Leu Lys Lys Pro Lys Lys Ser Lys Thr Asn
            260                 265                 270

<210>  41
<211>  1077
<212>  DNA
<213>  Saprolegnia diclina

<400>  41
atgactgagg ataagacgaa ggtcgagttc ccgacgctca cggagctcaa gcactcgatc      60
ccgaacgcgt gctttgagtc gaacctcggc ctctcgctct actacacggc ccgcgcgatc     120
ttcaacgcgt cggcctcggc ggcgctgctc tacgcggcgc gctcgacgcc gttcattgcc     180
gataacgttc tgctccacgc gctcgtttgc gccacctaca tctacgtgca gggcgtcatc     240
ttctggggct tcttcacggt cggccacgac tgcggccact cggccttctc gcgctaccac     300
agcgtcaact ttatcatcgg ctgcatcatg cactctgcga ttttgacgcc gttcgagagc     360
tggcgcgtga cgcaccgcca ccaccacaag aacacgggca acattgataa ggacgagatc     420
ttttacccgc accggtcggt caaggacctc caggacgtgc gccaatgggt ctacacgctc     480
ggcggtgcgt ggtttgtcta cttgaaggtc gggtatgccc cgcgcacgat gagccacttt     540
gacccgtggg acccgctcct ccttcgccgc gcgtcggccg tcatcgtgtc gctcggcgtc     600
tgggccgcct tcttcgccgc gtacgcgtac ctcacatact cgctcggctt tgccgtcatg     660

ggcctctact actatgcgcc gctctttgtc tttgcttcgt tcctcgtcat tacgaccttc 720
ttgcaccaca acgacgaagc gacgccgtgg tacggcgact cggagtggac gtacgtcaag 780
ggcaacctct cgagcgtcga ccgctcgtac ggcgcgttcg tggacaacct gagccaccac 840
attggcacgc accaggtcca ccacttgttc ccgatcattc cgcactacaa gctcaacgaa 900
gccaccaagc actttgcggc cgcgtacccg cacctcgtgc gcaggaacga cgagcccatc 960
atcacggcct tcttcaagac cgcgcacctc tttgtcaact acggcgctgt gcccgagacg 1020
gcgcagatct tcacgctcaa agagtcggcc gcggccgcca aggccaagtc ggactaa 1077

<210> 42
<211> 358
<212> PRT
<213> Saprolegnia diclina

<400> 42
Met Thr Glu Asp Lys Thr Lys Val Glu Phe Pro Thr Leu Thr Glu Leu
1               5                   10                  15

Lys His Ser Ile Pro Asn Ala Cys Phe Glu Ser Asn Leu Gly Leu Ser
            20                  25                  30

Leu Tyr Tyr Thr Ala Arg Ala Ile Phe Asn Ala Ser Ala Ser Ala Ala
        35                  40                  45

Leu Leu Tyr Ala Ala Arg Ser Thr Pro Phe Ile Ala Asp Asn Val Leu
    50                  55                  60

Leu His Ala Leu Val Cys Ala Thr Tyr Ile Tyr Val Gln Gly Val Ile
65                  70                  75                  80

Phe Trp Gly Phe Phe Thr Val Gly His Asp Cys Gly His Ser Ala Phe
                85                  90                  95

Ser Arg Tyr His Ser Val Asn Phe Ile Ile Gly Cys Ile Met His Ser
            100                 105                 110

Ala Ile Leu Thr Pro Phe Glu Ser Trp Arg Val Thr His Arg His His
        115                 120                 125

His Lys Asn Thr Gly Asn Ile Asp Lys Asp Glu Ile Phe Tyr Pro His
    130                 135                 140

Arg Ser Val Lys Asp Leu Gln Asp Val Arg Gln Trp Val Tyr Thr Leu
145                 150                 155                 160

Gly Gly Ala Trp Phe Val Tyr Leu Lys Val Gly Tyr Ala Pro Arg Thr
                165                 170                 175

Met Ser His Phe Asp Pro Trp Asp Pro Leu Leu Leu Arg Arg Ala Ser
            180                 185                 190

Ala Val Ile Val Ser Leu Gly Val Trp Ala Ala Phe Phe Ala Ala Tyr
        195                 200                 205

Ala Tyr Leu Thr Tyr Ser Leu Gly Phe Ala Val Met Gly Leu Tyr Tyr
    210                 215                 220

Tyr Ala Pro Leu Phe Val Phe Ala Ser Phe Leu Val Ile Thr Thr Phe
225                 230                 235                 240

Leu His His Asn Asp Glu Ala Thr Pro Trp Tyr Gly Asp Ser Glu Trp
                245                 250                 255

Thr Tyr Val Lys Gly Asn Leu Ser Ser Val Asp Arg Ser Tyr Gly Ala
            260                 265                 270

Phe Val Asp Asn Leu Ser His His Ile Gly Thr His Gln Val His His
        275                 280                 285

Leu Phe Pro Ile Ile Pro His Tyr Lys Leu Asn Glu Ala Thr Lys His
    290                 295                 300

Phe Ala Ala Ala Tyr Pro His Leu Val Arg Arg Asn Asp Glu Pro Ile
305                 310                 315                 320

Ile Thr Ala Phe Phe Lys Thr Ala His Leu Phe Val Asn Tyr Gly Ala
                325                 330                 335

Val Pro Glu Thr Ala Gln Ile Phe Thr Leu Lys Glu Ser Ala Ala Ala
            340                 345                 350

Ala Lys Ala Lys Ser Asp
        355

<210>  43
<211>  954
<212>  DNA
<213>  Mortierella alpina

<400>  43
atggccgccg caatcttgga caaggtcaac ttcggcattg atcagccctt cggaatcaag      60
ctcgacacct actttgctca ggcctatgaa ctcgtcaccg gaaagtccat cgactccttc     120
gtcttccagg agggcgtcac gcctctctcg acccagagag aggtcgccat gtggactatc     180
acttacttcg tcgtcatctt tggtggtcgc cagatcatga agagccagga cgccttcaag     240
ctcaagcccc tcttcatcct ccacaacttc ctcctgacga tcgcgtccgg atcgctgttg     300
ctcctgttca tcgagaacct ggtccccatc ctcgccagaa acggactttt ctacgccatc     360
tgcgacgacg gtgcctggac ccagcgcctc gagctcctct actacctcaa ctacctggtc     420
aagtactggg agttggccga caccgtcttt ttggtcctca agaagaagcc tcttgagttc     480
ctgcactact tccaccactc gatgaccatg gttctctgct ttgtccagct tggaggatac     540
acttcagtgt cctgggtccc tattaccctc aacttgactg tccacgtctt catgtactac     600
tactacatgc gctccgctgc cggtgttcgc atctggtgga agcagtactt gaccactctc     660
cagatcgtcc agttcgttct tgacctcgga ttcatctact tctgcgccta cacctacttc     720
gccttcacct acttcccctg ggctcccaac gtcggcaagt gcgccggtac cgagggtgct     780
gctctctttg gctgcggact cctctccagc tatctcttgc tctttatcaa cttctaccgc     840
attacctaca atgccaaggc caaggcagcc aaggagcgtg gaagcaactt tacccccaag     900
actgtcaagt ccggcggatc gcccaagaag ccctccaaga gcaagcacat ctaa           954

<210>  44
<211>  317
<212>  PRT
<213>  Mortierella alpina

<400>  44
Met Ala Ala Ala Ile Leu Asp Lys Val Asn Phe Gly Ile Asp Gln Pro
1               5                   10                  15

Phe Gly Ile Lys Leu Asp Thr Tyr Phe Ala Gln Ala Tyr Glu Leu Val
            20                  25                  30

Thr Gly Lys Ser Ile Asp Ser Phe Val Phe Gln Glu Gly Val Thr Pro
        35                  40                  45

Leu Ser Thr Gln Arg Glu Val Ala Met Trp Thr Ile Thr Tyr Phe Val
    50                  55                  60

Val Ile Phe Gly Gly Arg Gln Ile Met Lys Ser Gln Asp Ala Phe Lys
65                  70                  75                  80

Leu Lys Pro Leu Phe Ile Leu His Asn Phe Leu Leu Thr Ile Ala Ser
                85                  90                  95

Gly Ser Leu Leu Leu Leu Phe Ile Glu Asn Leu Val Pro Ile Leu Ala
            100                 105                 110

Arg Asn Gly Leu Phe Tyr Ala Ile Cys Asp Asp Gly Ala Trp Thr Gln
        115                 120                 125

Arg Leu Glu Leu Leu Tyr Tyr Leu Asn Tyr Leu Val Lys Tyr Trp Glu
    130                 135                 140

Leu Ala Asp Thr Val Phe Leu Val Leu Lys Lys Lys Pro Leu Glu Phe
145                 150                 155                 160

Leu His Tyr Phe His His Ser Met Thr Met Val Leu Cys Phe Val Gln
                165                 170                 175

Leu Gly Gly Tyr Thr Ser Val Ser Trp Val Pro Ile Thr Leu Asn Leu
            180                 185                 190

Thr Val His Val Phe Met Tyr Tyr Tyr Tyr Met Arg Ser Ala Ala Gly
        195                 200                 205

Val Arg Ile Trp Trp Lys Gln Tyr Leu Thr Thr Leu Gln Ile Val Gln
    210                 215                 220

Phe Val Leu Asp Leu Gly Phe Ile Tyr Phe Cys Ala Tyr Thr Tyr Phe
225                 230                 235                 240

Ala Phe Thr Tyr Phe Pro Trp Ala Pro Asn Val Gly Lys Cys Ala Gly
                245                 250                 255

Thr Glu Gly Ala Ala Leu Phe Gly Cys Gly Leu Leu Ser Ser Tyr Leu
            260                 265                 270

Leu Leu Phe Ile Asn Phe Tyr Arg Ile Thr Tyr Asn Ala Lys Ala Lys
        275                 280                 285

Ala Ala Lys Glu Arg Gly Ser Asn Phe Thr Pro Lys Thr Val Lys Ser
    290                 295                 300

Gly Gly Ser Pro Lys Lys Pro Ser Lys Ser Lys His Ile
305                 310                 315

<210>  45
<211>  1483
<212>  DNA
<213>  Mortierella alpina

<400>  45
gcttcctcca gttcatcctc catttcgcca cctgcattct ttacgaccgt taagcaagat      60
gggaacggac caaggaaaaa ccttcacctg ggaagagctg gcggcccata acaccaagga     120
cgacctactc ttggccatcc gcggcagggt gtacgatgtc acaaagttct tgagccgcca     180
tcctggtgga gtggacactc tcctgctcgg agctggccga gatgttactc cggtctttga     240
gatgtatcac gcgtttgggg ctgcagatgc cattatgaag aagtactatg tcggtacact     300
ggtctcgaat gagctgccca tcttcccgga gccaacggtg ttccacaaaa ccatcaagac     360
gagagtcgag ggctacttta cggatcggaa cattgatccc aagaatagac cagagatctg     420
gggacgatac gctcttatct ttggatcctt gatcgcttcc tactacgcgc agctctttgt     480
gcctttcgtt gtcgaacgca catggcttca ggtggtgttt gcaatcatca tgggatttgc     540
gtgcgcacaa gtcggactca accctcttca tgatgcgtct cacttttcag tgacccacaa     600
ccccactgtc tggaagattc tgggagccac gcacgacttt ttcaacggag catcgtacct     660
ggtgtggatg taccaacata tgctcggcca tcacccctac accaacattg ctggagcaga     720
tcccgacgtg tcgacgtctg agcccgatgt tcgtcgtatc aagcccaacc aaaagtggtt     780
tgtcaaccac atcaaccagc acatgtttgt tcctttcctg tacggactgc tggcgttcaa     840
ggtgcgcatt caggacatca acattttgta ctttgtcaag accaatgacg ctattcgtgt     900
caatcccatc tcgacatggc acactgtgat gttctggggc ggcaaggctt tctttgtctg     960
gtatcgcctg attgttcccc tgcagtatct gcccctgggc aaggtgctgc tcttgttcac    1020

ggtcgcggac atggtgtcgt cttactggct ggcgctgacc ttccaggcga accacgttgt 1080
tgaggaagtt cagtggccgt tgcctgacga gaacgggatc atccaaaagg actgggcagc 1140
tatgcaggtc gagactacgc aggattacgc acacgattcg cacctctgga ccagcatcac 1200
tggcagcttg aactaccagg ctgtgcacca tctgttcccc aacgtgtcgc agcaccatta 1260
tcccgatatt ctggccatca tcaagaacac ctgcagcgag tacaaggttc cataccttgt 1320
caaggatacg ttttggcaag catttgcttc acatttggag cacttgcgtg ttcttggact 1380
ccgtcccaag gaagagtaga agaaaaaaag cgccgaatga agtattgccc cctttttctc 1440
caagaatggc aaaaggagat caagtggaca ttctctatga aga 1483

<210> 46
<211> 446
<212> PRT
<213> Mortierella alpina

<400> 46
Met Gly Thr Asp Gln Gly Lys Thr Phe Thr Trp Glu Glu Leu Ala Ala
1               5                   10                  15

His Asn Thr Lys Asp Asp Leu Leu Leu Ala Ile Arg Gly Arg Val Tyr
            20                  25                  30

Asp Val Thr Lys Phe Leu Ser Arg His Pro Gly Gly Val Asp Thr Leu
        35                  40                  45

Leu Leu Gly Ala Gly Arg Asp Val Thr Pro Val Phe Glu Met Tyr His
    50                  55                  60

Ala Phe Gly Ala Ala Asp Ala Ile Met Lys Lys Tyr Tyr Val Gly Thr
65                  70                  75                  80

Leu Val Ser Asn Glu Leu Pro Ile Phe Pro Glu Pro Thr Val Phe His
                85                  90                  95

Lys Thr Ile Lys Thr Arg Val Glu Gly Tyr Phe Thr Asp Arg Asn Ile
            100                 105                 110

Asp Pro Lys Asn Arg Pro Glu Ile Trp Gly Arg Tyr Ala Leu Ile Phe
        115                 120                 125

Gly Ser Leu Ile Ala Ser Tyr Tyr Ala Gln Leu Phe Val Pro Phe Val
    130                 135                 140

Val Glu Arg Thr Trp Leu Gln Val Val Phe Ala Ile Ile Met Gly Phe
145                 150                 155                 160

Ala Cys Ala Gln Val Gly Leu Asn Pro Leu His Asp Ala Ser His Phe
                165                 170                 175

Ser Val Thr His Asn Pro Thr Val Trp Lys Ile Leu Gly Ala Thr His
            180                 185                 190

Asp Phe Phe Asn Gly Ala Ser Tyr Leu Val Trp Met Tyr Gln His Met
        195                 200                 205

Leu Gly His His Pro Tyr Thr Asn Ile Ala Gly Ala Asp Pro Asp Val
    210                 215                 220

Ser Thr Ser Glu Pro Asp Val Arg Arg Ile Lys Pro Asn Gln Lys Trp
225                 230                 235                 240

Phe Val Asn His Ile Asn Gln His Met Phe Val Pro Phe Leu Tyr Gly
                245                 250                 255

Leu Leu Ala Phe Lys Val Arg Ile Gln Asp Ile Asn Ile Leu Tyr Phe
            260                 265                 270

Val Lys Thr Asn Asp Ala Ile Arg Val Asn Pro Ile Ser Thr Trp His
        275                 280                 285

Thr Val Met Phe Trp Gly Gly Lys Ala Phe Phe Val Trp Tyr Arg Leu
    290                 295                 300

Ile Val Pro Leu Gln Tyr Leu Pro Leu Gly Lys Val Leu Leu Leu Phe
305                 310                 315                 320

Thr Val Ala Asp Met Val Ser Ser Tyr Trp Leu Ala Leu Thr Phe Gln
                325                 330                 335

Ala Asn His Val Val Glu Glu Val Gln Trp Pro Leu Pro Asp Glu Asn
            340                 345                 350

Gly Ile Ile Gln Lys Asp Trp Ala Ala Met Gln Val Glu Thr Thr Gln
        355                 360                 365

Asp Tyr Ala His Asp Ser His Leu Trp Thr Ser Ile Thr Gly Ser Leu
    370                 375                 380

Asn Tyr Gln Ala Val His His Leu Phe Pro Asn Val Ser Gln His His
385                 390                 395                 400

Tyr Pro Asp Ile Leu Ala Ile Ile Lys Asn Thr Cys Ser Glu Tyr Lys
                405                 410                 415

Val Pro Tyr Leu Val Lys Asp Thr Phe Trp Gln Ala Phe Ala Ser His
            420                 425                 430

Leu Glu His Leu Arg Val Leu Gly Leu Arg Pro Lys Glu Glu
        435                 440                 445

<210>  47
<211>  1350
<212>  DNA
<213>  Arabidopsis thaliana

<400>  47
ctctctctct ctctcttctc tctttctctc cccctctctc cggcgatggt tgttgctatg      60
gaccaacgca ccaatgtgaa cggagatccc ggcgccggag accggaagaa agaagaaagg     120
tttgatccga gtgcacaacc accgttcaag atcggagata taagggcggc gattcctaag     180
cactgttggg ttaagagtcc tttgagatca atgagttacg tcgtcagaga cattatcgcc     240
gtcgcggctt tggccatcgc tgccgtgtat gttgatagct ggttcctttg gcctctttat     300
tgggccgccc aaggaacact tttctgggcc atctttgttc tcggccacga ctgtggacat     360
gggagtttct cagacattcc tctactgaat agtgtggttg gtcacattct tcattctttc     420
atcctcgttc cttaccatgg ttggagaata agccaccgga cacaccacca gaaccatggc     480
catgttgaaa acgacgagtc atgggttccg ttaccagaaa gggtgtacaa gaaattgccc     540
cacagtactc ggatgctcag atacactgtc cctctcccca tgctcgcata tcctctctat     600
ttgtgctaca gaagtcctgg aaaagaagga tcacatttta acccatacag tagtttattt     660
gctccaagcg agagaaagct tattgcaact tcaactactt gttggtccat aatgttcgtc     720
agtcttatcg ctctatcttt cgtcttcggt ccactcgcgg ttcttaaagt ctacggtgta     780
ccgtacatta tctttgtgat gtggttggat gctgtcacgt atttgcatca tcatggtcac     840
gatgagaagt tgccttggta tagaggcaag gaatggagtt atctacgtgg aggattaaca     900
acaattgata gagattacgg aatctttaac aacattcatc acgacattgg aactcacgtg     960
atccatcatc tcttcccaca aatccctcac tatcacttgg tcgacgccac gaaagcagct    1020
aaacatgtgt tgggaagata ctacagagaa ccaaagacgt caggagcaat accgatccac    1080
ttggtggaga gtttggtcgc aagtattaag aaagatcatt acgtcagcga cactggtgat    1140
attgtcttct acgagacaga tccagatctc tacgtttacg cttctgacaa atctaaaatc    1200
aattaatctc catttgttta gctctattag gaataaacca gcccactttt aaaattttta    1260
tttcttgttg tttttaagtt aaaagtgtac tcgtgaaact cttttttttt tctttttttt    1320
tattaatgta tttacattac aaggcgtaaa                                     1350

<210>  48

<211>  386
<212>  PRT
<213>  Arabidopsis thaliana

<400>  48
Met Val Val Ala Met Asp Gln Arg Thr Asn Val Asn Gly Asp Pro Gly
1               5                   10                  15

Ala Gly Asp Arg Lys Lys Glu Glu Arg Phe Asp Pro Ser Ala Gln Pro
            20                  25                  30

Pro Phe Lys Ile Gly Asp Ile Arg Ala Ala Ile Pro Lys His Cys Trp
        35                  40                  45

Val Lys Ser Pro Leu Arg Ser Met Ser Tyr Val Val Arg Asp Ile Ile
    50                  55                  60

Ala Val Ala Ala Leu Ala Ile Ala Ala Val Tyr Val Asp Ser Trp Phe
65                  70                  75                  80

Leu Trp Pro Leu Tyr Trp Ala Ala Gln Gly Thr Leu Phe Trp Ala Ile
                85                  90                  95

Phe Val Leu Gly His Asp Cys Gly His Gly Ser Phe Ser Asp Ile Pro
            100                 105                 110

Leu Leu Asn Ser Val Val Gly His Ile Leu His Ser Phe Ile Leu Val
        115                 120                 125

Pro Tyr His Gly Trp Arg Ile Ser His Arg Thr His His Gln Asn His
    130                 135                 140

Gly His Val Glu Asn Asp Glu Ser Trp Val Pro Leu Pro Glu Arg Val
145                 150                 155                 160

Tyr Lys Lys Leu Pro His Ser Thr Arg Met Leu Arg Tyr Thr Val Pro
                165                 170                 175

Leu Pro Met Leu Ala Tyr Pro Leu Tyr Leu Cys Tyr Arg Ser Pro Gly
            180                 185                 190

Lys Glu Gly Ser His Phe Asn Pro Tyr Ser Ser Leu Phe Ala Pro Ser
        195                 200                 205

Glu Arg Lys Leu Ile Ala Thr Ser Thr Thr Cys Trp Ser Ile Met Phe
    210                 215                 220

Val Ser Leu Ile Ala Leu Ser Phe Val Phe Gly Pro Leu Ala Val Leu
225                 230                 235                 240

Lys Val Tyr Gly Val Pro Tyr Ile Ile Phe Val Met Trp Leu Asp Ala
                245                 250                 255

Val Thr Tyr Leu His His His Gly His Asp Glu Lys Leu Pro Trp Tyr
            260                 265                 270

Arg Gly Lys Glu Trp Ser Tyr Leu Arg Gly Gly Leu Thr Thr Ile Asp
        275                 280                 285

Arg Asp Tyr Gly Ile Phe Asn Asn Ile His His Asp Ile Gly Thr His
    290                 295                 300

Val Ile His His Leu Phe Pro Gln Ile Pro His Tyr His Leu Val Asp
305                 310                 315                 320

Ala Thr Lys Ala Ala Lys His Val Leu Gly Arg Tyr Tyr Arg Glu Pro
                325                 330                 335

Lys Thr Ser Gly Ala Ile Pro Ile His Leu Val Glu Ser Leu Val Ala
            340                 345                 350

Ser Ile Lys Lys Asp His Tyr Val Ser Asp Thr Gly Asp Ile Val Phe
        355                 360                 365

Tyr Glu Thr Asp Pro Asp Leu Tyr Val Tyr Ala Ser Asp Lys Ser Lys
    370                 375                 380

Ile Asn
385

<210>  49
<211>  834
<212>  DNA
<213>  Pavlova sp.

<400>  49
atgatgttgg ccgcaggcta tcttctagtg ctctcggccg ctcgccagag cttccagcag      60
gacattgaca accccaacgg ggcctactcg acctcgtgga ctggcctgcc cattgtgatg     120
tctgtggtct atctcagcgg tgtgtttggg ctcacaaagt acttcgagaa ccggaagccc     180
atgacggggc tgaaggacta catgttcact tacaatctct accaggtgat catcaacgtg     240
tggtgcgtgg tggcctttct cctggaggtg cggcgtgcgg gcatgtcact catcggcaat     300
aaggtggacc ttgggcccaa ctccttcagg ctcggcttcg tcacgtgggt gcactacaac     360
aacaagtacg tggagctcct cgacacccta tggatggtgc tgcgcaagaa gacgcagcag     420
gtctccttcc tccacgtcta tcatcacgtg cttctgatgt gggcctggtt cgttgtcgtc     480
aagctcggca atggtggtga cgcatatttt ggcggtctca tgaactcgat catccacgtg     540
atgatgtatt cctactacac catggcgctc ctgggctggt catgcccctg gaagcgctac     600
ctcacgcagg cacagctcgt gcagttttgc atctgcctcg cccactccac atgggcggca      660
gtaacgggtg cctacccgtg gcgaatttgc ttggtggagg tgtgggtgat ggtgtccatg     720
ctggtgctct tcacacgctt ctaccgccag gcctatgcca aggaggcgaa ggccaaggag     780
gcgaaaaagc tcgcacagga ggcatcacag gccaaggcgg tcaaggcgga gtaa           834

<210>  50
<211>  277
<212>  PRT
<213>  Pavlova sp.

<400>  50
Met Met Leu Ala Ala Gly Tyr Leu Leu Val Leu Ser Ala Ala Arg Gln
1               5                   10                  15

Ser Phe Gln Gln Asp Ile Asp Asn Pro Asn Gly Ala Tyr Ser Thr Ser
            20                  25                  30

Trp Thr Gly Leu Pro Ile Val Met Ser Val Val Tyr Leu Ser Gly Val
        35                  40                  45

Phe Gly Leu Thr Lys Tyr Phe Glu Asn Arg Lys Pro Met Thr Gly Leu
    50                  55                  60

Lys Asp Tyr Met Phe Thr Tyr Asn Leu Tyr Gln Val Ile Ile Asn Val
65                  70                  75                  80

Trp Cys Val Val Ala Phe Leu Leu Glu Val Arg Arg Ala Gly Met Ser
                85                  90                  95

Leu Ile Gly Asn Lys Val Asp Leu Gly Pro Asn Ser Phe Arg Leu Gly
            100                 105                 110

Phe Val Thr Trp Val His Tyr Asn Asn Lys Tyr Val Glu Leu Leu Asp
        115                 120                 125

Thr Leu Trp Met Val Leu Arg Lys Lys Thr Gln Gln Val Ser Phe Leu
    130                 135                 140

His Val Tyr His His Val Leu Leu Met Trp Ala Trp Phe Val Val Val
145                 150                 155                 160

Lys Leu Gly Asn Gly Gly Asp Ala Tyr Phe Gly Gly Leu Met Asn Ser
                165                 170                 175

Ile Ile His Val Met Met Tyr Ser Tyr Tyr Thr Met Ala Leu Leu Gly
            180                 185                 190

Trp Ser Cys Pro Trp Lys Arg Tyr Leu Thr Gln Ala Gln Leu Val Gln
        195                 200                 205

Phe Cys Ile Cys Leu Ala His Ser Thr Trp Ala Ala Val Thr Gly Ala
    210                 215                 220

Tyr Pro Trp Arg Ile Cys Leu Val Glu Val Trp Val Met Val Ser Met
225                 230                 235                 240

Leu Val Leu Phe Thr Arg Phe Tyr Arg Gln Ala Tyr Ala Lys Glu Ala
                245                 250                 255

Lys Ala Lys Glu Ala Lys Lys Leu Ala Gln Glu Ala Ser Gln Ala Lys
            260                 265                 270

Ala Val Lys Ala Glu
        275

<210>  51
<211>  1542
<212>  DNA
<213>  Schizochytrium aggregatum

<400>  51
gaattcatga cggtgggcgg cgatgaggtg tacagcatgg cgcaggtgcg cgaccacaac      60
accccggacg acgcctggtg cgccatccac ggcgaggtgt acgagctgac caagttcgcc     120
cgcacccacc ccggggggga catcatcttg ctggccgccg gcaaggaggc caccatcctg     180
ttcgagacgt accacgtgcg ccccatctcc gacgcggtcc tgcgcaagta ccgcatcggc     240
aagctcgccg ccgccggcaa ggatgagccg gccaacgaca gcacctacta cagctgggac     300
agcgactttt acaaggtgct ccgccagcgt gtcgtggcgc gcctcgagga gcgcaagatc     360
gcccgccgcg gcggccccga gatctggatc aaggccgcca tcctcgtcag cggcttctgg     420
tccatgctct acctcatgtg caccctggac ccgaaccgcg gcgccatcct ggccgccatc     480
gcgctgggca tcgtcgccgc cttcgtcggc acgtgcattc agcacgacgg caaccacggc     540
gcgttcgcct tctctccgtt catgaacaag ctctctggct ggacgctcga catgatcggc     600
gccagtgcca tgacctggga gatgcagcac gtgctgggcc accacccgta caccaacctg     660
atcgagatgg agaacggcac ccaaaaggtc acccacgccg acgtcgaccc caagaaggcc     720
gaccaggaga gcgacccgga cgtcttcagc acctacccca tgctccgtct gcacccgtgg     780
caccgcaagc gcttctacca ccgcttccag cacctgtacg cgccgctgct cttcggtttc     840
atgaccatca acaaggtgat cacccaggat gtgggagttg tcctcagcaa gcgtctgttt     900
cagatcgatg ccaactgccg ttacgccagc aagtcgtacg ttgcgcgctt ctggatcatg     960
aagctgctca ccgtcctcta catggtcgcc ctccccgtgt acacccaggg ccttgtcgac    1020
gggctcaagc tcttcttcat cgcccacttt tcgtgcggcg agctgctggc caccatgttc    1080
atcgtcaacc acatcatcga gggcgtctcg tacgcctcca aggactctgt caagggcacc    1140
atggcgccgc cgcgcacggt gcacggcgtg accccgatgc atgacacccg cgacgcgctc    1200
ggcaaggaga aggcagccac caagcacgtg ccgctcaacg actgggccgc ggtccagtgc    1260
cagacctcgg tcaactggtc gatcggctcg tggttctgga accacttctc cggcgggctc    1320
aaccaccaga tcgagcacca cctcttcccc ggcctcaccc acaccaccta cgtgtacatt    1380
caggatgtgg tgcaggcgac gtgcgccgag tacggggtcc cgtaccagtc ggagcagagc    1440
ctcttctccg cctacttcaa gatgctctcc caccttcggg cgctcggcaa cgagccgatg    1500
ccctcgtggg agaaggacca ccccaagtcc aagtgaaagc tt                       1542

<210>  52

<211>  511
<212>  PRT
<213>  Schizochytrium aggregatum

<400>  52
Glu Phe Met Thr Val Gly Gly Asp Glu Val Tyr Ser Met Ala Gln Val
1               5                   10                  15

Arg Asp His Asn Thr Pro Asp Asp Ala Trp Cys Ala Ile His Gly Glu
            20                  25                  30

Val Tyr Glu Leu Thr Lys Phe Ala Arg Thr His Pro Gly Gly Asp Ile
        35                  40                  45

Ile Leu Leu Ala Ala Gly Lys Glu Ala Thr Ile Leu Phe Glu Thr Tyr
    50                  55                  60

His Val Arg Pro Ile Ser Asp Ala Val Leu Arg Lys Tyr Arg Ile Gly
65                  70                  75                  80

Lys Leu Ala Ala Ala Gly Lys Asp Glu Pro Ala Asn Asp Ser Thr Tyr
                85                  90                  95

Tyr Ser Trp Asp Ser Asp Phe Tyr Lys Val Leu Arg Gln Arg Val Val
            100                 105                 110

Ala Arg Leu Glu Glu Arg Lys Ile Ala Arg Arg Gly Gly Pro Glu Ile
        115                 120                 125

Trp Ile Lys Ala Ala Ile Leu Val Ser Gly Phe Trp Ser Met Leu Tyr
    130                 135                 140

Leu Met Cys Thr Leu Asp Pro Asn Arg Gly Ala Ile Leu Ala Ala Ile
145                 150                 155                 160

Ala Leu Gly Ile Val Ala Ala Phe Val Gly Thr Cys Ile Gln His Asp
                165                 170                 175

Gly Asn His Gly Ala Phe Ala Phe Ser Pro Phe Met Asn Lys Leu Ser
            180                 185                 190

Gly Trp Thr Leu Asp Met Ile Gly Ala Ser Ala Met Thr Trp Glu Met
        195                 200                 205

Gln His Val Leu Gly His His Pro Tyr Thr Asn Leu Ile Glu Met Glu
    210                 215                 220

Asn Gly Thr Gln Lys Val Thr His Ala Asp Val Asp Pro Lys Lys Ala
225                 230                 235                 240

Asp Gln Glu Ser Asp Pro Asp Val Phe Ser Thr Tyr Pro Met Leu Arg
                245                 250                 255

Leu His Pro Trp His Arg Lys Arg Phe Tyr His Arg Phe Gln His Leu
            260                 265                 270

Tyr Ala Pro Leu Leu Phe Gly Phe Met Thr Ile Asn Lys Val Ile Thr
        275                 280                 285

Gln Asp Val Gly Val Val Leu Ser Lys Arg Leu Phe Gln Ile Asp Ala
    290                 295                 300

Asn Cys Arg Tyr Ala Ser Lys Ser Tyr Val Ala Arg Phe Trp Ile Met
305                 310                 315                 320

Lys Leu Leu Thr Val Leu Tyr Met Val Ala Leu Pro Val Tyr Thr Gln
                325                 330                 335

Gly Leu Val Asp Gly Leu Lys Leu Phe Phe Ile Ala His Phe Ser Cys
            340                 345                 350

Gly Glu Leu Leu Ala Thr Met Phe Ile Val Asn His Ile Ile Glu Gly
        355                 360                 365

Val Ser Tyr Ala Ser Lys Asp Ser Val Lys Gly Thr Met Ala Pro Pro
    370                 375                 380

Arg Thr Val His Gly Val Thr Pro Met His Asp Thr Arg Asp Ala Leu
385                 390                 395                 400

Gly Lys Glu Lys Ala Ala Thr Lys His Val Pro Leu Asn Asp Trp Ala
                405                 410                 415

Ala Val Gln Cys Gln Thr Ser Val Asn Trp Ser Ile Gly Ser Trp Phe
            420                 425                 430

Trp Asn His Phe Ser Gly Gly Leu Asn His Gln Ile Glu His His Leu
        435                 440                 445

Phe Pro Gly Leu Thr His Thr Thr Tyr Val Tyr Ile Gln Asp Val Val
    450                 455                 460

Gln Ala Thr Cys Ala Glu Tyr Gly Val Pro Tyr Gln Ser Glu Gln Ser
465                 470                 475                 480

Leu Phe Ser Ala Tyr Phe Lys Met Leu Ser His Leu Arg Ala Leu Gly
                485                 490                 495

Asn Glu Pro Met Pro Ser Trp Glu Lys Asp His Pro Lys Ser Lys
            500                 505                 510

<210>  53
<211>  27
<212>  DNA
<213>  Artificial Sequence

<220>
<223>  synthetic oligonucleotide

<400>  53
gcggccgcat gactgaggat aagacga                                          27

<210>  54
<211>  27
<212>  DNA
<213>  Artificial Sequence

<220>
<223>  synthetic oligonucleotide

<400>  54
gcggccgctt agtccgactt ggccttg                                          27

<210>  55
<211>  24
<212>  DNA
<213>  Artificial Sequence

<220>
<223>  synthetic oligonucleotide

<400>  55
gcggccgcat ggagtcgatt gcgc                                              24

<210>  56
<211>  24
<212>  DNA
<213>  Artificial Sequence

<220>
<223>  synthetic oligonucleotide

<400>  56
gcggccgctt actgcaactt cctt                                              24

<210>  57
<211>  24
<212>  DNA
<213>  Artificial Sequence

<220>
<223>  synthetic oligonucleotide

<400>  57
gcggccgcat gggaacggac caag                                              24

<210>  58
<211>  24
<212>  DNA
<213>  Artificial Sequence

<220>
<223>  synthetic oligonucleotide

<400>  58
gcggccgcct actcttcctt ggga                                              24

<210>  59
<211>  29
<212>  DNA
<213>  Artificial Sequence

<220>
<223>  synthetic oligonucleotide

<400>  59
ttcctgcagg ctagcctaag tacgtactc                                         29

<210>  60
<211>  21
<212>  DNA
<213>  Artificial Sequence

<220>
<223>  synthetic oligonucleotide

<400>  60
aagcggccgc ggtgatgact g                                                 21

<210>  61
<211>  12
<212>  PRT
<213>  Artificial Sequence

<220>

<223>  consensus peptide

<400>  61
Thr Arg Ala Ala Ile Pro Lys His Cys Trp Val Lys
1               5                   10

<210>  62
<211>  36
<212>  DNA
<213>  Artificial Sequence

<220>
<223>  synthetic oligonucleotide

<400>  62
atccgcgccg ccatccccaa gcactgctgg gtcaag                                36

<210>  63
<211>  15
<212>  PRT
<213>  Artificial Sequence

<220>
<223>  consensus peptide

<400>  63
Ala Leu Phe Val Leu Gly His Asp Cys Gly His Gly Ser Phe Ser
1               5                   10                  15

<210>  64
<211>  45
<212>  DNA
<213>  Artificial Sequence

<220>
<223>  synthetic oligonucleotide

<220>
<221>  unsure
<222>  (21)..(21)
<223>  y = c or t

<220>
<221>  unsure
<222>  (33)..(33)
<223>  y = c or t

<400>  64
gccctcttcg tcctcggcca ygactgcggc cayggctcgt tctcg                      45

<210>  65
<211>  45
<212>  DNA
<213>  Artificial Sequence

<220>
<223>  synthetic oligonucleotide

<220>
<221>  unsure
<222>  (4)..(4)
<223>  r = a or g

<220>
<221>  unsure

<222>  (10)..(10)
<223>  r = a or g

<220>
<221>  unsure
<222>  (30)..(30)
<223>  r = a or g

<220>
<221>  unsure
<222>  (31)..(31)
<223>  r = a or g

<220>
<221>  unsure
<222>  (34)..(34)
<223>  r = a or g

<220>
<221>  unsure
<222>  (38)..(38)
<223>  r = a or g

<220>
<221>  unsure
<222>  (39)..(39)
<223>  y = c or t

<220>
<221>  unsure
<222>  (43)..(43)
<223>  r = a or g

<400>  65
gagrtggtar tgggggatct gggggaagar rtgrtggryg acrtg                    45

<210>  66
<211>  15
<212>  PRT
<213>  Artificial Sequence

<220>
<223>  consensus peptide

<400>  66
Pro Tyr His Gly Trp Arg Ile Ser His Arg Thr His His Gln Asn
1               5                   10                  15

<210>  67
<211>  45
<212>  DNA
<213>  Artificial Sequence

<220>
<223>  synthetic oligonucleotide

<220>
<221>  unsure
<222>  (9)..(9)
<223>  y = c or t

<220>
<221>  unsure
<222>  (27)..(27)
<223>  y = c or t

<220>
<221>  unsure
<222>  (36)..(36)
<223>  y = c or t

<220>
<221>  unsure
<222>  (39)..(39)
<223>  y = c or t

<400>  67
ccctaccayg gctggcgcat ctcgcaycgc acccaycayc agaac                    45

<210>  68
<211>  45
<212>  DNA
<213>  Artificial Sequence

<220>
<223>  synthetic oligonucleotide

<220>
<221>  unsure
<222>  (7)..(7)
<223>  r = a or g

<220>
<221>  unsure
<222>  (10)..(10)
<223>  r = a or g

<220>
<221>  unsure
<222>  (19)..(19)
<223>  r = a or g

<220>
<221>  unsure
<222>  (37)..(37)
<223>  r = a or g

<400>  68
gttctgrtgr tgggtccgrt gcgagatgcg ccagccrtgg taggg                    45

<210>  69
<211>  12
<212>  PRT
<213>  Artificial Sequence

<220>
<223>  consensus peptide

<220>
<221>  UNSURE
<222>  (5)..(5)
<223>  Xaa = Asp or His

<220>
<221>  UNSURE
<222>  (7)..(7)
<223>  Xaa = Asp or Tyr

<400>  69
Gly Ser His Phe Xaa Pro Xaa Ser Asp Leu Phe Val

1                    5                        10

<210>  70
<211>  36
<212>  DNA
<213>  Artificial Sequence

<220>
<223>  synthetic oligonucleotide

<220>
<221>  unsure
<222>  (13)..(13)
<223>  s = c or g

<220>
<221>  unsure
<222>  (19)..(19)
<223>  k = g or t

<400>  70
ggctcgcact tcsaccccka ctcggacctc ttcgtc                                    36

<210>  71
<211>  36
<212>  DNA
<213>  Artificial Sequence

<220>
<223>  synthetic oligonucleotide

<220>
<221>  unsure
<222>  (18)..(18)
<223>  m = a or c

<220>
<221>  unsure
<222>  (24)..(24)
<223>  w = a or t

<400>  71
gacgaagagg tccgagtmgg ggtwgaagtg cgagcc                                    36

<210>  72
<211>  13
<212>  PRT
<213>  Artificial Sequence

<220>
<223>  consensus peptide

<220>
<221>  UNSURE
<222>  (3)..(3)
<223>  Xaa = Tyr or Phe

<220>
<221>  UNSURE
<222>  (4)..(4)
<223>  Xaa = Leu or Val

<220>
<221>  UNSURE
<222>  (11)..(11)

<223>  Xaa = Leu or Ile

<400>  72
Trp Ser Xaa Xaa Arg Gly Gly Leu Thr Thr Xaa Asp Arg
1               5                   10

<210>  73
<211>  39
<212>  DNA
<213>  Artificial Sequence

<220>
<223>  synthetic oligonucleotide

<220>
<221>  unsure
<222>  (9)..(9)
<223>  k = g or t

<220>
<221>  unsure
<222>  (30)..(30)
<223>  w = a or t

<220>
<221>  unsure
<222>  (32)..(32)
<223>  s = c or g

<400>  73
gcgctggakg gtggtgaggc cgccgcggaw gsacgacca                              39

<210>  74
<211>  15
<212>  PRT
<213>  Artificial Sequence

<220>
<223>  consensus peptide

<400>  74
His His Asp Ile Gly Thr His Val Ile His His Leu Phe Pro Gln
1               5                   10                  15

<210>  75
<211>  45
<212>  DNA
<213>  Artificial Sequence

<220>
<223>  synthetic oligonucleotide

<220>
<221>  unsure
<222>  (13)..(13)
<223>  r = a or g

<220>
<221>  unsure
<222>  (16)..(16)
<223>  r = a or g

<220>
<221>  unsure
<222>  (25)..(25)

<223>  r = a or g

<220>
<221>  unsure
<222>  (40)..(40)
<223>  r = a or g

<220>
<221>  unsure
<222>  (43)..(43)
<223>  r = a or g

<400>  75
ctgggggaag agrtgrtgga tgacrtgggt gccgatgtcr tgrtg                    45

<210>  76
<211>  15
<212>  PRT
<213>  Artificial Sequence

<220>
<223>  consensus peptide

<220>
<221>  UNSURE
<222>  (2)..(2)
<223>  Xaa = Leu or Phe

<220>
<221>  UNSURE
<222>  (5)..(5)
<223>  Xaa = Gln or Lys

<220>
<221>  UNSURE
<222>  (12)..(12)
<223>  Xaa = Val or Ile

<400>  76
His Xaa Phe Pro Xaa Ile Pro His Tyr His Leu Xaa Glu Ala Thr
1               5                   10                  15

<210>  77
<211>  45
<212>  DNA
<213>  Artificial Sequence

<220>
<223>  synthetic oligonucleotide

<220>
<221>  unsure
<222>  (12)..(12)
<223>  y = c or t

<220>
<221>  unsure
<222>  (16)..(16)
<223>  r = a or g

<220>
<221>  unsure
<222>  (22)..(22)
<223>  r = a or g

<220>
<221>  unsure
<222>  (33)..(33)
<223>  k = g or t

<220>
<221>  unsure
<222>  (42)..(42)
<223>  r = a or g

<220>
<221>  unsure
<222>  (43)..(43)
<223>  r = a or g

<400>  77
ggtggcctcg aygagrtggt artgggggat ctkggggaag arrtg                    45

<210>  78
<211>  15
<212>  PRT
<213>  Artificial Sequence

<220>
<223>  consensus peptide

<220>
<221>  UNSURE
<222>  (3)..(3)
<223>  Xaa = Ala or Ile

<220>
<221>  UNSURE
<222>  (6)..(6)
<223>  Xaa = Leu or Phe

<400>  78
His Val Xaa His His Xaa Phe Pro Gln Ile Pro His Tyr His Leu
1               5                   10                  15

<210>  79
<211>  25
<212>  DNA
<213>  Artificial Sequence

<220>
<223>  synthetic oligonucleotide

<400>  79
tacgcgtacc tcacgtactc gctcg                                          25

<210>  80
<211>  27
<212>  DNA
<213>  Artificial Sequence

<220>
<223>  synthetic oligonucleotide

<400>  80
ttcttgcacc acaacgacga agcgacg                                        27

<210>  81
<211>  25
<212>  DNA

<213>  Artificial Sequence

<220>
<223>  synthetic oligonucleotide

<400>  81
ggagtggacg tacgtcaagg gcaac                                             25

<210>  82
<211>  26
<212>  DNA
<213>  Artificial Sequence

<220>
<223>  synthetic oligonucleotide

<400>  82
tcaagggcaa cctctcgagc gtcgac                                            26

<210>  83
<211>  31
<212>  DNA
<213>  Artificial Sequence

<220>
<223>  synthetic oligonucleotide

<400>  83
cccagtcacg acgttgtaaa acgacggcca g                                      31

<210>  84
<211>  30
<212>  DNA
<213>  Artificial Sequence

<220>
<223>  synthetic oligonucleotide

<400>  84
agcggataac aatttcacac aggaaacagc                                        30
<210>  85
<211>  30
<212>  DNA
<213>  Artificial Sequence

<220>
<223>  synthetic oligonucleotide

<400>  85
ggtaaaagat ctcgtccttg tcgatgttgc                                        30
<210>  86
<211>  20
<212>  DNA
<213>  Artificial Sequence

<220>
<223>  synthetic oligonucleotide

<400>  86
gtcaaagtgg ctcatcgtgc                                                   20
<210>  87
<211>  26
<212>  DNA
<213>  Artificial Sequence

<220>
<223>  synthetic oligonucleotide

<400>  87
cgagcgagta cgtgaggtac gcgtac                                          26

<210>  88
<211>  45
<212>  DNA
<213>  Artificial Sequence

<220>
<223>  synthetic oligonucleotide

<400>  88
tcaacagaat tcatgaccga ggataagacg aaggtcgagt tcccg                     45

<210>  89
<211>  45
<212>  DNA
<213>  Artificial Sequence

<220>
<223>  synthetic oligonucleotide

<400>  89
aaaagaaagc ttcgcttcct agtcttagtc cgacttggcc ttggc                     45

<210>  90
<211>  3979
<212>  DNA
<213>  Glycine max

<400>  90
ggccgcagat ttaggtgaca ctatagaata tgcatcacta gtaagctttg ctctagatca      60
aactcacatc caaacataac atggatatct tccttaccaa tcatactaat tattttgggt     120
taaatattaa tcattatttt taagatatta attaagaaat taaaagattt tttaaaaaaa     180
tgtataaaat tatattattc atgatttttc atacatttga ttttgataat aaatatattt     240
tttttaattt cttaaaaaat gttgcaagac acttattaga catagtcttg ttctgtttac     300
aaaagcattc atcatttaat acattaaaaa atatttaata ctaacagtag aatcttcttg     360
tgagtggtgt gggagtaggc aacctggcat tgaaacgaga gaaagagagt cagaaccaga     420
agacaaataa aaagtatgca acaaacaaat caaaatcaaa gggcaaaggc tggggttggc     480
tcaattggtt gctacattca attttcaact cagtcaacgg ttgagattca ctctgacttc     540
cccaatctaa gccgcggatg caaacggttg aatctaaccc acaatccaat ctcgttactt     600
aggggctttt ccgtcattaa ctcacccctg ccacccggtt tccctataaa ttggaactca     660
atgctcccct ctaaactcgt atcgcttcag agttgagacc aagacacact cgttcatata     720
tctctctgct cttctcttct cttctacctc tcaaggtact tttcttctcc ctctaccaaa     780
tcctagattc cgtggttcaa tttcggatct tgcacttctg gtttgctttg ccttgctttt     840
tcctcaactg ggtccatcta ggatccatgt gaaactctac tctttcttta atatctgcgg     900
aatacgcgtt ggactttcag atctagtcga aatcatttca taattgcctt tctttctttt     960
agcttatgag aaataaaatc actttttttt tatttcaaaa taaaccttgg gccttgtgct    1020
gactgagatg gggtttggtg attacagaat tttagcgaat tttgtaattg tacttgtttg    1080
tctgtagttt tgttttgttt tcttgtttct catacattcc ttaggcttca attttattcg    1140
agtataggtc acaataggaa ttcaaacttt gagcagggga attaatccct tccttcaaat    1200
ccagtttgtt tgtatatatg tttaaaaaat gaaacttttg ctttaaattc tattataact    1260
ttttttatgg ctgaaatttt tgcatgtgtc tttgctctct gttgtaaatt tactgtttag    1320
gtactaactc taggcttgtt gtgcagtttt tgaagtataa ccatgccaca caacacaatg    1380
gcggccaccg cttccagaac cacccgattc tcttcttcct cttcacaccc caccttcccc    1440
aaacgcatta ctagatccac cctccctctc tctcatcaaa ccctcaccaa acccaaccac    1500
gctctcaaaa tcaaatgttc catctccaaa ccccccacgg cggcgccctt caccaaggaa    1560
gcgccgacca cggagccctt cgtgtcacgg ttcgcctccg gcgaacctcg caagggcgcg    1620
gacatccttg tggaggcgct ggagaggcag ggcgtgacga cggtgttcgc gtaccccggc    1680
ggtgcgtcga tggagatcca ccaggcgctc acgcgctccg ccgccatccg caacgtgctc    1740
ccgcgccacg agcagggcgg cgtcttcgcc gccgaaggct acgcgcgttc ctccggcctc    1800
cccggcgtct gcattgccac ctccggcccc ggcgccacca acctcgtgag cggcctcgcc    1860

gacgctttaa tggacagcgt cccagtcgtc gccatcaccg gccaggtcgc ccgccggatg 1920
atcggcaccg acgccttcca agaaaccccg atcgtggagg tgagcagatc catcacgaag 1980
cacaactacc tcatcctcga cgtcgacgac atcccccgcg tcgtcgccga ggctttcttc 2040
gtcgccacct ccggccgccc cggtccggtc ctcatcgaca ttcccaaaga cgttcagcag 2100
caactcgccg tgcctaattg ggacgagccc gttaacctcc ccggttacct cgccaggctg 2160
cccaggcccc ccgccgaggc ccaattggaa cacattgtca gactcatcat ggaggcccaa 2220
aagcccgttc tctacgtcgg cggtggcagt ttgaattcca gtgctgaatt gaggcgcttt 2280
gttgaactca ctggtattcc cgttgctagc actttaatgg gtcttggaac ttttcctatt 2340
ggtgatgaat attcccttca gatgctgggt atgcatggta ctgtttatgc taactatgct 2400
gttgacaata gtgatttgtt gcttgccttt ggggtaaggt ttgatgaccg tgttactggg 2460
aagcttgagg cttttgctag tagggctaag attgttcaca ttgatattga ttctgccgag 2520
attgggaaga acaagcaggc gcacgtgtcg gtttgcgcgg atttgaagtt ggccttgaag 2580
ggaattaata tgattttgga ggagaaagga gtggagggta agtttgatct tggaggttgg 2640
agagaagaga ttaatgtgca gaaacacaag tttccattgg gttacaagac attccaggac 2700
gcgatttctc cgcagcatgc tatcgaggtt cttgatgagt tgactaatgg agatgctatt 2760
gttagtactg gggttgggca gcatcaaatg tgggctgcgc agttttacaa gtacaagaga 2820
ccgaggcagt ggttgacctc agggggtctt ggagccatgg gttttggatt gcctgcggct 2880
attggtgctg ctgttgctaa ccctggggct gttgtggttg acattgatgg ggatggtagt 2940
ttcatcatga atgttcagga gttggccact ataagagtgg agaatctccc agttaagata 3000
ttgttgttga acaatcagca tttgggtatg gtggttcagt tggaggatag gttctacaag 3060
tccaatagag ctcacaccta tcttggagat ccgtctagcg agagcgagat attcccaaac 3120
atgctcaagt ttgctgatgc ttgtgggata ccggcagcgc gagtgacgaa gaaggaagag 3180
cttagagcgg caattcagag aatgttggac acccctggcc cctaccttct tgatgtcatt 3240
gtgccccatc aggagcatgt gttgccgatg attcccagta atggatcctt caaggatgtg 3300
ataactgagg gtgatggtag aacgaggtac tgattgccta gaccaaatgt tccttgatgc 3360
ttgttttgta caatatatat aagataatgc tgtcctagtt gcaggatttg gcctgtggtg 3420
agcatcatag tctgtagtag ttttggtagc aagacatttt attttccttt tatttaactt 3480
actacatgca gtagcatcta tctatctctg tagtctgata tctcctgttg tctgtattgt 3540
gccgttggat tttttgctgt agtgagactg aaaatgatgt gctagtaata atatttctgt 3600
tagaaatcta agtagagaat ctgttgaaga agtcaaaagc taatggaatc aggttacata 3660
tcaatgtttt tcttttttta gcggttggta gacgtgtaga ttcaacttct cttggagctc 3720
acctaggcaa tcagtaaaat gcatattcct tttttaactt gccatttatt tacttttagt 3780
ggaaattgtg accaatttgt tcatgtagaa cggatttgga ccattgcgtc cacaaaacgt 3840
ctcttttgct cgatcttcac aaagcgatac cgaaatccag agatagtttt caaaagtcag 3900
aaatggcaaa gttataaata gtaaaacaga atagatgctg taatcgactt caataacaag 3960
tggcatcacg tttctagtt 3979

<210> 91
<211> 17
<212> DNA
<213> Artificial Sequence

<220>
<223> synthetic oligonucleotide

<400> 91
tgcggccgca tgagccg 17

<210> 92
<211> 32
<212> DNA
<213> Artificial Sequence

<220>
<223> synthetic oligonucleotide

<400> 92
acgtacggta ccatctgcta atattttaaa tc 32

<210> 93
<211> 20
<212> DNA
<213> Artificial Sequence

<220>

<223>  synthetic oligonucleotide

<400>  93
taatacgact cactattagg                                                    20
<210>  94
<211>  35
<212>  DNA
<213>  Artificial Sequence

<220>
<223>  synthetic oligonucleotide

<400>  94
tgcccatgat gttggccgca ggctatcttc tagtg                                   35

<210>  95
<211>  25
<212>  DNA
<213>  Artificial Sequence

<220>
<223>  synthetic oligonucleotide

<400>  95
gctgtcaacg atacgctacg taacg                                              25

<210>  96
<211>  25
<212>  DNA
<213>  Artificial Sequence

<220>
<223>  synthetic oligonucleotide

<400>  96
gccaattgga gcgagttcca atctc                                              25

<210>  97
<211>  28
<212>  DNA
<213>  Artificial Sequence

<220>
<223>  synthetic oligonucleotide

<400>  97
gcgatatccg tttcttctga ccttcatc                                           28

<210>  98
<211>  28
<212>  DNA
<213>  Artificial Sequence

<220>
<223>  synthetic oligonucleotide

<400>  98
ttctagacct gcaggatata atgagccg                                           28

## Description

This application claims the benefit of U.S. Provisional Application No. 60/446,941, filed February 12, 2003, the disclosure of which is hereby incorporated by reference in its entirety.

### FIELD OF THE INVENTION

This invention pertains to oilseed plants which have been transformed to produce very long chain polyunsaturated fatty acids and to recombinant constructs and method for producing such fatty acids in a plant.

### BACKGROUND OF THE INVENTION

Lipids/fatty acids are water-insoluble organic biomolecules that can be extracted from cells and tissues by nonpolar solvents such as chloroform, ether or benzene. Lipids have several important biological functions, serving (1) as structural components of membranes, (2) as storage and transport forms of metabolic fuel, (3) as a protective coating on the surface of many organisms, and (4) as celll-surface components concerned in cell recognition, species specificity and tissue immunity.

The human body is capable of producing most of the fatty acids which it requires to function. Two long chain polyunsaturated fatty acids, eicosapentaenoic acid (EPA) and docosahexaenoic acid (DHA), however, cannot be synthesized efficiently by the human body and, thus, have to be supplied through the diet. Since the human body cannot produce adequate quantities of these polyunsaturated fatty acids, they are called essential fatty acids.

PUFAs are important components of the plasma membrane of the cell, where they may be found in such forms as phospholipids and also can be found in triglycerides. PUFAs also serve as precursors to other molecules of importance in human beings and animals, including the prostacyclins, leukotrienes and prostaglandins. There are two main families of polyunsaturated fatty acids (PUFAs), specifically, the omega-3 fatty acids and the omega-6 fatty acids.

DHA is a fatty acid of the omega-3 series according to the location of the last double bond in the methyl end. It is synthesized via alternating steps of desaturation and elongation. Production of DHA is important because of its beneficial effect on human health. Currently the major sources of DHA are oils from fish and algae.

EPA and arachidonic acid (AA) are both delta-5 essential fatty acids. EPA belongs to the omega-3 series with five double bonds in the acyl chain, is found in marine food, and is abundant in oily fish from the North Atlantic. AA belongs to the omega-6 series with four double bonds. The lack of a double bond in the omega-3 position confers on AA different properties than those found in EPA. The eicosanoids produced from AA have strong inflammatory and platelet aggregating properties, whereas those derived from EPA have anti-inflammatory and antiplatelet aggregating properties. AA can be obtained from some foods such as meat, fish, and eggs, but the concentration is low.

Gamma-linolenic acid (GLA) is another essential fatty acid found in mammals. GLA is the metabolic intermediate for very long chain omega-6 fatty acids and for various active molecules. In mammals, formation of long chain PUFAs is rate-limited by delta-6 desaturation. Many physiological and pathological conditions such as aging, stress, diabetes, eczema, and some infections have been shown to depress the delta-6 desaturation step. In addition, GLA is readily catabolized from the oxidation and rapid cell division associated with certain disorders, e.g., cancer or inflammation.

Research has shown that omega-3 fatty acids reduce the risk of heart disease as well as having a positive effect on children's development. Results have been disclosed indicating the positive effect of these fatty acids on certain mental illnesses, autoimmune diseases and joint complaints. Thus, there are many health benefits associated with a diet supplemented with these fatty acids.

Unfortunately, there are several disadvantages associated with commercial production of PUFAs from natural sources. Natural sources of PUFAs, such as animals and plants, tend to have highly heterogeneous oil compositions. The oils obtained from these sources can require extensive purification to separate out one or more desired PUFAs or to produce an oil which is enriched in one or more PUFAs. Natural sources also are subject to uncontrollable fluctuations in availability. Fish stocks may undergo natural variation or may be depleted by overfishing. Fish oils have unpleasant tastes and odors which may be difficult, if not impossible, to economically separate from the desired product, and can render such products unacceptable as food supplements. Animal oils and, in particular, fish oils, can accumulate envrionmental pollutants. Weather and disease can cause fluctuation in yields from both fish and plant sources.

An expansive supply of polyunsaturated fatty acids from natural sources and from chemical syntheis are not sufficient for commercial needs. Therefore, it is of interest to find alternative means to allow production of commercial quantities of PUFAs. Biotechnology offers an attractive route for producing LCPUFAs in a safe, cost efficient manner.

WO 02/26946, published April 4, 2002, describes isolated nucleic acid molecules encoding FAD4, FAD5, FAD5-2 and FAD6 fatty acid desaturase family members which are expressed in LCPUFA-producing organisms, e.g., *Thraustochytrium, Pythium irregulare, Schizichytrium* and *Crypthecodinium.* It is indicated that constructs containing the desaturase genes can be used in any expression system including plants, animals, and microorganisms for the production of cells capable of producing LCPUFAs.

WO 02/26946, published April 4, 2002, describes FAD4, FAD5, FAD5-2, and FAD6 fatty acid desaturase members and uses thereof to produce long chain polyunsaturated fatty acids.

WO 98/55625, published December 19, 1998, describes the production of polyunsaturated fatty acids by expression of polyketide-like synthesis genes in plants.

WO 98/46764, published October 22, 1998, describes compositions and methods for preparing long chain fatty acids in plants, plant parts and plant cells which utilize nucleic acid sequences and constructs encoding fatty acid desaturases, including delta-5 desaturases, delta-6 desaturases and delta-12 desaturases.

U.S. Patent No. 6,075,183, issued to Knutzon et al. on June 13, 2000, describes methods and compositions for synthesis of long chain polyunsaturated fatty acids in plants.

U.S. Patent No. 6,459,018, issued to Knutzon on October 1, 2002, describes a method for producing stearidonic acid in plant seed utilizing a construct comprising a DNA sequence encoding a delta-six desaturase.

Spychalla et al., Proc. Natal. Acad. Sci. USA, Vol.94, 1142-1147 (Feb. 1997), describes the isolation and characterization of a cDNA from *C. elegans* that, when expressed in *Arabidopsis*, encodes a fatty acid desaturase which can catalyze the introduction of an omega-3 double bond into a range of 18- and 20-carbon fatty acids.

### SUMMARY OF THE INVENTION

The invention includes an oilseed plant that produces mature seeds in which the total seed fatty acid profile comprises at least 1.0 % of at least one polyunsaturated fatty acid having at least twenty carbon atoms and five or more carbon-carbon double bonds.

In a second embodiment, this invention includes an oilseed plant that produces mature seeds in which the total seed fatty acid profile comprises at least 5.0 % of at least one polyunsaturated fatty acid having at least twenty carbon atoms and five or more carbon-carbon double bonds.

In a third embodiment, this invention includes an oilseed plant that produces mature seeds in which the total seed fatty acid profile comprises at least 10.0 % of at least one polyunsaturated fatty acid having at least twenty carbon atoms and five or more carbon-carbon double bonds.

Additional embodiments of this invention include an oilseed plant that produces mature seeds in which the total seed fatty acid profile comprises at least 15.0 %, 20 %, 25 %, 30 %, 40 %, 50 %, or 60 % of at least one polyunsaturated fatty acid having at least twenty carbon atoms and five or more carbon-carbon double bonds.

In a fourth embodiment this invention includes an oilseed plant that produces mature seeds in which the total seed fatty acid profile comprises at least 10.0 % of at least one polyunsaturated fatty acid having at least twenty carbon atoms and five or more carbon-carbon double bonds and less than 2.0% arachidonic acid.

Again additional embodiments would include an oilseed plant that produces mature seeds in which the total seed fatty acid profile comprises at least 15.0 %, 20 %, 25 %, 30 %, 40 %, 50 %, or 60 % of at least one polyunsaturated fatty acid having at least twenty carbon atoms and five or more carbon-carbon double bonds and less than 2.0 % arachidonic acid.

The PUFA can be an omega-3 fatty acid selected from the group consisting of EPA, DPA and DHA.

Also of interest are seeds obtained from such plants and oil obtained from the seeds of such plants.

In a fifth embodiment, this invention includes a recombinant construct for altering the total fatty acid profile of mature seeds of an oilseed plant, said construct comprising at least two promoters wherein each promoter is operably linked to a nucleic acid sequence encoding a polypeptide required for making at least one polyunsaturated fatty acid having at least twenty carbon atoms and four or more carbon-carbon double bonds and further wherein the total fatty acid profile comprises at least 2 % of at least one polyunsaturated fatty acid having at least twenty carbon atoms and four or more carbon-carbon double bonds and further wherein said polypeptide is an enzyme selected from the group consisting of a Δ4 desaturase, a Δ5 desaturase, a Δ6 desaturase, a Δ5 desaturase, a Δ17 desaturase, a C18 to C22 elongase and a C20 to C24 elongase.

In a further aspect, the promoter is selected from the group consisting of the alpha prime subunit of beta conglycinin promoter, Kunitz trypsin inhibitor 3 promoter, annexin promoter, Gly1 promoter, beta subunit of beta conglycinin promoter, P34/Gly Bd m 30K promoter, albumin promoter, Leg A1 promoter and Leg A2 promoter. Also of interests are oilseed plants comprising in their genome such recombinant constructs, seeds obtained from such plants and oil obtained from the seeds of such plants.

In a sixth embodiment, this invention includes a method for making an oilseed plant having an altered fatty acid profile which comprises:
a) transforming a plant with the recombinant construct of the fifth embodiment;
b) growing the transformed plant of step (a); and
c) selecting those plants wherein the total fatty acid profile comprises at least 1.0 % of at least one polyunsaturated fatty acid having at least twenty carbon atoms and five or more carbon-carbon double bonds.

In a seventh embodiment, this invention includes a method for making an oilseed plant having an altered fatty acid profile which comprises:
a) transforming a plant with the recombinant construct of the fifth embodiment including any one of the promoters recited therein,
b) growing the transformed plant of step (a); and
c) selecting those plants wherein the total fatty acid profile comprises at least 1.0 % of at least one polyunsaturated fatty acid having at least twenty carbon atoms and five or more carbon-carbon double bonds.

Also of interest are oilseed plants made by such methods, seeds obtained from such plants and oil obtained from the seeds of such plants.

In an eighth embodiment, this invention includes a food product, beverage, infant formula, or nutritional supplement incorporating any of the oils of the invention.

In a ninth embodiment, this invention includes a food product, pet food or animal feed which has incorporated therein any of the seeds of the invention.

In a tenth embodiment, this invention includes an oilseed plant that produces mature seeds in which the total seed fatty acid profile comprises polyunsaturated fatty acids having at least twenty carbon atoms and five or more carbon-carbon double bonds wherein the ratio of EPA:DHA is in the range from 1:100 to 860:100. The oilseed plant may further have a total seed fatty acid profile comprising less than 2.0% arachidonic acid. Also of interest are seeds obtained from such plants and oil obtained from the seeds of such plants.

In an eleventh embodiment, this invention includes an oilseed plant that produces mature seeds in which the total seed fatty acid profile comprises polyunsaturated fatty acids having at least twenty carbon atoms and five or more carbon-carbon double bonds wherein the ratio of DHA:EPA is in the range from 1:100 to 110:100. The oilseed plant may further have a total seed fatty acid profile comprising less than 2.0% arachidonic acid. Also of interest are seeds obtained from such plants and oil obtained from the seeds of such plants.

### BIOLOGICAL DEPOSITS

The following plasmids have been deposited with the American Type Culture Collection (ATCC), 10801 University Boulevard, Manassas, VA 20110-2209, and bears the following designation, accession number and date of deposit.

| Plasmid | Accession Number | Date of Deposit |
|---|---|---|
| pKR274 | ATCC PTA-4988 | Jan. 30, 2003 |
| pKR275 | ATCC PTA-4989 | Jan. 30, 2003 |
| pKR357 | ATCC PTA-4990 | Jan. 30, 2003 |
| pKR365 | ATCC PTA-4991 | Jan. 30, 2003 |
| pKKE2 | ATCC PTA-4987 | Jan. 30, 2003 |

### BRIEF DESCRIPTION OF THE DRAWINGS AND SEQUENCE LISTINGS

The invention can be more fully understood from the following detailed description and the accompanying drawings and Sequence Listing, which form a part of this application.

The sequence descriptions summarize the Sequences Listing attached hereto. The Sequence Listing contains one letter codes for nucleotide sequence characters and the single and three letter codes for amino acids as defined in the IUPAC-IUB standards described in Nucleic Acids Research 13:3021-3030 (1985) and in the Biochemical Journal 219 (No. 2):345-373 (1984).

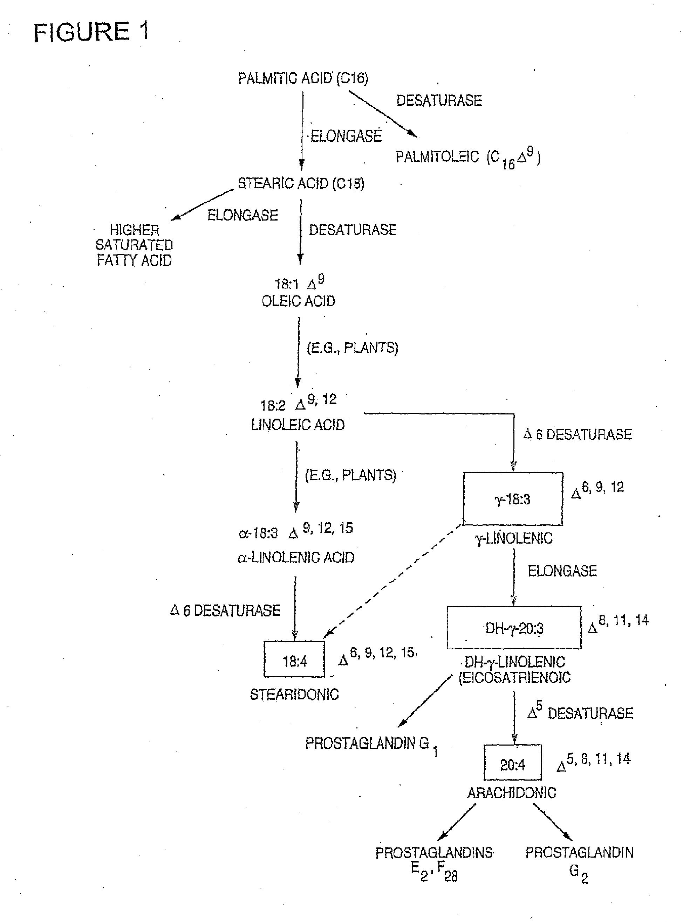

Figure 1 shows possible biosynthetic pathways for PUFAs.

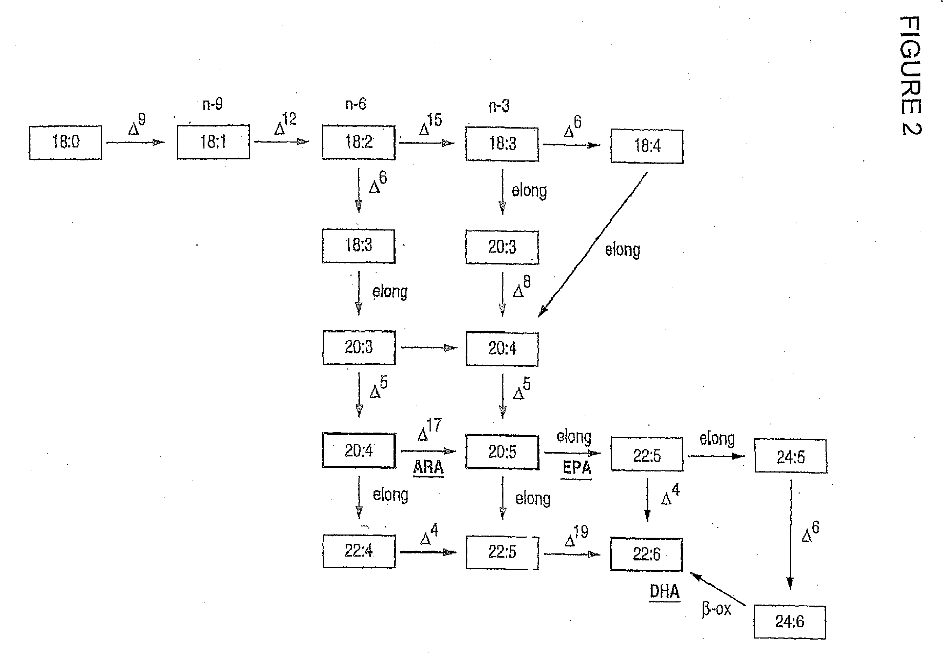

Figure 2 shows possible pathways for production of LC-PUFAs included EPA and DHA compiled from a variety of organisms.
Figure 3 is a schematic depiction of plasmid pKR274.
Figure 4 is a schematic depiction of plasmid pKKE2.
Figure 5 is a schematic depiction of plasmid pKR275.
Figure 6 is a schematic depiction of plasmid pKR365.
Figure 7 is a schematic depiction of plasmid pKR364.
Figure 8 is a schematic depiction of plasmid pKR357.

SEQ. ID. NO:1 sets forth oligonucleotide primer GSP1 used to amplify the soybean annexin promoter.

SEQ. ID. NO:2 sets forth oligonucleotide primer GSP2 used to amplify the soybean annexin promoter.

SEQ. ID. NO:3 sets forth the sequence of the annexin promoter.

SEQ. ID. NO:4 sets forth oligonucleotide primer GSP3 used to amplify the soybean BD30 promoter.

SEQ. ID. NO:5 sets forth oligonucleotide primer GSP4 used to amplify the soybean BD30 promoter.

SEQ. ID. NO:6 sets forth the sequence of the soybean BD30 promoter.

SEQ. ID. NO:7 sets forth the sequence of the soybean β-conglycinin β-subunit promoter.

SEQ. ID. NO:8 sets forth oligonucleotide primer β-con oligo Bam used to amplify the promoter for soybean β-conglycinin β-subunit.

SEQ. ID. NO:9 sets forth oligonucleotide primer β-con oligo Not used to amplify the promoter for soybean β-conglycinin β-subunit.

SEQ. ID. NO:10 sets forth the sequence of the soybean glycinin Gly-1 promoter.

SEQ. ID. NO:11 sets forth oligonucleotide primer glyoligo Bam used to amplify the Gly-1 promoter.

SEQ. ID. NO:12 sets forth oligonucleotide primer glyoligo Not used to amplify the Gly-1 promoter.

SEQ. ID. NO:13 sets forth oligonucleotide primer oCGR5-1.

SEQ. ID. NO:14 sets forth oligonucleotide primer oCGR5-2.

SEQ. ID. NO:15 sets forth oligonucleotide primer oSAlb-9.

SEQ. ID. NO:16 sets forth oligonucleotide primer oSAlb-3.

SEQ. ID. NO:17 sets forth oligonucleotide primer oSAlb-4.

SEQ. ID. NO:18 sets forth oligonucleotide primer oSAlb-2.

SEQ. ID. NO:19 sets forth oligonucleotide primer LegPro5'.

SEQ. ID. NO:20 sets forth oligonucleotide primer LegPro3'.

SEQ. ID. NO:21 sets forth oligonucleotide primer LegTerm5'.

SEQ. ID. NO:22 sets forth oligonucleotide primer LegTerm3'.

SEQ. ID. NO:23 sets forth oligonucleotide primer oKTi5.

SEQ. ID. NO:24 sets forth oligonucleotide primer oKTi6.

SEQ. ID. NO:25 sets forth oligonucleotide primer LegA1Pro5'.

SEQ. ID. NO:26 sets forth oligonucleotide primer LegA1Pro3'.

SEQ. ID. NO:27 sets forth oligonucleotide primer LegA1Term5'.

SEQ. ID. NO:28 sets forth oligonucleotide primer LegA1Term3'.

SEQ. ID. NO:29 sets forth oligonucleotide primer annreamp5'.

SEQ. ID. NO:30 sets forth oligonucleotide primer annreamp3'.

SEQ. ID. NO:31 sets forth oligonucleotide primer BD30 reamp5'.

SEQ. ID. NO:32 sets forth oligonucleotide primer BD30 reamp3'.

SEQ. ID. NO:33 sets forth the sequence of the gene for Mortierella alpina delta-6 desaturase.

SEQ. ID. NO:34 sets forth the protein sequence of the Mortierella alpina delta-6 desaturase.

SEQ. ID. NO:35 sets forth the sequence of the gene for Saprolegnia diclina delta-6 desaturase.

SEQ. ID. NO:36 sets forth the protein sequence of the Saprolegnia diclina delta-6 desaturase.

SEQ. ID. NO:37 sets forth the sequence of the gene for Saprolegnia diclina delta-5 desaturase.

SEQ. ID. NO:38 sets forth the protein sequence of the Saprolegnia diclina delta-5 desaturases.

SEQ. ID. NO:39 sets forth the sequence of the gene for Thraustochytrium aureum elongase.

SEQ. ID. NO:40 sets forth the protein sequence of the Thraustochytrium aureum elongase.

SEQ. ID. NO:41 sets forth the sequence of the gene for *Saprolegnia diclina* delta-17 desaturase.

SEQ. ID. NO:42 sets forth the protein sequence of the Saprolegnia diclina delta-17 desaturase.

SEQ. ID. NO:43 sets forth the sequence of the gene for Mortierella alpina elongase.

SEQ. ID. NO:44 sets forth the protein sequence of the Mortierella alpina elongase.

SEQ. ID. NO:45 sets forth the sequence of the gene for Mortierella alpina delta-5 desaturase.

SEQ. ID. NO:46 sets forth the protein sequence of the Mortierella alpina delta-5 desaturase.

SEQ. ID. NO:47 sets forth the sequence of At FAD3, the gene for Arabidopsis thaliana delta-15 desaturase.

SEQ. ID. NO:48 sets forth the protein sequence of the Arabidopsis thaliana delta-15 desaturase.

SEQ. ID. NO:49 sets forth the sequence of the gene for Pavlova sp. elongase.

SEQ. ID. NO:50 sets forth the protein sequence of the Pavlova sp. elongase.

SEQ. ID. NO:51 sets forth the sequence of the gene for Schizochytrium aggregatum delta-4 desaturase.

SEQ. ID. NO:52 sets forth the protein sequence of the Schizochytrium - aggregatum delta-4 desaturase.

SEQ. ID. NO:53 sets forth oligonucleotide primer RSP19F.

SEQ. ID. NO:54 sets forth oligonucleotide primer RSP19R.

SEQ. ID. NO:55 sets forth oligonucleotide primer RBP2F.

SEQ. ID. NO:56 sets forth oligonucleotide primer RBP2R.

SEQ. ID. NO:57 sets forth oligonucleotide primer CGR4F.

SEQ. ID. NO:58 sets forth oligonucleotide primer CGR4R.

SEQ. ID. NO:59 sets forth oligonucleotide primer oSGly-1.

SEQ. ID. NO:60 sets forth oligonucleotide primer oSGly-2.

SEQ. ID. NO:61 sets forth consensus desaturase Protein Motif 1.

SEQ. ID. NO:62 sets forth oligonucleotide primer RO1144.

SEQ. ID. NO:63 sets forth consensus desaturase Protein Motif 2.

SEQ. ID. NO:64 sets forth oligonucleotide primer RO1119.

SEQ. ID. NO:65 sets forth oligonucleotide primer RO1118.

SEQ. ID. NO:66 sets forth consensus desaturase Protein Motif 3.

SEQ. ID. NO:67 sets forth oligonucleotide primer RO1121.

SEQ. ID. NO:68 sets forth oligonucleotide primer RO1122.

SEQ. ID. NO:69 sets forth consensus desaturase Protein Motif 4.

SEQ. ID. NO:70 sets forth oligonucleotide primer RO1146.

SEQ. ID. NO:71 sets forth oligonucleotide primer RO1147.

SEQ. ID. NO:72 sets forth consensus desaturase Protein Motif 5.

SEQ. ID. NO:73 sets forth oligonucleotide primer RO1148.

SEQ. ID. NO:74 sets forth consensus desaturase Protein Motif 6.

SEQ. ID. NO:75 sets forth oligonucleotide primer RO1114.

SEQ. ID. NO:76 sets forth consensus desaturase Protein Motif 7.

SEQ. ID. NO:77 sets forth oligonucleotide primer RO1116.

SEQ. ID. NO:78 sets forth consensus desaturase Protein Motif 8.

SEQ. ID. NO:80 sets forth oligonucleotide primer RO1189.

SEQ. ID. NO:81 sets forth oligonucleotide primer RO1190.

SEQ. ID. NO:82 sets forth oligonucleotide primer RO1191.

SEQ. ID. NO:83 sets forth oligonucleotide primer RO898.

SEQ. ID. NO:84 sets forth oligonucleotide primer RO899.

SEQ. ID. NO:85 sets forth oligonucleotide primer RO1185.

SEQ. ID. NO:86 sets forth oligonucleotide primer RO1186.

SEQ. ID. NO:87 sets forth oligonucleotide primer RO1187.

SEQ. ID. NO:88 sets forth oligonucleotide primer RO1212.

SEQ. ID. NO:89 sets forth oligonucleotide primer RO1213.

SEQ. ID. NO:90 sets forth the sequence of the expression cassette that comprises the constitutive soybean S-adenosylmethionine synthetase (SAMS) promoter operably linked to a gene for a form of soybean acetolactate synthase (ALS) that is capable of conferring resistance to sulfonylurea herbicides.

SEQ. ID. NO:91 sets forth oligonucleotide primer oSB030-1.

SEQ. ID. NO:92 sets forth oligonucleotide primer oSBD30-2.

SEQ. ID. NO:93 sets forth oligonucleotide primer T7pro.

SEQ. ID. NO:94 sets forth oligonucleotide primer RO1327.

SEQ. ID. NO:95 sets forth oligonucleotide primer GenRacer3'.

SEQ. ID. NO:96 sets forth oligonucleotide primer oCal-26.

SEQ. ID. NO:97 sets forth oligonucleotide primer oCal-27.

SEQ. ID. NO:98 sets forth oligonucleotide primer oKTi7.

### DETAILED DESCRIPTION OF THE INVENTION

All patents, patent applications, and publications cited are incorporated herein by reference in their entirety.

In the context of this disclosure, a number of terms shall be utilized.

Fatty acids are described herein by a numbering system in which the number before the colon indicates the number of carbon atoms in the fatty acid, whereas the number after the colon is the number of double bonds that are present. The number following the fatty acid designation indicates the position of the double bond from the carboxyl end of the fatty acid with the "c" affix for the cis-configuration of the double bond, e.g., palmitic acid (16:0), stearic acid (18:0), oleic acid (18:1,9c), petroselinic acid (18:1, 6c), linoleic acid (18:2,9c, 12c), γ-linolenic acid (18:3, 6c,9c,12c) and α-linolenic acid (18:3, 9c,12c,15c). Unless otherwise specified 18:1, 18:2 and 18:3 refer to oleic, linoleic and linolenic fatty acids.

"Omega-3 fatty acid" (also referred to as an n-3 fatty acid) includes the essential fatty acid α-linolenic acid (18:3n-3) (ALA) and its long-chain metabolites. In n-3 fatty acids, the first double bond is located at the third carbon from the methyl end of the hydrocarbon chain. For n-6 fatty acids, it is located at the sixth carbon. Eicosapentaneoic acid (EPA), docosapentaenoic acid (DPA), and docosahexanenoic acid (DHA) are examples of omega-3 fatty acids.

"Desaturase" is a polypeptide which can desaturate one or more fatty acids to produce a mono- or poly-unsaturated fatty acid or precursor which is of interest.

A "food analog" is a food-like product manufactured to resemble its food counterpart, whether meat, cheese, milk or the like, and is intended to have the appearance, taste, and texture of its counterpart.

"Aquaculture feed" refers to feed used in aquafarming which concerns the propagation, cultivation or farming of aquatic organisms, animals and/or plants in fresh or marine waters.

The terms "polynucleotide", "polynucleotide sequence", "nucleic acid sequence", and "nucleic acid fragment"/"isolated nucleic acid fragment" are used interchangeably herein. These terms encompass nucleotide sequences and the like. A polynucleotide may be a polymer of RNA or DNA that is single- or double-stranded, that optionally contains synthetic, non-natural or altered nucleotide bases. A polynucleotide in the form of a polymer of DNA may be comprised of one or more segments of cDNA, genomic DNA, synthetic DNA, or mixtures thereof. Nucleotides (usually found in their 5'-monophosphate form) are referred to by a single letter designation as follows: "A" for adenylate or deoxyadenylate (for RNA or DNA, respectively), "C" for cytidylate or deoxycytidylate, "G" for guanylate or deoxyguanylate, "U" for uridylate, "T" for deoxythymidylate, "R" for purines (A or G), "Y" for pyrimidines (C or T), "K" for G or T, "H" for A or C or T, "I" for inosine, and "N" for any nucleotide.

The terms "subfragment that is functionally equivalent" and "functionally equivalent subfragment" are used interchangeably herein. These terms refer to a portion or subsequence of an isolated nucleic acid fragment in which the ability to alter gene expression or produce a certain phenotype is retained whether or not the fragment or subfragment encodes an active enzyme. For example, the fragment or subfragment can be used in the design of chimeric genes to produce the desired phenotype in a transformed plant. Chimeric genes can be designed for use in suppression by linking a nucleic acid fragment or subfragment thereof, whether or not it encodes an active enzyme, in the sense or antisense orientation relative to a plant promoter sequence.

The terms "homology", "homologous", "substantially similar" and "corresponding substantially" are used interchangeably herein. They refer to nucleic acid fragments wherein changes in one or more nucleotide bases do not affect the ability of the nucleic acid fragment to mediate gene expression or produce a certain phenotype. These terms also refer to modifications of the nucleic acid fragments of the instant invention such as deletion or insertion of one or more nucleotides that do not substantially alter the functional properties of the resulting nucleic acid fragment relative to the initial, unmodified fragment. It is therefore understood, as those skilled in the art will appreciate, that the invention encompasses more than the specific exemplary sequences.

Moreover, the skilled artisan recognizes that substantially similar nucleic acid sequences encompassed by this invention are also defined by their ability to hybridize, under moderately stringent conditions (for example, 0.5 X SSC, 0.1% SDS, 60°C) with the sequences exemplified herein, or to any portion of the nucleotide sequences disclosed herein and which are functionally equivalent to any of the nucleic acid sequences disclosed herein. Stringency conditions can be adjusted to screen for moderately similar fragments, such as homologous sequences from distantly related organisms, to highly similar fragments, such as genes that duplicate functional enzymes from closely related organisms. Post-hybridization washes determine stringency conditions. One set of preferred conditions involves a series of washes starting with 6X SSC, 0.5% SDS at room temperature for 15 min, then repeated with 2X SSC, 0.5% SDS at 45°C for 30 min, and then repeated twice with 0.2X SSC, 0.5% SDS at 50°C for 30 min. A more preferred set of stringent conditions involves the use of higher temperatures in which the washes are identical to those above except for the temperature of the final two 30 min washes in 0.2X SSC, 0.5% SDS was increased to 60°C. Another preferred set of highly stringent conditions involves the use of two final washes in 0.1X SSC, 0.1% SDS at 65°C.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure. An "allele" is one of several alternative forms of a gene occupying a given locus on a chromosome. When all the alleles present at a given locus on a chromosome are the same that plant is homozygous at that locus. If the alleles present at a given locus on a chromosome differ that plant is heterozygous at that locus.

"Coding sequence" refers to a DNA sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include, but are not limited to, promoters, translation leader sequences, introns, and polyadenylation recognition sequences.

"Promoter" refers to a DNA sequence capable of controlling the expression of a coding sequence or functional RNA. The promoter sequence consists of proximal and more distal upstream elements, the latter elements often referred to as enhancers. Accordingly, an "enhancer" is a DNA sequence that can stimulate promoter activity, and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. It is further recognized that since in most cases the exact boundaries of regulatory sequences have not been completely defined, DNA fragments of some variation may have identical promoter activity. Promoters that cause a gene to be expressed in most cell types at most times are commonly referred to as "constitutive promoters". New promoters of various types useful in plant cells are constantly being discovered; numerous examples may be found in the compilation by Okamuro, J. K., and Goldberg, R. B. (1989) Biochemistry of Plants 15:1-82.

The "translation leader sequence" refers to a polynucleotide sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner, R. and Foster, G. D. (1995) Mol. Biotechnol. 3:225-236).

The "3' non-coding sequences" or "transcription terminator/termination sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht, I. L., et al. (1989) Plant Cell 1:671-680.

"RNA transcript" refers to the product resulting from RNA polymerase-catalyzed transcription of a DNA sequence. When the RNA transcript is a perfect complementary copy of the DNA sequence, it is referred to as the primary transcript. An RNA transcript is referred to as the mature RNA when it is an RNA sequence derived from post-transcriptional processing of the primary transcript. "Messenger RNA (mRNA)" refers to the RNA that is without introns and that can be translated into protein by the cell. "cDNA" refers to a DNA that is complementary to and synthesized from a mRNA template using the enzyme reverse transcriptase. The cDNA can be single-stranded or converted into the double-stranded form using the Klenow fragment of DNA polymerase I. "Sense" RNA refers to RNA transcript that includes the mRNA and can be translated into protein within a cell or in vitro. "Antisense RNA" refers to an RNA transcript that is complementary to all or part of a target primary transcript or mRNA, and that blocks the expression of a target gene (U.S. Patent No. 5,107,065). The complementarity of an antisense RNA may be with any part of the specific gene transcript, i.e., at the 5' non-coding sequence, 3' non-coding sequence, introns, or the coding sequence. "Functional RNA" refers to antisense RNA, ribozyme RNA, or other RNA that may not be translated but yet has an effect on cellular processes. The terms "complement" and "reverse complement" are used interchangeably herein with respect to mRNA transcripts, and are meant to define the antisense RNA of the message.

The term "operably linked" refers to the association of nucleic acid sequences on a single nucleic acid fragment so that the function of one is regulated by the other. For example, a promoter is operably linked with a coding sequence when it is capable of regulating the expression of that coding sequence (i.e., that the coding sequence is under the transcriptional control of the promoter). Coding sequences can be operably linked to regulatory sequences in a sense or antisense orientation. In another example, the complementary RNA regions of the invention can be operably linked, either directly or indirectly, 5' to the target mRNA, or 3' to the target mRNA, or within the target mRNA, or a first complementary region is 5' and its complement is 3' to the target mRNA.

Standard recombinant DNA and molecular cloning techniques used herein are well known in the art and are described more fully in Sambrook, J., Fritsch, E.F. and Maniatis, T. Molecular Cloning: A Laboratory Manual; Cold Spring Harbor Laboratory Press: Cold Spring Harbor, 1989. Transformation methods are well known to those skilled in the art and are described below.

"PCR" or "Polymerase Chain Reaction" is a technique for the synthesis of large quantities of specific DNA segments, consists of a series of repetitive cycles (Perkin Elmer Cetus Instruments, Norwalk, CT). Typically, the double stranded DNA is heat denatured, the two primers complementary to the 3' boundaries of the target segment are annealed at low temperature and then extended at an intermediate temperature. One set of these three consecutive steps is referred to as a cycle.

The term "recombinant" refers to an artificial combination of two otherwise separated segments of sequence, e.g., by chemical synthesis or by the manipulation of isolated segments of nucleic acids by genetic engineering techniques.

The terms "recombinant construct", "expression construct", "chimeric construct", "construct", and "recombinant DNA construct" are used interchangeably herein. A recombinant construct comprises an artificial combination of nucleic acid fragments, e.g., regulatory and coding sequences that are not found together in nature. For example, a chimeric construct may comprise regulatory sequences and coding sequences that are derived from different sources, or regulatory sequences and coding sequences derived from the same source, but arranged in a manner different than that found in nature. Such construct may be used by itself or may be used in conjunction with a vector. If a vector is used then the choice of vector is dependent upon the method that will be used to transform host cells as is well known to those skilled in the art. For example, a plasmid vector can be used. The skilled artisan is well aware of the genetic elements that must be present on the vector in order to successfully transform, select and propagate host cells comprising any of the isolated nucleic acid fragments of the invention. The skilled artisan will also recognize that different independent transformation events will result in different levels and patterns of expression (Jones et al., (1985) EMBO J. 4:2411-2418; De Almeida et al., (1989) Mol. Gen. Genetics 218:78-86), and thus that multiple events must be screened in order to obtain lines displaying the desired expression level and pattern. Such screening may be accomplished by Southern analysis of DNA, Northern analysis of mRNA expression, immunoblotting analysis of protein expression, or phenotypic analysis, among others.

The term "expression", as used herein, refers to the production of a functional end-product e.g., a mRNA or a protein (precursor or mature).

The term "expression cassette" as used herein, refers to a discrete nucleic acid fragment into which a nucleic acid sequence or fragment can be moved.

"Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre- and propeptides may be but are not limited to intracellular localization signals.

"Stable transformation" refers to the transfer of a nucleic acid fragment into a genome of a host organism, including-both nuclear and organellar genomes, resulting in genetically stable inheritance. In contrast, "transient transformation" refers to the transfer of a nucleic acid fragment into the nucleus, or DNA-containing organelle, of a host organism resulting in gene expression without integration or stable inheritance. Host organisms containing the transformed nucleic acid fragments are referred to as "transgenic" organisms.

"Antisense inhibition" refers to the production of antisense RNA transcripts capable of suppressing the expression of the target protein. "Co-suppression" refers to the production of sense RNA transcripts capable of suppressing the expression of identical or substantially similar foreign or endogenous genes (U.S. Patent No. 5,231,020). Co-suppression constructs in plants previously have been designed by focusing on overexpression of a nucleic acid sequence having homology to an endogenous mRNA, in the sense orientation, which results in the reduction of all RNA having homology to the overexpressed sequence (see Vaucheret et al. (1998) Plant J. 16:651-659; and Gura (2000) Nature 404:804-808). The overall efficiency of this phenomenon is low, and the extent of the RNA reduction is widely variable. Recent work has described the use of "hairpin" structures that incorporate all, or part, of an mRNA encoding sequence in a complementary orientation that results in a potential "stem-loop" structure for the expressed RNA (PCT Publication WO 99/53050 published on October 21, 1999 and more recently, Applicants' assignee's PCT Application having international publication number WO 02/00904 published on January 3, 2002). This increases the frequency of co-suppression in the recovered transgenic plants. Another variation describes the use of plant viral sequences to direct the suppression, or "silencing", of proximal mRNA encoding sequences (PCT Publication WO 98/36083 published on August 20, 1998). Both of these co-suppressing phenomena have not been elucidated mechanistically, although genetic evidence has begun to unravel this complex situation (Elmayan et al. (1998) Plant Cell 10:1747-1757).

The polynucleotide sequences used for suppression do not necessarily have to be 100% complementary to the polynucleotide sequences found in the gene to be suppressed. For example, suppression of all the subunits of the soybean seed storage protein ß-conglycinin has been accomplished using a polynucleotide derived from a portion of the gene encoding the α subunit (U.S. Patent No. 6,362,399). ß-conglycinin is a heterogeneous glycoprotein composed of varying combinations of three highly negatively charged subunits identified as α, α' and β. The polynucleotide sequences encoding the α and α' subunits are 85% identical to each other while the polynucleotide sequences encoding the β subunit are 75 to 80% identical to the α and α' subunits. Thus, polynucleotides that are at least 75% identical to a region of the polynucleotide that is target for suppression have been shown to be effective in suppressing the desired target. The polynucleotide should be at least 80% identical, preferably at least 90% identical, most preferably at least 95% identical, or the polynucleotide may be 100% identical to the desired target.

The present invention concerns an oilseed plant that produces mature seeds in which the total seed fatty acid profile comprises at least 1.0 % of at least one polyunsaturated fatty acid having at least twenty carbon atoms and five or more carbon-carbon double bonds.

In a second embodiment, this invention concerns an oilseed plant that produces mature seeds in which the total seed fatty acid profile comprises at least 5.0 % of at least one polyunsaturated fatty acid having at least twenty carbon atoms and five or more carbon-carbon double bonds.

In a third embodiment, this invention concerns an oilseed plant that produces mature seeds in which the total seed fatty acid profile comprises at least 10.0 % of at least one polyunsaturated fatty acid having at least twenty carbon atoms and five or more carbon-carbon double bonds.

Additional embodiments of this invention include an oilseed plant that produces mature seeds in which the total seed fatty acid profile comprises at least 15.0 %, 20 %, 25 %, 30 %, 40 %, 50 %, or 60 % of at least one polyunsaturated fatty acid having at least twenty carbon atoms and five or more carbon-carbon double bonds. Indeed, one might expect that any integer level of accumulation of at least one polyunsaturated fatty acid from about 1 % to about 60 % of the total seed fatty acid profile could be obtained.

In a fourth embodiment, this invention concerns an oilseed plant that produces mature seeds in which the total seed fatty acid profile comprises at least 10.0 % of at least one polyunsaturated fatty acid having at least twenty carbon atoms and five or more carbon-carbon double bonds and less than 2.0% arachidonic acid.

Again additional embodiments would include an oilseed plant that produces mature seeds in which the total seed fatty acid profile comprises at least 15.0 %, 20 %, 25 %, 30 %, 40 %, 50 %, or 60 % of at least one polyunsaturated fatty acid having at least twenty carbon atoms and five or more carbon-carbon double bonds and less than 2.0% arachidonic acid. Indeed, one might expect that any integer level of accumulation of at least one polyunsaturated fatty acid from about 1 % to about 60 % of the total seed fatty acid profile could be obtained while accumulating less than 2 % arachidonic acid.

Examples of oilseed plants include, but are not limited to, soybean, Brassica species, sunflower, maize, cotton, flax, and safflower.

Examples of polyunsaturated fatty acids having at least twenty carbon atoms and five or more carbon-carbon double bonds include, but are not limited to, omega-3 fatty acids such as EPA, DPA and DHA. Seeds obtained from such plants are also within the scope of this invention as well as oil obtained from such seeds.

In a fifth embodiment this invention concerns a recombinant construct for altering the total fatty acid profile of mature seeds of an oilseed plant, said construct comprising at least two promoters wherein each promoter is operably linked to a nucleic acid sequence encoding a polypeptide required for making at least one polyunsaturated fatty acid having at least twenty carbon atoms and four or more carbon-carbon double bonds and further wherein the total fatty acid profile comprises at least 2 % of at least one polyunsaturated fatty acid having at least twenty carbon atoms and four or more carbon-carbon double bonds and further wherein said polypeptide is an enzyme selected from the group consisting of a Δ 4 desaturase, a Δ 5 desaturase, Δ 6 desaturase, a Δ 15 desaturase, a Δ 17 desaturase, a C18 to C22 elongase and a C20 to C24 elongase.

Such desaturases are discussed in U.S. Patent Nos. 6,075,183, 5,968,809, 6,136,574, 5,972,664, 6,051,754, 6,410,288 and WO 98/46763, WO 98/46764, WO 00/12720, WO 00/40705

The choice of combination of cassettes used depends in part on the PUFA profile and/or desaturase profile of the oilseed plant cells to be transformed and the LC-PUFA which is to be expressed.

A number of enzymes are involved in PUFA biosynthesis. Linoleic acid (LA, 18:2 Δ9,12) is produced from oleic acid (18:1 Δ9) by a delta-12 desaturase. GLA (18:3 Δ 6, 9,12) is produced from linoleic acid (18:2 Δ9,12 ) by a delta-6 desaturase. ARA(20:4 Δ 5, 8, 11, 14 ) production from dihomo-gamma-linolenic acid (DGLA 20:3 Δ 8, 11, 14 ) is catalyzed by a delta-5 desaturase. However, animals cannot desaturate beyond the delta-9 position and therefore cannot convert oleic acid (18:1 Δ9) into linoleic acid (LA, 18:2 Δ9,12). Likewise, alpha-linolenic acid (ALA 18:3 Δ 9, 12, 15) cannot be synthesized by mammals. Other eukaryotes, including fungi and plants, have enzymes which desaturate at positions delta-12 and delta-5. The major poly-unsaturated fatty acids of animals therefore are either derived from diet and/or from desaturation and elongation of linoleic acid (LA, 18:2 Δ9,12) or alpha-linolenic acid (ALA 18:3 Δ 9, 12, 15).

The elongation process in plants involves a four-step process initiated by the crucial step of condensation of malonate and a fatty acid with release of a carbon dioxide molecule. The substrates in fatty acid elongation are CoA thioesters. The condensation step is mediated by a 3-ketoacyl synthase, which is generally rate limiting to the overall cycle of four reactions and provides some substrate specificity. The product of one elongation cycle regenerates a fatty acid that has been extended by two carbon atoms (Browse et al., Trends in Biochemical Sciences 27(9): 467-473 (September 2002); Napier, Trends in Plant Sciences 7(2): 51-54 (February 2002)).

As was noted above, a promoter is a DNA sequence that directs cellular machinery of a plant to produce RNA from the contiguous coding sequence downstream (3') of the promoter. The promoter region influences the rate, developmental stage, and cell type in which the RNA transcript of the gene is made. The RNA transcript is processed to produce messenger RNA (mRNA) which serves as a template for translation of the RNA sequence into the amino acid sequence of the encoded polypeptide. The 5' non-translated leader sequence is a region of the mRNA upstream of the protein coding region that may play a role in initiation and translation of the mRNA. The 3' transcription termination/polyadenylation signal is a non-translated region downstream of the protein coding region that functions in the plant cells to cause termination of the RNA transcript and the addition of polyadenylate nucleotides to the 3' end of the RNA.

The origin of the promoter chosen to drive expression of the coding sequence is not important as long as it has sufficient transcriptional activity to accomplish the invention by expressing translatable mRNA for the desired nucleic acid fragments in the desired host tissue at the right time. Either heterologous or non-heterologous (i.e., endogenous) promoters can be used to practice the invention.

Suitable promoters which can be used to practice the invention include, but are not limited to, the alpha prime subunit of beta conglycinin promoter, Kunitz trypsin inhibitor 3 promoter, annexin promoter, Gly1 promoter, beta subunit of beta conglycinin promoter, P34/Gly Bd m 30K promoter, albumin promoter, Leg A1 promoter and Leg A2 promoter. The level of activity of the annexin, or P34, promoter is comparable to that of many known strong promoters, such as the CaMV 35S promoter (Atanassova et al., (1998) Plant Mol. Biol. 37:275-285; Battraw and Hall, (1990) Plant Mol. Biol. 15:527-538; Holtorf ef al., (1995) Plant Mol. Biol. 29:637-646; Jefferson et al., (1987) EMBO J. 6:3901-3907; Wilmink et al., (1995) Plant Mol. Biol. 28:949-955), the Arabidopsis oleosin promoters (Plant et al., (1994) Plant Mol. Biol. 25:193-205; Li, (1997) Texas A&M University Ph.D. dissertation, pp. 107-128), the Arabidopsis ubiquitin extension protein promoters (Callis et al., 1990), a tomato ubiquitin gene promoter (Rollfinke et al., 1998), a soybean heat shock protein promoter (Schoffl et al., 1989), and a maize H3 histone gene promoter (Atanassova et al., 1998).

Expression of chimeric genes in most plant cell makes the annexin, or P34, promoter, which constitutes the subject matter of Applicants' Assignee's copending application having Application No. 60/446,833 and Attorney Docket No. BB-1531 which is filed concurrently herewith, especially useful when seed specific expression of a target heterologous nucleic acid fragment is required. Another useful feature of the annexin promoter is its expression profile in developing seeds. The annexin promoter of the invention is most active in developing seeds at early stages (before 10 days after pollination) and is largely quiescent in later stages. The expression profile of the annexin promoter is different from that of many seed-specific promoters, e.g., seed storage protein promoters, which often provide highest activity in later stages of development (Chen et al., (1989) Dev. Genet. 10:112-122; Ellerstrom et al., (1996) Plant Mol. Biol. 32:1019-1027; Keddie et al., (1994) Plant Mol. Biol. 24:327-340; Plant et al., (1994) Plant Mol. Biol. 25:193-205; Li, (1997) Texas A&M University Ph.D. dissertation, pp. 107-128). The P34 promoter has a more conventional expression profile but remains distinct from other known seed specific promoters. Thus, the annexin, or P34, promoter will be a very attractive candidate when overexpression, or suppression, of a gene in embryos is desired at an early developing stage. For example, it may be desirable to overexpress a gene regulating early embryo development or a gene involved in the metabolism prior to seed maturation.

The promoter is then operably linked in a sense orientation using conventional means well known to those skilled in the art.

Once the recombinant construct has been made, it may then be introduced into the oilseed plant cell of choice by methods well known to those of ordinary skill in the art including, for example, transfection, transformation and electroporation as described above. The transformed plant cell is then cultured and regenerated under suitable conditions permitting expression of the LC-PUFA which is then recovered and purified.

The recombinant constructs of the invention may be introduced into one plant cell or, alternatively, each construct may be introduced into separate plant cells.

Expression in a plant cell may be accomplished in a transient or stable fashion as is described above.

The desired LC-PUFAs can be expressed in seed. Also within the scope of this invention are seeds or plant parts obtained from such transformed plants.

Plant parts include differentiated and undifferentiated tissues, including but not limited to, roots, stems, shoots, leaves, pollen, seeds, tumor tissue, and various forms of cells and culture such as single cells, protoplasts, embryos, and callus tissue. The plant tissue may be in plant or in organ, tissue or cell culture.

Methods for transforming dicots, primarily by use of Agrobacterium tumefaciens, and obtaining transgenic plants have been published, among others, for cotton (U.S. Patent No. 5,004,863, U.S. Patent No. 5,159,135); soybean (U.S. Patent No. 5,569,834, U.S. Patent No. 5,416,011); Brassica (U.S. Patent No. 5,463,174); peanut (Cheng et al. (1996) Plant Cell Rep. 15:653-657, McKently et al. (1995) Plant Cell Rep. 14:699-703); papaya (Ling, K. et al. (1991) Bio/technology 9:752-758); and pea (Grant et al. (1995) Plant Cell Rep. 15:254-258). For a review of other commonly used methods of plant transformation see Newell, C.A. (2000) Mol. Biotechnol. 16:53-65. One of these methods of transformation uses Agrobacterium rhizogenes (Tepfler, M. and Casse-Delbart, F. (1987) Microbial Sci. 4:24-28). Transformation of soybeans using direct delivery of DNA has been published using PEG fusion (PCT publication WO 92/17598), electroporation (Chowrira, G.M. et al. (1995) Mol. Biotechnol. 3:17-23; Christou, P. et al. (1987) Proc. Natl. Acad. Sci. U.S.A. 84:3962-3966), microinjection, or particle bombardment (McCabe, D.E. et. al. (1988) Bio/Technology 6:923; Christou et al. (1988) Plant Physiol. 87:671-674).

There are a variety of methods for the regeneration of plants from plant tissue. The particular method of regeneration will depend on the starting plant tissue and the particular plant species to be regenerated. The regeneration, development and cultivation of plants from single plant protoplast transformants or from various transformed explants is well known in the art (Weissbach and Weissbach, (1988) In.: Methods for Plant Molecular Biology, (Eds.), Academic Press, Inc., San Diego, CA). This regeneration and growth process typically includes the steps of selection of transformed cells, culturing those individualized cells through the usual stages of embryonic development through the rooted plantlet stage. Transgenic embryos and seeds are similarly regenerated. The resulting transgenic rooted shoots are thereafter planted in an appropriate plant growth medium such as soil. Preferably, the regenerated plants are self-pollinated to provide homozygous transgenic plants. Otherwise, pollen obtained from the regenerated plants is crossed to seed-grown plants of agronomically important lines. Conversely, pollen from plants of these important lines is used to pollinate regenerated plants. A transgenic plant of the present invention containing a desired polypeptide is cultivated using methods well known to one skilled in the art.

In addition to the above discussed procedures, practitioners are familiar with the standard resource materials which describe specific conditions and procedures for the construction, manipulation and isolation of macromolecules (e.g., DNA molecules, plasmids, etc.), generation of recombinant DNA fragments and recombinant expression constructs and the screening and isolating of clones, (see for example, Sambrook et al. (1989) Molecular Cloning: A Laboratory Manual, Cold Spring Harbor Press; Maliga et al. (1995) Methods in Plant Molecular Biology, Cold Spring Harbor Press; Birren et al. (1998) Genome Analysis: Detecting Genes, 1, Cold Spring Harbor, New York; Birren et al. (1998) Genome Analysis: Analyzing DNA, 2, Cold Spring Harbor, New York; Plant Molecular Biology: A Laboratory Manual, eds. Clark, Springer, New York (1997)).

In another aspect, this invention concerns a method for making an oilseed plant having an altered fatty acid profile which comprises:
a) transforming a plant with the recombinant construct of the invention;
b) growing the transformed plant of step (a); and
c) selecting those plants wherein the total fatty acid profile comprises at least 1.0 % of at least one polyunsaturated fatty acid having at least twenty carbon atoms and five or more carbon-carbon double bonds.

Methods of isolating seed oils are well known in the art: (Young et al, Processing of Fats and Oils, in "The Lipid Handbook" (Gunstone et al eds.) Chapter 5 pp 253-257; London, Chapman & Hall, 1994).

The altered seed oils can then be added to nutritional compositions such as a nutritional supplement, food products, infant formula, animal feed, pet food and the like.

Compared to other vegetable oils, the oils of the invention are believed to function similarly to other oils in food applications from a physical standpoint. Partially hydrogenated oils, such as soybean oil, are widely used as ingredients for soft spreads, margarine and shortenings for baking and frying.

Examples of food products or food analogs into which altered seed oils or altered seeds of the invention may be incorporated include a meat product such as a processed meat product, a cereal food product, a snack food product, a baked goods product, a fried food product, a health food product, an infant formula, a beverage, a nutritional supplement, a dairy product, a pet food product, animal feed or an aquaculture food product. Food analogs can be made use processes well known to those skilled in the art. U.S. Patent Nos. 6,355,296 B1 and 6,187,367 B1 describe emulsified meat analogs and emulsified meat extenders. U.S. Patent No. 5,206,050 B1 describes soy protein curd useful for cooked food analogs (also can be used as a process to form a curd useful to make food analogs). U.S. Patent No. 4,284,656 to Hwa describes a soy protein curd useful for food analogs. U.S. Patent No. 3,988,485 to Hibbert et al. describes a meat-like protein food formed from spun vegetable protein fibers. U.S. Patent No. 3,950,564 to Puski et al. describes a process of making a soy based meat substitute and U.S. Patent No. 3,925,566 to Reinhart et al. describes a simulated meat product. For example, soy protein that has been processed to impart a structure, chunk or fiber for use as a food ingredient is called "textured soy protein" (TSP). TSPs are frequently made to resemble meat, seafood, or poultry in structure and appearance when hydrated.

There can be mentioned meat analogs, cheese analogs, milk analogs and the like.

Meat analogs made from soybeans contain soy protein or tofu and other ingredients mixed together to simulate various kinds of meats. These meat alternatives are sold as frozen, canned or dried foods. Usually, they can be used the same way as the foods they replace. Meat alternatives made from soybeans are excellent sources of protein, iron and B vitamins. Examples of meat analogs include, but are not limited to, ham analogs, sausage analogs, bacon analogs, and the like.

Food analogs can be classified as imitiation or substitutes depending on their functional and compositional characteristics. For example, an imitation cheese need only resemble the cheese it is designed to replace. However, a product can generally be called a substitute cheese only if it is nutritionally equivalent to the cheese it is replacing and meets the minimum compositional requirements for that cheese. Thus, substitute cheese will often have higher protein levels than imitation cheeses and be fortified with vitamins and minerals.

Milk analogs or nondairy food products include, but are not limited to, imitation milk, nondairy frozen desserts such as those made from soybeans and/or soy protein products.

Meat products encompass a broad variety of products. In the United States "meat" includes "red meats" produced from cattle, hogs and sheep. In addition to the red meats there are poultry items which includes chickens, turkeys, geese, guineas, ducks and the fish and shellfish. There is a wide assortment of seasoned and processes meat products: fresh, cured and fried, and cured and cooked. Sausages and hot dogs are examples of processed meat products. Thus, the term "meat products" as used herein includes, but is not limited to, processed meat products.

A cereal food product is a food product derived from the processing of a cereal grain. A cereal grain includes any plant from the grass family that yields an edible grain (seed). The most popular grains are barley, corn, millet, oats, quinoa, rice, rye, sorghum, triticale, wheat and wild rice. Examples of a cereal food product include, but are not limited to, whole grain, crushed grain, grits, flour, bran, germ, breakfast cereals, extruded foods, pastas, and the like.

A baked goods product comprises any of the cereal food products mentioned above and has been baked or processed in a manner comparable to baking, i.e., to dry or harden by subjecting to heat. Examples of a baked good product include, but are not limited to bread, cakes, doughnuts, bread crumbs, baked snacks, mini-biscuits, mini-crackers, mini-cookies, and mini-pretzels. As was mentioned above, oils of the invention can be used as an ingredient.

In general, soybean oil is produced using a series of steps involving the extraction and purification of an edible oil product from the oil bearing seed. Soybean oils and soybean byproducts are produced using the generalized steps shown in the diagram below.

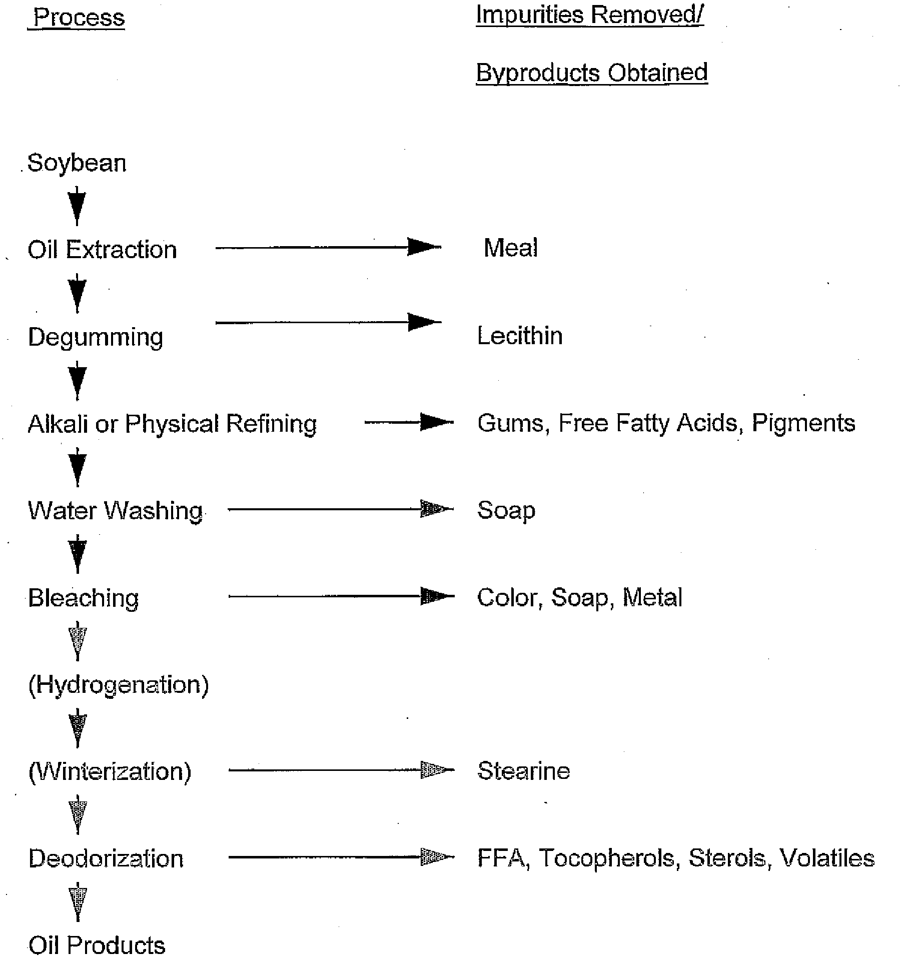

Soybean seeds are cleaned, tempered, dehulled, and flaked which increases the efficiency of oil extraction. Oil extraction is usually accomplished by solvent (hexane) extraction but can also be achieved by a combination of physical pressure and/or solvent extraction. The resulting oil is called crude oil. The crude oil may be degummed by hydrating phospholipids and other polar and neutral lipid complexes that facilitate their separation from the nonhydrating, triglyceride fraction (soybean oil). The resulting lecithin gums may be further processed to make commercially important lecithin products used in a variety of food and industrial products as emulsification and release (antisticking) agents. Degummed oil may be further refined for the removal of impurities; primarily free fatty acids, pigments, and residual gums. Refining is accomplished by the addition of a caustic agent that reacts with free fatty acid to form soap and hydrates phosphatides and proteins in the crude oil. Water is used to wash out traces of soap formed during refining. The soapstock byproduct may be used directly in animal feeds or acidulated to recover the free fatty acids. Color is removed through adsorption with a bleaching earth that removes most of the chlorophyll and carotenoid compounds. The refined oil can be hydrogenated resulting in fats with various melting properties and textures. Winterization (fractionation) may be used to remove stearine from the hydrogenated oil through crystallization under carefully controlled cooling conditions. Deodorization which is principally steam distillation under vacuum, is the last step and is designed to remove compounds which impart odor or flavor to the oil. Other valuable byproducts such as tocopherols and sterols may be removed during the deodorization process. Deodorized distillate containing these byproducts may be sold for production of natural vitamin E and other high-value pharmaceutical products. Refined, bleached, (hydrogenated, fractionated) and deodorized oils and fats may be packaged and sold directly or further processed into more specialized products. A more detailed reference to soybean seed processing, soybean oil production and byproduct utilization can be found in Erickson, 1995, Practical Handbook of Soybean Processing and Utilization, The American Oil Chemists' Society and United Soybean Board.

Soybean oil is liquid at room temperature because it is relatively low in saturated fatty acids when compared with oils such as coconut, palm, palm kernel and cocoa butter. Many processed fats, including spreads, confectionary fats, hard butters, margarines, baking shortenings, etc., require varying degrees of solidity at room temperature and can only be produced from soybean oil through alteration of its physical properties. This is most commonly achieved through catalytic hydrogenation.

Hydrogenation is a chemical reaction in which hydrogen is added to the unsaturated fatty acid double bonds with the aid of a catalyst such as nickel. High oleic soybean oil contains unsaturated oleic, linoleic, and linolenic fatty acids and each of these can be hydrogenated. Hydrogenation has two primary effects. First, the oxidative stability of the oil is increased as a result of the reduction of the unsaturated fatty acid content. Second, the physical properties of the oil are changed because the fatty acid modifications increase the melting point resulting in a semi-liquid or solid fat at room temperature.

There are many variables which affect the hydrogenation reaction which in turn alter the composition of the final product. Operating conditions including pressure, temperature, catalyst type and concentration, agitation and reactor design are among the more important parameters which can be controlled. Selective hydrogenation conditions can be used to hydrogenate the more unsaturated fatty acids in preference to the less unsaturated ones. Very light or brush hydrogenation is often employed to increase stability of liquid oils. Further hydrogenation converts a liquid oil to a physically solid fat. The degree of hydrogenation depends on the desired performance and melting characteristics designed for the particular end product. Liquid shortenings, used in the manufacture of baking products, solid fats and shortenings used for commercial frying and roasting operations, and base stocks for margarine manufacture are among the myriad of possible oil and fat products achieved through hydrogenation. A more detailed description of hydrogenation and hydrogenated products can be found in Patterson, H. B. W., 1994, Hydrogenation of Fats and Oils: Theory and Practice. The American Oil Chemists' Society.

Hydrogenated oils have also become controversial due to the presence of trans fatty acid isomers that result from the hydrogenation process. Ingestion of large amounts of trans isomers has been linked with detrimental health effects including increased ratios of low density to high density lipoproteins in the blood plasma and increased risk of coronary heart disease.

A snack food product comprises any of the above or below described food products.

A fried food product comprises any of the above or below described food products that has been fried.

A health food product is any food product that imparts a health benefit. Many oilseed-derived food products may be considered as health foods.

The beverage can be in a liquid or in a dry powdered form.

For example, there can be mentioned non-carbonated drinks; fruit juices, fresh, frozen, canned or concentrate; flavored or plain milk drinks, etc. Adult and infant nutritional formulas are well known in the art and commercially available (e.g., Similac®, Ensure®, Jevity®, and Alimentum® from Ross Products Division, Abbott Laboratories).

Infant formulas are liquids or reconstituted powders fed to infants and young children. They serve as substitutes for human milk. Infant formulas have a special role to play in the diets of infants because they are often the only source of nutrients for infants. Although breast-feeding is still the best nourishment for infants, infant formula is a close enough second that babies not only survive but thrive. Infant formula is becoming more and more increasingly close to breast milk.

A dairy product is a product derived from milk. A milk analog or nondairy product is derived from a source other than milk, for example, soymilk as was discussed above. These products include, but are not limited to, whole milk, skim milk, fermented milk products such as yogurt or sour milk, cream, butter, condensed milk, dehydrated milk, coffee whitener, coffee creamer, ice cream, cheese, etc.

A pet food product is a product intended to be fed to a pet such as a dog, cat, bird, reptile, fish, rodent and the like. These products can include the cereal and health food products above, as well as meat and meat byproducts, soy protein products, grass and hay products, including but not limited to alfalfa, timothy, oat or brome grass, vegetables and the like.

Animal feed is a product intended to be fed to animals such as turkeys, chickens, cattle and swine and the like. As with the pet foods above, these products can include cereal and health food products, soy protein products, meat and meat byproducts, and grass and hay products as listed above.

Aqualculture feed is a product intended to be used in aquafarming which concerns the propagation, cultivation or farming of aquatic organisms, animals and/or plants in fresh or marine waters.

In yet another embodiment, this invention includes an oilseed plant that produces mature seeds in which the total seed fatty acid profile comprises polyunsaturated fatty acids having at least twenty carbon atoms and five or more carbon-carbon double bonds wherein the ratio of EPA:DHA is in the range from 1:100 to 860:100. The oilseed plant may further have a total seed fatty acid profile comprising less than 2.0% arachidonic acid. Also of interest are seeds obtained from such plants and oil obtained from the seeds of such plants.

In still yet another embodiment, this invention includes an oilseed plant that produces mature seeds in which the total seed fatty acid profile comprises polyunsaturated fatty acids having at least twenty carbon atoms and five or more carbon-carbon double bonds wherein the ratio of DHA:EPA is in the range from 1:100 to 110:100. The oilseed plant may further have a total seed fatty acid profile comprising less than 2.0% arachidonic acid. Also of interest are seeds obtained from such plants and oil obtained from the seeds of such plants.

It is reasonable to believe that any integer ratio of EPA:DHA from 1:100 through 860:100, or DHA:EPA from 1:100 through 110:100, might be obtainable in plant described or envisioned within the scope and spirit of the present invention.

### EXAMPLES

The present invention is further defined in the following Examples, in which all parts and percentages are given as weight to volume, and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various uses and conditions. Thus, various modifications of the invention in addition to those shown and described herein will be apparent to those skilled in the art from the foregoing description. Such modifications are also intended to fall within the scope of the appended claims.

The disclosures contained within the references used herein are hereby incorporated by reference.

### GENERAL MATERIALS AND METHODS

Procedures for nucleic acid phosphorylation, restriction enzyme digests, ligation and transformation are well known in the art. Techniques suitable for use in the following examples may be found in Sambrook, J., Fritsch, E. F. and Maniatis, T., Molecular Cloning: A Laboratory Manual, Second Edition, Cold Spring Harbor Laboratory Press, Cold Spring Harbor, NY (1989) (hereinafter "Maniatis").

Materials and methods suitable for the maintenance and growth of bacterial cultures are well known in the art. Techniques suitable for use in the following examples may be found as set out in Manual of Methods for General Bacteriology (Phillipp Gerhardt, R. G. E. Murray, Ralph N. Costilow, Eugene W. Nester, Willis A. Wood, Noel R. Krieg and G. Briggs Phillips, eds), American Society for Microbiology, Washington, DC. (1994)) or by Thomas D. Brock in Biotechnology: A, Textbook of Industrial Microbiology, Second Edition, Sinauer Associates, Inc., Sunderland, MA (1989). All reagents, restriction enzymes and materials used for the growth and maintenance of bacterial and plant cells were obtained from Aldrich Chemicals (Milwaukee, WI), DIFCO Laboratories (Detroit, MI), GIBCO/BRL (Gaithersburg, MD), or Sigma Chemical Company (St. Louis, MO) unless otherwise specified.

The meaning of abbreviations is as follows: "h" or "hr" means hour(s), "min" or "min." means minute(s), "sec" or "s" means second(s), "d" or "day" means day(s), "mL" means milliliters, "L" means liters.

### Bacterial Strains and Plasmids:

*E. coli* TOP10 cells and *E. coli* electromax DH10B cells were obtained from Invitrogen (Carlsbad, CA). Max Efficiency competent cells of *E. coli* DH5α were obtained from GIBCO/BRL (Gaithersburg, MD). Plasmids containing EPA or DHA biosynthetic pathway genes were obtained from Ross Products Division, Abbott Laboratories, Columbus OH. The genes and the source plasmids are listed in Table 1.

**TABLE 1**

| EPA BIOSYNTHETIC PATHWAY GENES | | | |
|---|---|---|---|
| Gene | Organism | Plasmid Name | Reference |
| Delta-6 desaturase | *S. diclina* | pRSP1 | WO 02/081668 |
| Delta-6 desaturase | *M. alpina* | pCGR5 | US Patent 5,968,809 |
| Elongase | *M. alpina* | pRPB2 | WO 00/12720 |
| Delta-5 desaturase | *M*. *alpina* | pCGR4 | US Patent 6,075,183 |
| Delta-5 desaturase | *S. diclina* | pRSP3 | WO 02/081668 |
| Delta-17 desaturase | *S. diclina* | pRSP19 | Example 6 |
| Elongase | *T. aureum* | pRAT-4-A7 | WO 02/08401 |
| Elongase | *Pavlova sp.* | pRPL-6-B2 | Example 13 |
| Delta-4 desaturase | *S. aggregatum* | pRSA1 | WO 02/090493 |

Plasmids pKS102 and pKS121 are described in WO 02/00904. Plasmid pKS123 is described in WO 02/08269. Plasmid pCF3 is described in [Yadav, N.S. et al (1993) Plant Physiol. 103:467-76]. Cloning vector pCR-Script AMP SK(+) was from Stratagene (La Jolla, CA). Cloning vector pUC19 [Messing, J. (1983) Meth. Enzymol. 101:20] was from New England Biolabs (Beverly, MA). Cloning vector pGEM-T easy was from Promega (Madison, WI).

### Growth Conditions:

Bacterial cells were usually grown in Luria-Bertani (LB) medium containing 1% of bacto-tryptone, 0.5% of bacto-yeast extract and 1% of NaCl. Occasionaly, bacterial cells were grown in SOC medium containing 2% of bacto-tryptone, 0.5% of bacto-yeast extract, 0.5% of NaCl and 20 mM glucose or in Superbroth (SB) containing 3.5% of bacto-tryptone, 2% of bacto-yeast extract, 0.05% of NaCl and 0.005 M NaOH.

Antibiotics were often added to liquid or solid media in order to select for plasmids or insertions with appropriate antibiotic resistance genes. Kanamycin, ampicillin and hygromycin were routinely used at final concentrations of 50 µg/mL (Kan50), 100 µg/mL (Amp100) or 50 µg/mL (Hyg50), respectively.

### EXAMPLE 1

### Isolation of soybean seed-specific promoters

The soybean annexin and BD30 promoters were isolated with the Universal GenomeWalker system (Clontech) according to its user manual (PT3042-1). To make soybean GenomeWalker libraries, samples of soybean genomic DNA were digested with *Dra*I*, Eco*RV, *Pvu*II and *Stu*I separately for two hours. After DNA purification, the digested genomic DNAs were ligated to the GenomeWalker adaptors AP1 and AP2.

Two gene specific primers (GSP1 and GSP2) were designed for soybean annexin gene based on the 5' coding sequences in annexin cDNA in DuPont EST database. The sequences of GSP1 and GSP2 are set forth in SEQ ID NOS:1 and 2.
GCCCCCCATCCTTTGAAAGCCTGT SEQ ID NO:1
CGCGGATCCGAGAGCCTCAGCATCTTGAGCAGAA SEQ ID NO:2

The AP1 and the GSP1 primers were used in the first round PCR using the conditions defined in the GenomeWalker system protocol. Cycle conditions were 94°C for 4 minutes; 94°C for 2 second and 72°C for 3 minutes, 7 cycles; 94°C for 2 second and 67°C for 3 minutes, 32 cycles; 67°C for 4 minutes. The products from the first run PCR were diluted 50-fold. One microliter of the diluted products were used as templates for the second PCR with the AP2 and GSP2 as primers. Cycle conditions were 94°C for 4 minutes; 94°C for 2 second and 72°C for 3 min, 5 cycles; 94°C for 2 second and 67°C for 3 minutes, 20 cycles; 67°C for 3 minutes. A 2.1 kb genomic fragment was amplified and isolated from the EcoRV-digested GenomeWalker library. The genomic fragment was digested with *BamH* I and *Sal* I and cloned into Bluescript KS⁺ vector for sequencing. The DNA sequence of this 2012 bp soybean annexin promoter fragment is set forth in SEQ ID NO:3.

Two gene specific primers (GSP3 and GSP4) were designed for soybean BD30 based on the 5' coding sequences in BD30 cDNA in NCBI GenBank (J05560). The oligonucleotide sequences of the GSP3 and GSP4 primers have the sequences set forth in SEQ ID NOS:4 and 5.
GGTCCAATATGGAACGATGAGTTGATA SEQ ID NO:4
CGCGGATCCGCTGGAACTAGAAGAGAGACCTAAGA SEQ ID NO:5

The AP1 and the GSP3 primers were used in the first round PCR using the same conditions defined in the GenomeWalker system protocol. The cycle conditions used for soybean annexin promoter do not work well for the soybean BD30 promoter in GenomeWalker experiment. A modified touchdown PCR protocol was used. Cycle conditions were: 94°C for 4 minutes; 94°C for 2 second and 74°C for 3 minutes, 6 cycles in which annealing temperature drops 1°C every cycle; 94°C for 2 second and 69°C for 3 minutes, 32 cycles; 69°C for 4 minutes. The products from the 1^{st} run PCR were diluted 59-fald. One microliter of the diluted products were used as templates for the 2^{nd} PCR with the AP2 and GSP4 as primers. Cycle conditions were: 94°C for 4 minutes; 94°C for 2 second and 74°C for 3 min, 6 cycles in which annealing temperature drops 1°C every cycle; 94°C for 2 second and 69°C for 3 minutes, 20 cycles; 69°C for 3 minutes. A 1.5 kb genomic fragment was amplified and isolated from the *P*vuII-digested GenomeWalker library. The genomic fragment was digested with *Bam*HI and S*al*I and cloned into Bluescript KS⁺ vector for sequencing. DNA sequencing determined that this genomic fragment contained a 1408 bp soybean BD30 promoter sequence (SEQ ID NO:6).

Based on the sequences of the soybean β-conglycinin β-subunit promoter sequence in NCBI database (S44893), two oligos with either *Bam*HI or *Not*I sites at the 5' ends were designed to amplify the soybean β-conglycinin β-subunit promoter (SEQ ID NO:7). The oligonucleotide sequences of these two oligos are set forth in SEQ ID NOS: 8 and 9.
CGCGGATCCTATATATGTGAGGGTAGAGGGTATCAC SEQ ID NO:8
GAATTCGCGGCCGCAGTATATATATTATTGGACGATGAAACATG SEQ ID NO:9

Based on the sequences of the soybean Glycinin Gy1 promoter sequence in the NCBI GenBank database (X15121), two oligos with either *Bam*HI or *Not*I sites at the 5' ends were designed to amplify the soybean Glycinin Gy1 promoter (SEQ ID NO:10). The oligonucleotide sequences of these two oligos are set forth in SEQ ID NOS:11 and 12.
CGCGGATCCTAGCCTAAGTACGTACTCAAAATGCCA SEQ ID NO:11
GAATTCGCGGCCGCGGTGATGACTGATGAGTGTTTAAGGAC SEQ ID NO:12

### EXAMPLE 2

### Vector Construction for Characterizing Strong, Seed-specific Promoters

EPA can be produced at high levels in the seeds of important oil crops, such as soy, by strongly expressing each of the individual biosynthetic genes together, in a seed specific manner. To reduce the chance of co-suppression, each individual gene can be operably linked to a different, strong, seed-specific promoter. Because the biosynthetic pathway leading to EPA involves the concerted action of a large number of different genes, it was necessary to first identify and characterize many different promoters that could then be used to express each EPA biosynthetic gene. Promoters were identified and tested for their relative seed-specific strengths by linking them to the *M*. *alpina* delta-6 desaturase which, in these experiments, acted as a reporter gene. The *M*. *alpina* delta-6 desaturase can introduce a double bond between the C6 and C7 carbon atoms of linoleic acid (LA) and α-linolenic acid (ALA) to form y-linolenic acid (GLA) and stearidonic acid (STA), respectively. Because GLA and STA are not normally found in the lipids of soybean, their presence and concentration in soy was indicative of the relative strength of the promoter behind which the delta6 desaturase had been placed. Promoters tested in this way are listed in Table 2 and the plasmid construction for each is described below.

**TABLE 2**

| SEED-SPECIFIC PROMOTERS AND VECTORS | | | |
|---|---|---|---|
| Promoter | Organism | Vector Name | Promoter Reference |
| β-conglycinin α'-subunit | Soy | pKR162 | Beachy et al., (1985) EMBO J. 4:3047-3053 |
| Kunitz Trypsin Inhibitor | Soy | pKR124 | Jofuku et al., (1989) Plant Cell 1:1079-1093 |
| annexin | Soy | pJS92 | this report¹ |
| Glycinin Gy1 | Soy | pZBL119 | this report |
| Albumin 2S | Soy | pKR188 | US Patent 6,177,613 |
| Legumin A1 | Pea | pKR189 | Rerie et al. (1991) Mol. Gen. Genet. 225:148-157 |
| β-conglycinin β-subunit | Soy | ZBL118 | this report |
| BD30 (also called P34) | Soy | pJS93 | this report¹ |
| Legumin A2 | Pea | pKR187 | Rerie et al. (1991) Mol. Gen. Genet. 225:148-157 |

| | | | |
|---|---|---|---|
| ¹This also constitutes the subject matter of Applicant's Assignees's application having Application No. 60/446,833 (Attorney Docket No. BB1531PRV) filed concurrently herewith. | | | |

The gene for the *M*. *alpina* delta-6 desaturase was PCR-amplified from pCGR5 using primers oCGR5-1 (SEQ ID NO:13) and oCGR5-2 (SEQ ID NO:14), which were designed to introduce *Not*I restriction enzyme sites at both ends of the delta-6 desaturase and an *Nco*I site at the start codon of the reading frame for the enzyme.
TTGCGGCCGCAAACCATGGCTGCTGCTCCCAG (SEQ ID NO:13)
AAGCGGGCGCTTACTGCGCCTTAC (SEQ ID NO:14)

The resulting PCR fragment was subcloned into the intermediate cloning vector pCR-Script AMP SK(+) (Stratagene) according the manufacturer's protocol to give plasmid pKR159. Plasmid pKR159 was then digested with *Not*I to release the *M. alpina* delta-6 desaturase, which was, in turn, cloned into the *Not*I site of a selected soybean expression vector. Each expression vector tested contained a *Not*I site flanked by a suitable promoter and transcription terminator. Each vector also contained the hygromycin B phosphotransferase gene [Gritz, L. and Davies, J. (1983) Gene 25:179-188], flanked by the T7 promoter and transcription terminator (T7prom/hpt/T7term cassette), and a bacterial origin of replication (ori) for selection and replication in *E. coli.* In addition, each vector also contained the hygromycin B phosphotransferase gene, flanked by the 35S promoter [Odell et al., (1985) Nature 313:810-812] and NOS 3' transcription terminator [Depicker et al., (1982) J. Mol. Appl. Genet. 1:561:570] (35S/hpt/NOS3' cassette) for selection in soybean.

Vector pKR162 was constructed by cloning the *Not*I fragment of pKR159, containing the delta-6 desaturase, into the *Not*I site of vector KS123. Vector KS123 contains a *Not*I site flanked by the promoter for the α' subunit of β-conglycinin and the phaseolin 3' transcription terminator elements (βcon/*Not*I/Phas3' cassette).

Vector pKR188 was constructed by cloning the *Not*I fragment of pKR159, containing the delta-6 desaturase, into the *Not*I site of vector pKR135. Vector pKR135 contains a *Not*I site flanked by the 2S albumin promoter and the 2S albumin 3' transcription terminator elements (SA/*Not*I/SA3' cassette). Plasmid pKR135 was constructed by cloning the *Bam*HI/*Sal*I fragment of pKR132, containing the SA/*NotI*/*SA3'* cassette, into the *BamH*I/*Sal*I site of pKS120. Plasmid pKS120 is identical to pKS123 except the *Hind*III fragment containing the βcon/*Not*I/Phas3' cassette was removed. Plasmid pKR132, containing the SA/*Not*I/SA3' cassette flanked by *Bam*HI and *Sal*I sites, was constructed by cloning the *Xba*I fragment of the SA/*Not*I/SA3*'* cassette, made by PCR amplification, into the *Xbal* site of pUC19. The albumin promoter was amplified from plasmid AL3 promoter::pBI121 (US Patent 6,177,613) using PCR. Primer oSAlb-9 (SEQ ID NO:15) was designed to introduce an *Xbal* site at the 5' end of the promoter, and oSAlb-3 (SEQ ID NO:16) was designed to introduce a *Not*I site at the 3' end of the promoter.
ATCTAGACCTGCAGGCCAACTGCGTTTGGGGCTC (SEQ ID NO:15)
CTTTTAACTTCGCGGCCGCTTGCTATTGATGGGTGAAGTG (SEQ ID NO:16)

The albumin transcription terminator was amplified from soy genomic DNA using primer oSAlb-4 (SEQ ID NO:17), designed to introduce a *Not*I site at the 5' end of the terminator, and primer oSAlb-2 (SEQ ID NO:18), designed to introduce *Bsi*WI and *Xbal* sites at the 3' end of the terminator.
CAATAGCAAGCGGCCGCGAAGTTAAAAGCAATGTTGTC (SEQ ID NO:17)
AATCTAGACGTACGCAAAGGCAAAGATTTAAACTC (SEQ ID NO:18)

The resulting PCR fragments were then combined and re-amplified using primers oSAlb-9 and oSAlb-2, thus forming the SA/*Not*I/SA3' cassette, which was subsequently cloned into pUC19 to give pKR132.

Vector pKR187 was constructed by cloning the *Not*I fragment of pKR159, containing the delta-6 desaturase, into the *Not*I site of vector pKR145. Vector pKR145 contains a *Not*I site flanked by the pea leguminA2 promoter and the pea leguminA2 3' transcription terminator (legA2/*NotI*/legA23' cassette). Plasmid pKR145 was constructed by cloning the *Bam*HI/*Sal*I fragment of pKR142, containing the legA2/*NotI*/legA23' cassette, into the *Bam*HI/*Sal*I fragment of KS120 (described above). The legA2/*Not*I/legA23' cassette of pKR142 was flanked by *Bsi*WI sites and contained a Pstl site at the extreme 5' end of legA2 promoter. In addition, this cassette was flanked by *Bam*HI and *Sal*I sites. Plasmid pKR142 was constructed by cloning the *Bsi*WI fragment of pKR140, containing the legA2/*Not*I/legA23' cassette, into the *Bsi*WI site of pKR124, containing a bacterial ori and ampicillin resistance gene. This cloning step introduced the *Sal*I site and allowed further subcloning into pKS124. The legA2/*Not*I/legA23' cassette of pKR140 was made by PCR amplification from pea genomic DNA. The legA2 promoter was amplified from pea genomic DNA using primer LegPro5' (SEQ ID NO:19), designed to introduce *Xba*I and *Bsi*WI sites at the 5' end of the promoter, and primer LegPro3' (SEQ ID NO:20), designed to introduce a *Not*I site at the 3' end of the promoter.
TTTCTAGACGTACGTCCCTTCTTATCTTTGATCTCC (SEQ ID NO:19)
GCGGCCGCAGTTGGATAGAATATATGTTTGTGAC (SEQ ID NO:20)

The legA2 transcription terminator was amplified from pea genomic DNA using primer LegTerm5' (SEQ ID NO:21), designed to introduce *Not*I site at the 5' end of the terminator, and primer LegTerm3' (SEQ ID NO:22), designed to introduce *Bsi*WI and *Xba*I sites at the 3' end of the terminator.
CTATCCAACTGCGGCCGCATTTCGCACCAAATCAATGAAAG (SEQ ID NO:21)
AATCTAGACGTACGTGAAGGTTAAACATGGTGAATATG (SEQ ID NO:22)

The resulting PCR fragments were then combined and re-amplified using primers LegPro5' and LegTerm3', thus forming the legA2/*Not*I/legA23' cassette. The legA2/*Not*I/legA23' cassette PCR fragment was subcloned into the intermediate cloning vector pCR-Script AMP SK(+) (Stratagene) according the manufacturer's protocol to give plasmid pKR140. Plasmid pKR124 contains a *Not*I site flanked by the KTi promoter and the KTi transcription termination region (KTi/*NotI*/KTi3' cassette). In addition, the KTi/*NotI*/KTi3' cassette was flanked by *Bsi*WI sites. The KTi/*Not*I/KTi3' cassette was PCR-amplified from pKS126 using primers oKTi5 (SEQ ID NO:23) and oKTi6 (SEQ ID NO:24), designed to introduce an *Xbal* and *Bsi*WI site at both ends of the cassette.
ATCTAGACGTACGTCCTCGAAGAGAAGGG (SEQ ID NO:23)
TTCTAGACGTACGGATATAATG (SEQ ID NO:24)

The resulting PCR fragment was subcloned into the *Xba*I site of the cloning vector pUC19 to give plasmid pKR124. Plasmid pKS126 is similar to pKS121 (WO 02/00904), the former possessing a second hygromycin phosphotransferase gene that is operably linked to a 35S-CaMV promoter.

Vector pKR189 was constructed by cloning the *Not*I fragment of pKR159, containing the delta-6 desaturase, into the *Not*I site of vector pKR154. Vector pKR154 contains a *Not*I site flanked by the pea leguminaA1 promoter and the pea leguminA2 3' transcription terminator (legA1/*NotI*/legA23' cassette). Vector pKR154 was made by cloning the *Hind*III/*Not*I fragment of pKR151, containing the legA1 3' promoter into the *Hind*III/*Not*I fragment of pKR150. Plasmid pKR151 contained a *Not*I site flanked by the leguminA1 promoter and the leguminA1 3' transcription terminator (legA1/*Not*I/legA13' cassette). In addition, the legA1/*Not*I/legA13' cassette was flanked by *Bsi*WI site. The legA1/*Not*I/legA13' cassette was made by PCR amplification from pea genomic DNA. The legA1 promoter was PCR-amplified using primer LegA1 Pro5' (SEQ ID NO:25), designed to introduce *Xbal* and *Bsi*WI sites at the 5' end of the promoter, and primer LegA1Pro3' (SEQ ID NO:26), designed to introduce a Notl site at the 3' end of the promoter.
TTTCTAGACGTACGGTCTCAATAGATTAAGAAGTTG (SEQ ID NO:25)
GCGGCCGCGAAGAGAGATACTAAGAGAATGTTG (SEQ 10 NO:26)

The legA1 transcription terminator was amplified from pea genomic DNA using primer LegA1Term5'(SEQ ID NO:27), which was designed to introduce *Not*I site at the 5' end of the terminator, and primer LegA1Term3' (SEQ ID NO:28), which was designed to introduce BsiWI and *Xba*I sites at the 3' end of the terminator.
GTATCTCTCTTCGCGGCCGCATTTGGCACCAAATCAATG (SEQ ID NO:27)
TTTCTAGACGTACGTCAAAAAATTTCATTGTAACTC (SEQ ID NO:28)

The resulting PCR fragments were then combined and re-amplified using primer LegA1Pro5' and LegA1Term3', thus forming the legA1/*Not*I/legA13' cassette. The legA1/*Not*I/legA13' cassette PCR fragment was subcloned into the intermediate cloning vector pCR-Script AMP SK(+) (Stratagene) according the manufacturer's protocol to give plasmid pPL1A. The legA1/*Not*I/legA13' cassette was subsequently excised from pPL1A by digestion with *Bsi*WI and cloned into the *Bsi*WI site of pKR145 (described above) to give pKR151. Plasmid pKR150 was constructed by cloning the *BamH*I/*Hind*III fragment of pKR142 (described above), containing the legA2/*Not*I/legA23' cassette into the *BamH*I/*Hind*III site of KS120 (described above).

The amplified soybean β-conglycinin β-subunit promoter fragment (as described in Example 1) was digested with *Bam*H I and *Not*I, purified and cloned into the *Bam*H I and *Not*I sites of plasmid pZBL115 to make pZBL116. The pZBL115 plasmid contains the origin of replication from pBR322, the bacterial HPT hygromycin resistance gene driven by T7 promoter and T7 terminator, and a 35S promoter-HPT-Nos3' gene to serve as a hygromycin resistant plant selection marker. The Not I fragment of pKR159, containing the M. alpina delta 6 desaturase gene, was cloned into Not I site of pZBL116 in the sense orientation to make plant expression cassettes pZBL118.

The amplified soybean glycinin Gy1 promoter fragment (described in Example 1) was digested with *Bam*H I and *Not*I, purified and cloned into the *Bam*H I and *Notl* sites of plasmid pZBL115 to make pZBL117. The Notl fragment of pKR159, containing the *M*. *alpina* delta-6 desaturase gene, was cloned into *Not*l site of pZBL117 in the sense orientation to make plant expression cassettes pZBL119.

Based on the sequence of the soybean annexin promoter (SEQ ID NO:3), as described in Example 1, two oligos with either *Bam*H I or *Not*l sites at the 5' ends were designed to re-amplify the promoter. The oligonucleotide sequences of these two oligos are shown in SEQ ID NO:29 and SEQ ID NO:30.
CGCGGATCCATCTTAGGCCCTTGATTATATGGTGTTT (SEQ ID NO:29)
GAATTCGCGGCCGCTGAAGTATTGCTTCTTAGTTAACCTTTCC (SEQ ID NO:30)

Based on the sequences of cloned soybean BD30 promoter (SEQ ID NO:6), as described in Example 1, two oligos with either BamH I or Not I sites at the 5' ends were designed to re-amplify the BD30 promoter. The oligonucleotide sequences of these two oligos are shown in SEQ ID NO:31 and SEQ ID NO:32.
CGCGGATCCAACTAAAAAAAGCTCTCAAATTACATITTGAG (SEQ ID NO:31)
GAATTCGCGGCCGCAACTTGGTGGAAGAATTTTATGATTTGAAA (SEQ ID NO:32)

The re-amplified annexin and BD30 promoter fragments were digested with *Bam*H I and *Not*l, purified and cloned into the *Bam*H I and Notl sites of plasmid pZBL115 to make pJS88 and pJS89, respectively. The pZBL115 plasmid contains the origin of replication from pBR322, the bacterial HPT hygromycin resistance gene driven by T7 promoter and T7 terminator, and a 35S promoter-HPT-Nos3' gene to serve as a hygromycin resistant plant selection marker. The *M*. *alpina* delta-6 desaturase gene was cloned into *Not*l site of pJS88 and pJS89, in the sense orientation, to make plant expression cassettes pJS92 and pJS93, respectively.

### EXAMPLE 3

### Cloning of Individual EPA Biosynthetic Pathway_Genes for Expression In Somatic Soybean Embryos

Each of the EPA biosynthetic genes was tested individually in order to assess their activities in somatic soybean embryos before combining for large-scale production transformation into soybean. Each gene was cloned into an appropriate expression cassette as described below. For the *M*. *alpina* delta-5 desaturase and elongase, both genes were combined together on one plasmid. The genes and promoters used, and the corresponding vector names are listed in Table 3.

**TABLE 3**

| EPA BIOSYNTHETIC GENES EXPRESSED IN SOYBEAN SOMATIC EMBRYOS | | | | |
|---|---|---|---|---|
| Activity | Source Organism | Sequence (DNA) | Sequence (Protein) | Vector |
| Delta-6 desaturase | *M. alpina* | SEQ ID NO:33 | SEQ ID NO:34 | pKR162 |
| Delta-6 desaturase, | *S. diclina* | SEQ ID NO:35 | SEQ ID NO:36 | pKS208 |
| Delta-5 desaturase | *S. diclina* | SEQ ID NO:37 | SEQ ID NO:38 | pKR305 |
| elongase | *T. aureum* | SEQ ID NO:39 | SEQ ID NO:40 | pKS209 |
| Delta-17 desaturase | *S. diclina* | SEQ ID NO:41 | SEQ ID NO:42 | pKS203 |
| elongase | *M*. *alpina* | SEQ ID NO:43 | SEQ ID NO:44 | pK8134 |
| Delta-5 desaturase | *M. alpina* | SEQ ID NO:45 | SEQ ID NO:46 | pKS134 |

Construction of pKR162, for soy expression studies with the *M. alpina* delta-6 desaturase, was described in Example 2.

The *S*. *diclina* delta-6 desaturases was cloned into the *Notl* site of the βcon/*Notl*/Phas3' cassette of vector pKS123. The gene for the *S*. *diclina* delta-6 desaturase was removed from pRSP1 by digestion with *Eco*RI and *Hind*III. The ends of the resulting DNA fragment were filled and the fragment was cloned into the filled Notl site of pKS123 to give pKS208.

To release the *S*. *diclina* delta-5 desaturase from plasmid pRSP3, it was first digested with *Xho*l*,* the *Xho*l ends were filled, and the plasmid was then digested with *Eco*RI. The delta-5 desaturase-containing fragment was then cloned into pKR288 that had been digested with *Mfe*l and *Eco*RV to give pKR305. Plasmid pKR288 was identical to pKS123 except that a linker containing the *Mfe*l (on the promoter side) and *Eco*RV (on the 3' terminal side) sites had been inserted into the *Not*l site of the βcon/*Not*l/Phas3' cassette. This allowed for directional cloning of the delta-5 desaturase, which contained internal *Not*l sites, into pKS123. Construction of pKR288 is more thoroughly described in Example 13.

The *T, aureum* elongase was cloned into the *Not*l site of the βcon/*Notl*/Phas3' cassette of vector pKS123. The gene for the *T. aureum* elongase was removed from pRAT-4-A7 by digestion with EcoRI. The ends of the resulting DNA fragment were filled and the fragment was cloned into the filled *Not*l site of pKS123 to give pKS209.

The gene for the *S*. *diclina* delta-1 7 desaturase (Example 6) was amplified from pRSP19 using primers RSP19forward (SEQ ID NO:53) and RSP19reverse (SEQ ID NO:54) which were designed to introduce *Not*l restriction enzyme sites at both ends of the delta-17 desaturase.
GCGGCCGCATGACTGAGGATAAGACGA (SEQ ID NO:53)
GCGGCCGCTTAGTCCGACTTGGCCTTG (SEQ ID NO:54)

The resulting PCR fragment was subcloned into the intermediate cloning vector pGEM-T easy (Promega) according the manufacturer's protocol to give plasmid pRSP19/pGEM. The gene for the *S*. *diclina* delta-17 desaturase was released from pRSP19/pGEM by partial digestion with *Not*l and cloned into the *Not*l site of pKS123 to give pKS203.

In plasmid pKS134, both the *M*, *alpina* elongase and *M. alpina* delta-5 desaturase were cloned behind the β-conglycinin promoter followed by the phaseolin 3' transcription terminator (β con/Maelo/Phas3' cassette, βcon/Mad5/Phas3' cassette). Plasmid pKS134 was constructed by cloning the *Hind*III fragment of pKS129, containing the βcon/Mad5/Phas3' cassette, into a *Hind*III site of partially digested pKS128, containing the βcon/Maelo/Phas3' cassette, the T7prom/hpt/T7term cassette and the bacterial ori region. The gene for the *M*. *alpina* elongase was amplified from pRPB2 using primers RPB2foward (SEQ ID NO:55) and RPB2reverse (SEQ ID NO:56) which were designed to introduce *Not*l restriction enzyme sites at both ends of the elongase.
GCGGCCGCATGGAGTCGATTGCGC (SEQ ID NO:55)
GCGGCCGCTTACTGCAACTTCCTT (SEQ ID NO:56)

The resulting PCR fragment was digested with *Not*l and cloned into the *Not*l site of pKS119, containing a βcon/*Not*l/Phas3' cassette, the T7prom/hpt/T7term cassette and the bacterial *on* region, to give pKS128. Plasmid pKS119 is identical to pKS123, except that the 35S/HPT/NOS3' cassette had been removed. The gene for the *M*. *alpina* delta-5 desaturase was amplified from pCGR4 using primers CGR4foward (SEQ ID NO:57) and CGR4reverse (SEQ ID NO:58) which were designed to introduce *Not*l restriction enzyme sites at both ends of the desaturase.
GCGGCCGCATGGGAACGGACCAAG (SEQ ID NO:57)
GCGGCCGCCTACTCTTCCTTGGGA (SEQ ID NO:58)

The resulting PCR fragment was digested with *Not*l and cloned into the *Notl* site of pKS119, containing a βcon/*Not*l/Phas3' cassette flanked by *Hind*III sites, to give pKS129.

### EXAMPLE 4

### Assembling EPA biosynthetic pathway genes for expression in Somatic Soybean Embryos and Soybean Seeds (pKR274)

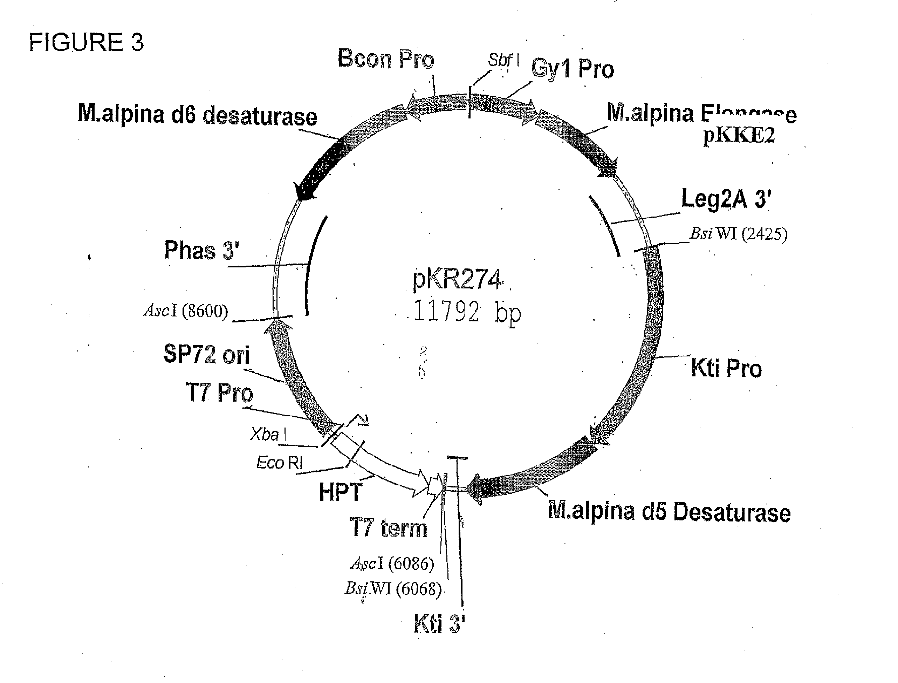

The *M*. *alpina* delta-6 desaturase, *M. alpina* elongase and *M*. *alpina* delta-5 desaturase were cloned into plasmid pKR274 (Figure 3) behind strong, seed-specific promoters allowing for high expression of these genes in somatic soybean embryos and soybean seeds. The delta-6 desaturase was cloned behind the promoter for the α' subunit of β-conglycinin [Beachy et al., (1985) EMBO J. 4:3047-3053] followed by the 3' transcription termination region of the phaseolin gene [Doyle, J.J. et al. (1986) J. Biol. Chem. 261:9228-9238] (βcon/Mad6/Phas3' cassette). The delta-5 desaturase was cloned behind the Kunitz soybean Trypsin Inhibitor (KTi) promoter [Jofuku et al., (1989) Plant Cell 1:1079-1093], followed by the KTi 3' termination region, the isolation of which is described in US Patent 6,372,965 (KTi/Mad5/KTi3' cassette). The elongase was cloned behind the glycinin Gy1 promoter followed by the pea leguminA2 3' termination region (Gy1/Maelo/legA2 cassette). All of these promoters exhibit strong tissue specific expression in the seeds of soybean. Plasmid pKR274 also contains the hygromycin B phosphotransferase gene [Gritz, L. and Davies, J. (1983) Gene 25:179-188] cloned behind the T7 RNA polymerase promoter and followed by the T7 terminator (T7prom/HPT/T7term cassette) for selection of the plasmid on hygromycin B in certain strains of *E. coli,* such as NovaBlue(DE3) (Novagen, Madison, WI), which is lysogenic for lambda DE3 (and carries the T7 RNA polymerase gene under *lacUV5* control). In addition, plasmid pKR274 contains a bacterial origin of replication (*ori*) functional in *E*. *coli* from the vector pSP72 (Stratagene).

Plasmid pKR274 was constructed in many steps from a number of different intermediate cloning vectors. The Gy1/Maelo/legA2 cassette was released from plasmid pKR270 by digestion with *Bsi*WI and *Sbf*I and was cloned into the *Bsi*WI/*Sbf*I sites of plasmid pKR269, containing the delta-6 desaturase, the T7prom/hpt/T7term cassette and the bacterial ori region. This was designated as plasmid pKR272. The KTi/Mad5/KTi3' cassette, released from pKR136 by digestion with *Bsi*WI, was then cloned into the *Bsi*WI site of pKR272 to give pKR274. A description for plasmid construction for pKR269, pKR270 and pKR136 is provided below.

Plasmid pKR159 (described in Example 2) was digested with Notl to release the *M. alpina* delta-6 desaturase, which was, in turn, cloned into the *Not*l site of the soybean expression vector pKR197 to give pKR269. Vector pKR197 contains a βcon/*Notl*/Phas3' cassette, the T7prom/hpt/T7term cassette and the bacterial *ori* region. Vector pKR197 was constructed by combining the Asp fragment from plasmid pKS102 (WO 02/00905), containing the T7prom/hpt/T7term cassette and bacterial *ori*, with the *Asc*I fragment of plasmid pKR72, containing the βcon/*Notl*/Phas cassette. Vector pKR72 is identical to the previously described vector pKS123 (WO 02/08269), except that *Sbf*I, F*se*I and *Bsi*WI restriction enzyme sites were introduced between the *Hind*III and *Bam*HI sites in front of the β-conglycinin promoter.

The gene for the *M. alpina* elongase was PCR-amplified (described in Example 3) digested with *Not*l and cloned into the *Not*l site of vector pKR263 to give pKR270. Vector pKR263 contains a *Not*l site flanked by the promoter for the glycininGy1 gene and the leguminA2 3' transcription termination region (Gy1/*Notl*/legA2 cassette), In addition, the Gy1/*Not*l/legA2 cassette was flanked by Sbfl and *Bsi*WI sites. Vector pKR263 was constructed by combining the *Pst*l/*Not*l fragment from plasmid pKR142, containing the leguminA2 3' transcription termination region, an ampicillin resistance gene and bacterial ori with the *Pst*l*Not*l fragment of plasmid pSGly12, containing the glycininGy1 promoter. The glycininGy1 promoter was amplified from pZBL119 (described in Example 2) using primer oSGly-I (SEQ ID NO:59), designed to introduce an *Sbf*l*lPst*l site at the 5' end of the promoter, and primer oSGly-2 (SEQ ID NO:60), designed to introduce a *Not*l site at the 3' end of the promoter.
TTCCTGCAGGCTAGCCTAAGTACGTACTC . (SEQ ID NO:59)
AAGCGGCCGCGGTGATGACTG (SEQ ID NO:60)

The resulting PCR fragment was subcloned into the intermediate cloning vector pCR-Script AMP SK(+) (Stratagene) according the manufacturer's protocol to give plasmid pSGly12. Construction of pKR142, containing the legA2[*Not*l/legA23' cassette is described in Example 2. The gene for the *M*. *alpina* delta-5 desaturase was PCR-amplified as described in Example 3, digested with Notl and cloned into the *Not*l site of vector pKR124 (described in Example 2) to give pKR136.

### EXAMPLE 5

### Assembling EPA biosynthetic pathway genes for expression in Somatic Soybean Embryos and Soybean Seeds (pKKE2)

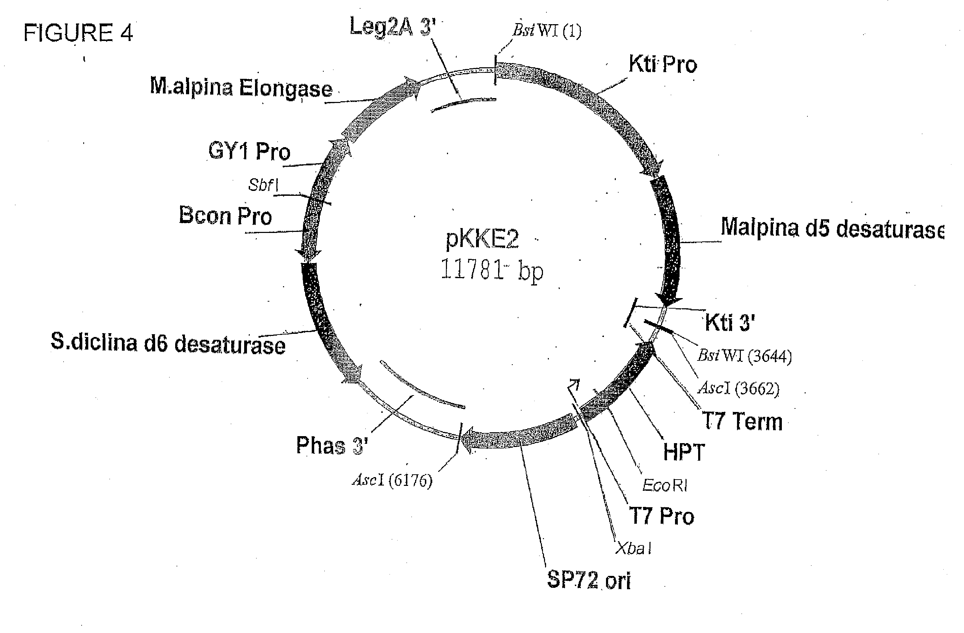

The *S*. *diclina* delta-6 desaturase, *M*. *alpina* elongase and *M. alpina* delta-5 desaturase were cloned into plasmid pKKE2 (Figure 4) behind strong, seed-specific promoters allowing for high expression of these genes in somatic soybean embryos and soybean seeds. Plasmid pKKE2 was identical to pKR274, described in Example 4, except that in pKKE2 the *M*. *alpina* delta-6 desaturase was replaced with the *S*. *diclina* delta-6 desaturase. As in pKR274, the *S*. *diclina* delta-6 desaturase was cloned behind the promoter for the α' subunit of β-conglycinin followed by the 3' transcription termination region of the phaseolin gene (βcon/Sdd6/Phas3' cassette).

Plasmid pKKE2 was constructed from a number of different intermediate cloning vectors as follows: The βcon/Sdd6/Phas3' cassette was released from plasmid pKS208 (described in Example 2) by digestion with *Hind*III and was cloned into the *Hind*III site of plasmid pKR272 (Example 3) to give pKR301. The KTi/Mad5/KTi3' cassette, released from pKR136, (Example 4) by digestion with *Bsi*WI was then donned into the *Bsi*WI site of pKR301 to give pKKE2.

### EXAMPLE 6

### Cloning of S.diclina (ATCC 56851) delta-17 desaturase,

### Construction of Saproleginia diclina (ATCC 56851) cDNA Library

To isolate genes encoding for functional desaturase enzymes, a cDNA library was constructed. *Saprolegnia diclina* cultures were grown in potato dextrose media (Difco # 336, BD Diagnostic Systems, Sparks, MD) at room temperature for four days with constant agitation. The mycelia were harvested by filtration through several layers of cheesecloth, and the cultures were crushed in liquid nitrogen using a mortar and pestle. The cell lysates were resuspended in RT buffer (Qiagen, Valencia, California) containing β-mercaptoethanol and incubated at 55°C for three minutes. These lysates were homogenized either by repeated aspirations through a syringe or over a "Qiashredder"-brand column (Qiagen). The total RNA was finally purified using the "RNeasy Maxi"-brand kit (Qiagen), as per the manufacturer's protocol.

mRNA was isolated from total RNA from each organism using an oligo dT cellulose resin. The "pBluescript II XR"-brand library construction kit (Stratagene, La Jolla, CA) was used to synthesize double-stranded cDNA. The double-stranded cDNA was then directionally cloned (5' *EcoRII3' Xhol)* into pBluescript II SK(+) vector (Stratagene). The *S*. *diclina* library contained approximately 2.5 x 10⁶ clones, each with an average insert size of approximately 700 bp. Genomic DNA of *S*. *diclina* was isolated by crushing the culture in liquid nitrogen followed by purification using the "Genomic DNA Extraction"-brand kit (Qiagen), as per the manufacturer's protocol.

### Determination of Codon Usage in Saprolegnia diclina

The 5' ends of 350 random cDNA clones were sequenced from the *Saprolegnia diclina* cDNA library described above. The sequences were translated into six reading frames using GCG program (Genetics Computer Group, Madison, WI) with the "FastA"-brand algorithm to search for similarity between a query sequence and a group of sequences of the same type, specifically within the GenBank database. Many of the clones were identified as putative housekeeping genes based on protein homology to known genes. Eight *S*. *diclina* cDNA sequences were thus selected. Additionally, the full-length *S*. *diclina* delta 5-desaturase and delta 6-desaturase sequences were also used (see Table 4 below). These sequences were then used to generate the *S, diclina* codon bias table shown in Table 2 below by employing the "CodonFrequency" program from GCG (Madison, WI).

**TABLE 4**

| GENES FROM *Saprolegnia diclina* USED IN CODON BIAS TABLE | | | |
|---|---|---|---|
| Clone | Database Match | # bases | # amino acids |
| 3 | Actin gene | 615 | 205 |
| 20 | Ribosomal protein L23 | 420 | 140 |
| 55 | Heat Shock protein 70 gene | 468 | 156 |
| 83 | Glyceraldehyde-3-P-dehydrogenase gene | 588 | 196 |
| 138 | Ribosomal protein S13 gene | 329 | 110 |
| 179 | Alpha-tubulin 3 gene | 591 | 197 |
| 190 | Casein kinase II alpha subunit gene | 627 | 209 |
| 250 | Cyclophilin gene | 489 | 163 |
| | Delta 6-desaturase | 1362 | 453 |

| Clone | Database Match | # bases | # amino acids |
|---|---|---|---|
| | Delta 5-desaturase | 1413 | 471 |
| | Total | 6573 | 2191 |

**TABLE 5**

| CODON BIAS TABLE FOR *Saprolegnia diclina* | | |
|---|---|---|
| Amino acid | Codon Bias | % used |
| Ala | GCC | 55% |
| Arg | CGC | 50% |
| Asn | AAC | 94% |
| Asp | GAC | 85% |
| Cys | TGC | 77% |
| Gln | CAG | 90% |
| Glu | GAG | 80% |
| Gly | GGC | 67% |
| His | CAC | 86% |
| Ile | ATC | 82% |
| Leu | CTC | 52% |
| Lys | AAG | 87% |
| Met | ATG | 100% |
| Phe | TTC | 72% |
| Pro | CCG | 55% |
| Ser | TCG | 47% |
| Thr | ACG | 46% |
| Trp | TGG | 100% |
| Tyr | TAC | 90% |
| Val | GTC | 73% |
| Stop | TGA | 67% |

### Design of Degenerate Oligonucleotides for the Isolation of an Omega-3 Desaturase from Saprolegnia diclina (ATCC 56851)

The method for identification of a delta-17 desaturase (an omega-3 desaturase) gene from *S*. *diclina* involved PCR amplification of a region of the putative desaturase gene using degenerate oligonucleotides (primers) that contained conserved motifs present in other known omega-3 desaturases. Omega-3 desaturases from the following organisms were used for the design of these degenerate primers: *Arabidopsis thaliana* (Swissprot # P46310), *Ricunus communis* (Swissprot # P48619), *Glycine max* (Swissprot # P48621), *Sesamum indium* (Swissprot # P48620), *Nicotiana tabacum* (GenBank # D79979), *Perilla frutescens* (GenBank # U59477), *Capsicum annuum* (GenBank # AF222989), *Limnanthes douglassi* (GenBank # U17063), and *Caenorhabditis elegans* (GenBank # L41807). Some primers were designed to contain the conserved histidine-box motifs that are important components of the active site of the enzymes. See Shanklin, J.E., McDonough, V.M., and Martin, C.E. (1994) Biochemistry 33, 12787-12794,

Alignment of sequences was carried out using the CLUSTALW Multiple Sequence Alignment Program (Thompson, J.D. et al. (1994) Nucl. Acids Res. 22:4673-4680).

The following degenerate primers were designed and used in various combinations:
Protein Motif 1: NH₃- TRAAIPKHCWVK -COOH (SEQ ID NO:61)
Primer RO 1144 (Forward):
ATCCGCGCCGCCATCCCCAAGCACTGCTGGGTCAAG (SEQ ID NO: 62)
Protein Motif 2: NH₃- ALFVLGHDCGHGSFS -COOH (SEQ ID NO:63)
   This primer contains the histidine-box 1 (underlined).
Primer RO 1119 (Forward):
GCCCTCTTCGTCCTCGGCCAYGACTGCGGCCAYGGCTCGTTCTCG (SEQ ID NO: 64).
Primer RO 1118 (Reverse):
GAGRTGGTARTGGGGGATCTGGGGGAAGARRTGRTGGRYGACRTG (SEQ ID NO: 65).
Protein Motif 3: NH₃- PYHGWRISHRTHHQN -COOH (SEQ ID NO:66)
   This primer contains the histidine-box 2 (underlined).
Primer RO 1121 (Forward):
CCCTACCAYGGCTGGCGCATCTCGCAYCGCACCCAYCAYCAGAAC (SEQ ID NO: 67).
Primer RO 1122 (Reverse):
GTTCTGRTGRTGGGTCCGRTGCGAGATGCGCCAGCCRTGGTAGGG (SEQ ID NO: 68).
Protein Motif 4: NH₃- GSHF D/H P D/Y SDLFV -COOH (SEQ ID NO:69)
Primer RO 1146 (Forward):
GGCTCGCACTTCSACCCCKACTCGGACCTCTTCGTC (SEQ ID NO: 70).
Primer RO 1147 (Reverse):
GACGAAGAGGTCCGAGTMGGGGTWGAAGTGCGAGCC (SEQ ID NO: 71).
Protein Motif 5: NH₃- WS Y/F L/V RGGLTT L/I DR -COOH (SEQ ID NO:72)
Primer RO 1148 (Reverse):
GGGCTGGAKGGTGGTGAGGGGGGGGGGGAWGSAGGACCA (SEQ ID NO: 73)
Protein Motif 6: NH₃- HHDIGTHVIHHLFPQ -COOH (SEQ ID NO:74)
   This sequence contains the third histidine-box (underlined).
Primer RO 1114 (Reverse):
CTGGGGGAAGAGRTGRTGGATGACRTGGGTGCCGATGTCRTGRTG (SEQ ID NO: 75).
Protein Motif 7: NH₃- H L/F FP Q/K IPHYHL V/I EAT -COOH (SEQ ID NO:76)
Primer RO 1116 (Reverse):
GGTGGCCTCGAYGAGRTGGTARTGGGGGATCTKGGGGAAGARRTG (SEQ ID NO: 77).
Protein Motif 8: NH₃- HV A/I HH L/F FPQIPHYHL -COOH (SEQ ID NO:78)
   This primer contains the third histidine-box (underlined) and accounts for differences between the plant omege-3 desaturases and the *C*. *elegans* omega-3-desaturase.

The nucleic acid degeneracy code used for SEQ. ID. NOS: 62 through 77 was as follows. R= A/G; Y=C/T; M=A/C; K=G/T; W=A/T; S=C/G; B=C/G/T; D=A/G/T; H=A/C/T; V=A/C/G; and N=A/C/G/T.

### Identification and Isolation of Delta-17 Desaturase Gene from Saprolegnia diclina (ATCC 56851)

Various sets of the degenerate primers above were used in PCR amplification reactions, using as a template either the *S*. *diclina* cDNA library plasmid DNA, or *S*. *diclina* genomic DNA. Also various different DNA polymerases and reaction conditions were used for the PCR amplifications. These reactions variously involved using "Platinum Taq"-brand DNA polymerase (Life Technologies Inc., Rockville, MD), or cDNA polymerase (Clontech, Palo Alto, CA), or Taq PCR-mix (Qiagen), at differing annealing temperatures.

PCR amplification using the primers RO 1121 (Forward) (SEQ. ID. NO:67) and RO 1116 (Reverse) (SEQ. ID. NO:77) resulted in the amplification of a fragment homologous to a known omega-3 desaturase. The RO 1121 (Forward) primer corresponds to the protein motif 3; the RO 1116 (Reverse) primer corresponds to the protein motif 7.

PCR amplification was carried out in a 50 µl total volume containing: 3 µl of the cDNA library template, PCR buffer containing 40 mM Tricine-KOH (pH 9.2), 15 mM KOAc, 3.5 mM Mg(OAc)₂, 3.75 µg/ml BSA (final concentration), 200 µM each deoxyribonucleotide triphosphate, 10 pmole of each primer and 0,5 µl of "Advantage"-brand cDNA polymerase (Clontech). Amplification was carried out as follows: initial denaturation at 94°C for 3 minutes, followed by 35 cycles of the following: 94°C for 1 min, 60°C for 30 sec, 72°C for 1 min. A final extension cycle of 72°C for 7 min was carried out, followed by reaction termination at 4°C.

A single ~480 bp PCR band was generated which was resolved on a 1 % "SeaKem Gold"-brand agarose gel (FMC BioProducts, Rockland, ME), and gel-purified using the Qiagen Gel Extraction Kit. The staggered ends on the fragment were "filled-in" using T4 DNA polymerase (Life Technologies, Rockville, MD) as per the manufacturer's instructions, and the DNA fragments were cloned into the PCR-Blunt vector (Invitrogen, Carlsbad, CA). The recombinant plasmids were transformed into TOP10 supercompetent cells (Invitrogen), and eight clones were sequenced and a database search (Gen-EMBL) was carried out.

Clones "sdd17-7-1" to "sdd17-7-8' were all found to contain and ~483 bp insert. The deduced amino acid sequence from this fragment showed highest identity to the following proteins (based on a "tFastA" search):
1. 37.9% identity in 161 amino acid overlap with an omega-3 (delta-15) desaturase from *Synechocystis* sp. (Accession # D13780).
2. 40.7% identity in 113 amino acid overlap with *Picea abies* plastidic omega-3 desaturase (Accession # AJ302017).
3. 35.9% identity in 128 amino acid overlap with *Zea mays* FAD8 fatty acid desaturase (Accession # D63953).

Based on its sequence homology to known omega-3 fatty acid desaturases, it seemed likely that this DNA fragment was part of a delta-17 desaturase gene present in *S*. *diclina.*

The DNA sequence identified above was used in the design oligonucleotides to isolate the 3' and the 5' ends of this gene from the *S*. *diclina* cDNA library. To isolate the 3' end of the gene, the following oligonucleotides were designed:
RO 1188 (Forward): 5'-TACGCGTACCTCACGTACTCGCTCG-3' (SEQ ID NO: 79)
RO 1189 (Forward): TTCTTGCACCACAACGACGAAGCGACG (SEQ ID NO: 80)
RO 1190 (Forward): GGAGTGGACGTACGTCAAGGGCAAC (SEQ ID NO: 81)
RO 1191 (Forward): TCAAGGGCAACCTCTCGAGCGTCGAC (SEQ ID NO: 82)

These primers (SEQ ID NOS: 79-82) were used in combinations with the pBluescript SK(+) vector oligonucleotide:
RO 898: 5'-CCCAGTCACGACGTGTAAAA CGACGGCCAG-3' (SEQ ID NO: 83).

PCR amplifications were carried out using either the "Taq PCR Master Mix" brand polymerase (Qiagen) or "Advantage"-brand cDNA polymerase (Clontech) or "Platinum"-brand Taq DNA polymerase (Life Technologies), as follows:

For the "Taq PCR Master Mix" polymerase, 10 pmoles of each primer were used along with 1 µl of the cDNA library DNA from Example 1. Amplification was carried out as follows: initial denaturation at 94°C for 3 min, followed by 35 cycles of the following: 94°C for 1 min, 60°C for 30 sec, 72°C for 1 min. A final extension cycle of 72°C for 7 min was carried out, followed by the reaction termination at 4°C. This amplification resulted in the most distinct bands as compared with the other two conditions tested.

For the "Advantage"-brand cDNA polymerase reaction, PCR amplification was carried out in a 50 µl total volume containing: 1 µl of the cDNA library template from Example 1, PCR buffer containing 40 mM Tricine-KOH (pH 9.2), 15 mM KOAc, 3.5 mM Mg(OAc)₂, 3.75 µg/ml BSA (final concentration), 200 µM each deoxyribonucleotide triphosphate, 10 pmole of each primer and 0.5 µl of cDNA polymerase (Clontech). Amplification was carried out as described for the Taq PCR Master Mix.

For the "Platinum"-brand Taq DNA polymerase reaction, PCR amplification was carried out in a 50 µl total volume containing: 1 µl of the cDNA library template from Example 1, PCR buffer containing 20 mM Tris-Cl, pH 8.4, 50 mM KCl (final concentration), 200 µM each deoxyribonucleotide triphosphate, 10 pmole of each primer, 1.5 mM MgSO₄ and 0.5 µl of Platinum Taq DNA polymerase. Amplification was carried out as follows: initial denaturation at 94°°C for 3 min, followed by 30 cycles of the following: 94°C for 45 sec, 55°C for 30 sec, 68°C for 2 min. The reaction was terminated at 4°C.

All four sets of primers in combination with the vector primer generated distinct bands. PCR bands from the combination (RO 1188 + RO 898) were >500 bp and this was gel-purified and cloned separately. The PCR bands generated from the other primer combinations were <500 bp. The bands were gel-purified, pooled together, and cloned into PCR-Blunt vector (Invitrogen) as described earlier. The recombinant plasmids were transformed into TOP10 supercompetent cells (Invitrogen) and clones were sequenced and analyzed.

Clone "'sdd17-16-4" and "sdd16-6" containing the ~500 bp insert, and clones "sdd17-17-6" "sdd17-17-10," and "sdd17-20-3" containing the ~400 bp inserts, were all found to contain the 3'-end of the putative delta-17 desaturase. These sequences overlapped with each other, as well as with the originally identified fragment of this putative omega-3 desaturase gene. All of the sequences contained the 'TAA' stop codon and a poly-A tail typical of 3'-ends of eukaryotic genes. This 3'-end sequence was homologous to other known omega-3 desaturases, sharing the highest identity (41.5% in 130 amino acid overlap) with the *Synechocystis* delta-15 desaturase (Accession # D13780).

For the isolation of the 5'-end of the this gene, the following oligonucleotides were designed and used in combinations with the pBluescript SK(+) vector oligonucleotide:
RO 899: 5'- AGCGGATAACAATTTCACACAGGAAACAGC -3' (SEQ ID NO: 84)
RO 1188 (Reverse):GGTAAAAGATCTCGTCCTTGTCGATGTTGC (SEQ ID NO: 85).
RO 1186 (Reverse): 5'-GTCAAAGTGGCTCATCGTGC-3' (SEQ ID NO: 86)
RO 1187 (Reverse): CGAGCGAGTACGTGAGGTACGCGTAC (SEQ ID NO: 87)

Amplifications were carried out using either the "Taq PCR Master Mix"-brand polymerase (Qiagen) or the "Advantage"-brand cDNA polymerase (Clontech) or the "Platinum"-brand Taq DNA polymerase (Life Technologies), as described hereinabove for the 3' end isolation.

PCR bands generated from primer combinations (RO 1185 or RO 1186 + RO 899) were between ∼580 to ∼440 bp and these were pooled and cloned into PCR-Blunt vector as described above. Clones thus generated included "sdd17-14-1," "sdd17-14-10," "sdd 17-18-2," and "sdd17-18-8," all of which showed homology with known omega-3 desaturases.

Additionally, bands generated from (RO 1187 + RO 899) were ∼ 680 bp, and these were cloned separately to generate clones "sdd 17-22-2" and "sdd 17-22-5" which also showed homology with known omega-3 desaturases. All these clones overlapped with each other, as well as with the original fragment of the unknown putative delta-17 desaturase. These sequences contained an 'ATG' site followed by an open reading frame, indicating that it is the start site of this gene. These fragments showed highest identity (39.7% in 146 amino acid overlap) with the delta-15 desaturase from *Calendula officinalis* (Accession # AJ245938).

The full-length reading frame for this delta-17 desaturase was obtained by PCR amplification of the S. *diclina* cDNA library using the following oligonucleotides:
RO 1212 (Forward):
   5'-TGAACAGAATTCATGACCGAGGATAAGACGAAGGTCGAGTTCCCG-3' (SEQ ID NO: 88)
This primer contains the 'ATG' start site (single underline) followed by the 5' sequence of the omega-3 desaturase. In addition, an *EcoRI* site (double underline) was introduced upstream of the start site to facilitate cloning into the yeast expression vector pYX242.
RO 1213 (Reverse):
   5'-AAAAGAAAGCTTCGCTTCCTAGTCTTAGTCCGACTTGGCCTTGGC-3' (SEQ ID NO: 89)

This primer contains the 'TAA' stop codon (single underline) of the gene as well as sequence downstream from the stop codon. This sequence was included because regions within the gene were very G+C rich, and thus could not be included in the design of oligonucleotides for PCR amplification. In addition, a *HindI*II site (double underline) was included for convenient cloning into a yeast expression vector pYX242.

PCR amplification was carried out using the "Taq PCR Master Mix"-brand polymerase (Qiagen), 10 pmoles of each primer, and 1 µl of the cDNA library DNA from Example 1. Amplification was carried out as follows: initial denaturation at 94°C for 3 min, followed by 35 cycles of the following: 94°C for 1 min, 60°C for 30 sec, 72°C for 1 min. A final extension cycle of 72°C for 7 min was carried out, followed by the reaction termination at 4°C.

A PCR band of ∼1 kb was thus obtained and this band was isolated, purified, cloned into PCR-Blunt vector (Invitrogen), and transformed into TOP10 cells. The inserts were sequenced to verify the gene sequence. Clone "sdd17-27-2" was digested with *EcoR*I / *Hind*III to release the full-length insert, and this insert was cloned into yeast expression vector pYX242, previously digested with *EcoR*I / *Hind*III. This construct contained 1077 bp of sdd17 cloned into pYX242. This construct was labeled pRSP19.

### EXAMPLE 7

### Assembly of EPA biosynthetic pathway genes for expression in Somatic Soybean Embryos and Soybean Seeds (pKR275)

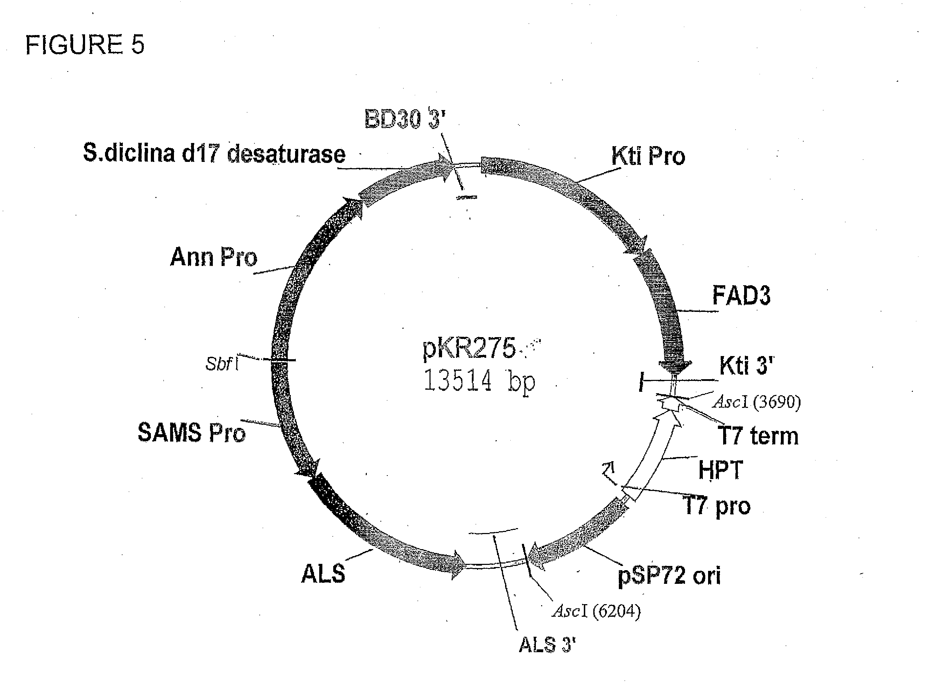

The *Arabidopsis* Fad3 gene [Yadav, N.S. et al. (1993), Plant Physiol. 103:467-76] and S. *diclina* delta-17 desaturase were cloned into plasmid pKR275 (Figure 5) behind strong, seed-specific promoters allowing for high expression of these genes in somatic soybean embryos and soybean seeds. The Fad3 gene SEQ ID NO:47, and its protein translation product in SEQ ID NO:48, was cloned behind the KTi promoter, and upstream of the KTi 3' termination region (KTi/Fad3/KTi3' cassette). The S. *diclina* delta-17 desaturase was cloned behind the soybean annexin promoter followed by the soy BD30 3' termination region (Ann/Sdd17/BD30 cassette). Plasmid pKR275 also contains a mutated form of the soy acetolactate synthase (ALS) that is resistant to sulfonylurea herbicides. ALS catalyzes the first common step in the biosynthesis of the branched chain amino acids isoleucine, leucine, and valine (Keeler et al, Plant Physiol 1993 102: 1009-18). Inhibition of native plant ALS by several classes of structurally unrelated herbicides including sulfonylureas, imidazolinones, and triazolopyrimidines, is lethal (Chong CK, Choi JD Biochem Biophys Res Commun 2000 279:462-7). Overexpression of the mutated sulfonylurea-resistant ALS gene allows for selection of transformed plant cells on sulfonylurea herbicdes. The ALS gene is cloned behind the SAMS promoter (described in WO 00/37662). This expression cassette is set forth in SEQ ID NO:90. In addition, plasmid pKR275 contains a bacterial *ori* region and the T7prom/HPT/T7term cassette for replication and selection of the plasmid on hygromycin B in bacteria.

Plasmid pKR275 was constructed from a number of different intermediate cloning vectors as follows: The KTi/Fad3/KTi3' cassette was released from plasmid pKR201 by digestion with *Bsi*WI and was cloned into the *Bsi*WI site of plasmid pKR226, containing the ALS gene for selection, the T7prom/hpt/T7term cassette and the bacterial *ori* region. This was designated plasmid pKR273. The Ann/Sdd17/BD30 cassette, released from pKR271 by digestion with *Pst*I, was then cloned into the *Sbf*I site of pKR273 to give pKR275. A detailed description for plasmid construction for pKR226, pKR201 and pKR271 is provided below.

Plasmid pKR226 was constructed by digesting pKR218 with *Bsi*WI to remove the legA2/Noti/legA3' cassette. Plasmid pKR213 was made by combining the filled *Hind*III/*Sbf*I fragment of pKR217, containing the *leg*A2/*Not*I/legA23' cassette, the bacterial *ori* and the T7prom/HPT/T7term cassette, with the *Pst*I/*Sma*I fragment of pZSL131euB, containing the SAMS/ALS/ALS3' cassette. Plasmid pKR217 was constructed by cloning the BamHI/HindIII fragment of pKR142 (described in Example 2), containing the legA2/NotI/legA23' cassette, into the *Bam*HI/*Hind*III site of KS102. The Arabidopsis Fad3 gene was released from vector pKS131 as a *Not*I fragment and cloned into the *Not*I site of pKR124 (described in Example 2) to form pKR201. The *Not*I fragment from pKS131 is identical to that from pCF3 [Yadav, N.S. et al (1993) Plant Physiol. 103:467-76])

The gene for the S. *diclina* delta-17 desaturase was released from pRSP19/pGEM (described in Example 2) by partial digestion with *Not*I, and it was then cloned into the *NotI* site of pKR268 to give pKR271. Vector pKR268 contains a *Not*I site flanked by the annexin promoter and the BD30 3' transcription termination region (Ann/*NotI*/BD30 cassette). In addition, the Ann/*Not*I/BD30 cassette was flanked by *Pst*l sites.

To construct pKR268, the annexin promoter from pJS92 was released by BamHI digestion and the ends were filled. The resulting fragment was ligated into the filled BsiWI fragment of pKR124 (described in Example 2), containing the bacterial ori and ampicillin resistance gene, to give pKR265. This cloning step added *Sbf*I*, Pst*I and *Bsi*WI sites to the 5' end of the annexin promoter. The annexin promoter was released from pKR265 by digestion with *Sbf*I and *Not*I and was cloned into the *Sbf*I/*Not*I fragment of pKR256, containing the BD30 3' transcription terminator, an ampicillin resistance gene and a bacterial ori region, to give pKR268. Vector pKR256 was constructed by cloning an *Eco*RI/*Not*I fragment from pKR251 r, containing the BD30 3' transcription terminator, into the *Eco*RI/*Not*I fragment of intermediate cloning vector pKR227. This step also added a *Pst*I site to the 3' end the BD30 3' transcription terminator. Plasmid pKR227 was derived by ligating the *Sal*I fragment of pJS93 containing soy BD30 promoter (WO 01/68887) with the *Sal*I fragment of pUC19. The BD30 3' transcription terminator was PCR-amplified from soy genomic DNAusing primer oSBD30-1 (SEQ ID NO:91), designed to introduce an *Not*I site at the 5' end of the terminator, and primer oSBD30-2 (SEQ ID NO:92), designed to introduce a *Bsi*WI site at the 3' end of the terminator.
TGCGGCCGCATGAGCCG (SEQ ID NO:91)
ACGTACGGTACCATCTGCTAATATTTTAAATC (SEQ ID NO:92)

The resulting PCR fragment was subcloned into the intermediate cloning vector pCR-Script AMP SK(+) (Stratagene) according the manufacturer's protocol to give plasmid pKR251r.

### EXAMPLE 8

### Assembling EPA biosynthetic pathways genes for expression in Somatic Soybean Embryos-pKR328 & pKR329

The EPA biosynthetic genes were tested in combination in order to assess their combined activities in somatic soybean embryos before large-scale production transformation into soybean. Each gene was cloned into an appropriate expression cassette as described below.

Plasmid pKR329 was similar to pKR275, described in detail in Example 4, in that it contained the same KTi/Fad3/KTi3' and Ann/Sdd17/BD30 cassettes allowing for strong, seed specific expression of the Arabidopsis Fad3 and S. *diclina* delta 17 desaturase genes. It also contained the T7prom/HPT/T7term cassette and a bacterial *ori.* Plasmid pKR329 differed from pKR275 in that it contained the hygromycin phosphotransferase gene cloned behind the 35S promoter followed by the NOS 3' untranslated region (35S/HPT/NOS3' cassette) instead of the SAMS/ALS/ALS3' cassette. The 35S/HPT/NOS3' cassette allowed for selection of transformed plant cells on hygromycin-containing media.

Plasmid pKR329 was constructed in many steps from a number of different intermediate cloning vectors. The KTi/Fad3/KTi3' cassette was released from plasmid pKR201 (Example 7) by digestion with *Bsi*WI and was cloned into the *Bsi*WI site of plasmid pKR325, containing the 35S/HPT/NOS3' cassette, the T7prom/hpt/T7term cassette and bacterial *ori.* This was called plasmid pKR327. The Ann/Sdd17/BD30 cassette, released from pKR271 (Example 3) by digestion with *Pst*I, was then cloned into the *Sbf*I site of pKR327 to give pKR329. Plasmid pKR325 was generated from pKR72 (Example 4) by digestion with *Hind*III to remove the βcon/NotI/Phas3' cassette.

Plasmid pKR328 was identical to pKR329, described above, except that it did not contain the KTi/Fad3/KTi3' cassette. The Ann/Sdd17/BD30 cassette, released from pKR271 (Example 3) by digestion with *Pst*I*,* was cloned into the *Sbf*I site of pKR325 (described above) to give pKR328.

### EXAMPLE 9

### Transformation of Somatic Soybean_Embryo Cultures

### Culture Conditions

Soybean embryogenic suspension cultures (cv. Jack) were maintained in 35 ml liquid medium SB196 (see recipes below) on rotary shaker, 150 rpm, 26°C with cool white fluorescent lights on 16:8 hr day/night photoperiod at light intensity of 60-85 µE/m2/s. Cultures are subcultured every 7 days to two weeks by inoculating approximately 35 mg of tissue into 35 ml of fresh liquid SB196 (the preferred subculture interval is every 7 days).

Soybean embryogenic suspension cultures were transformed with the plasmids and DNA fragments described in the following examples by the method of particle gun bombardment (Klein et al. 1987; Nature, 327:70). A DuPont Biolistic PDS1000/HE instrument (helium retrofit) was used for all transformations.

### Soybean Embryogenic Suspension Culture Initiation

Soybean cultures were initiated twice each month with 5-7 days between each initiation.

Pods with immature seeds from available soybean plants 45-55 days after planting were picked, removed from their shells and placed into a sterilized magenta box. The soybean seeds were sterilized by shaking them for 15 minutes in a 5% Clorox solution with 1 drop of ivory soap (95 ml of autoclaved distilled water plus 5 ml Clorox and 1 drop of soap). Mix well. Seeds were rinsed using 2 1-liter bottles of sterile distilled water and those less than 4 mm were placed on individual microscope slides. The small end of the seed was cut and the cotyledons pressed out of the seed coat. Cotyledons were transferred to plates containing SB1 medium (25-30 cotyledons per plate). Plates were wrapped with fiber tape and stored for 8 weeks. After this time secondary embryos were cut and placed into SB196 liquid media for 7 days.

### Preparation of DNA for Bombardment

Either an intact plasmid or a DNA plasmid fragment containing the genes of interest and the selectable marker gene was used for bombardment. Plasmid DNA for bombardment was routinely prepared and purified using the method described in the Promega™ Protocols and Applications Guide, Second Edition (page 106). Fragments of pKR274 (Example 4), pKKE2 (Example 5) and pKR275 (Example 7) were obtained by gel isolation of double digested plasmids. In each case, 100 ug of plasmid DNA was digested in 0.5 ml of the specific enzyme mix described below. Plasmid pKR274 (Example 4) and pKKE2 (Example 5) were digested with *Asc*I (100 units) and *Eco*RI (100 units) in NEBuffer 4 (20 mM Tris-acetate, 10 mM magnesium acetate, 50 mM potassium acetate, 1 mM dithiothreitol, pH 7.9), 100 ug/ml BSA, and 5 mM beta-mercaptoethanol at 37°C for 1.5 hr. Plasmid pKR275 (Example 7) was digested with *Asc*I (100 units) and *Sgf*I (50 units) in NEBuffer2 (10 mM Tris-HCl, 10 mM MgCl₂, 50 mM NaCl, 1 mM dithiothreitol, pH 7.9), 100 ug/ml BSA, and 5 mM beta-mercaptoethanol at 37°C for 1.5 hr. The resulting DNA fragments were separated by gel electrophoresis on 1% SeaPlaque GTG agarose (BioWhitaker Molecular Applications) and the DNA fragments containing EPA biosynthetic genes were cut from the agarose gel. DNA was purified from the agarose using the GELase digesting enzyme following the manufacturer's protocol.

A 50 µl aliquot of sterile distilled water containing 3 mg of gold particles (3 mg gold) was added to 5 µl of a 1 µg/µl DNA solution (either intact plasmid or DNA fragment prepared as described above), 50 µl 2.5M CaCl₂ and 20 µl of 0.1 M spermidine. The mixture was shaken 3 min on level 3 of a vortex shaker and spun for 10 sec in a bench microfuge. After a wash with 400 µl 100% ethanol the pellet was suspended by sonication in 40 µl of 100% ethanol. Five µl of DNA suspension was dispensed to each flying disk of the Biolistic PDS1000/HE instrument disk. Each 5 µl aliquot contained approximately 0.375 mg gold per bombardment (i.e. per disk).

### Tissue Preparation and Bombardment with DNA.

Approximately 150-200 mg of 7 day old embryonic suspension cultures were placed in an empty, sterile 60 x 15 mm petri dish and the dish covered with plastic mesh. Tissue was bombarded 1 or 2 shots per plate with membrane rupture pressure set at 1100 PSI and the chamber evacuated to a vacuum of 27-28 inches of mercury. Tissue was placed approximately 3.5 inches from the retaining / stopping screen.

### Selection of Transformed Embryos

Transformed embryos were selected either using hygromycin (when the hygromycin phosphotransferase, HPT, gene was used as the selectable marker) or chlorsulfuron (when the acetolactate synthase, ALS, gene was used as the selectable marker).

### Hygromycin (HPT) Selection

Following bombardment, the tissue was placed into fresh SB196 media and cultured as described above. Six days post-bombardment, the SB196 is exchanged with fresh SB196 containing a selection agent of 30 mg/L hygromycin. The selection media is refreshed weekly. Four to six weeks post selection, green, transformed tissue may be observed growing from untransformed, necrotic embryogenic clusters. Isolated, green tissue was removed and inoculated into multiwell plates to generate new, clonally propagated, transformed embryogenic suspension cultures.

### Chlorsulfuron (ALS) Selection

Following bombardment, the tissue was divided between 2 flasks with fresh SB196 media and cultured as described above. Six to seven days post-bombardment, the SB196 was exchanged with fresh SB196 containing selection agent of 100 ng/ml Chlorsulfuron. The selection media was refreshed weekly. Four to six weeks post selection, green, transformed tissue may be observed growing from untransformed, necrotic embryogenic clusters. Isolated, green tissue was removed and inoculated into multiwell plates containing SB196 to generate new, clonally propagated, transformed embryogenic suspension cultures.

### Regeneration of Soybean Somatic Embryos into Plants

In order to obtain whole plants from embryogenic suspension cultures, the tissue must be regenerated.

### Embryo Maturation

Embryos were cultured for 4-6 weeks at 26°C in SB195 under cool white fluorescent (Phillips cool white Econowatt F40/CW/RS/EW) and Agro (Phillips F40 Agro) bulbs (40 watt) on a 16:8 hr photoperiod with light intensity of 90-120 uE/m2s. After this time embryo clusters were removed to a solid agar media, SB166, for 1-2 weeks. Clusters were then subcultured to medium SB103 for 3 weeks. During this period, individual embryos can be removed from the clusters and screened for alterations in their fatty acid compositions as described in Example 11. It should be noted that any detectable phenotype, resulting from the expression of the genes of interest, could be screened at this stage. This would include, but not be limited to, alterations in fatty acid profile, protein profile and content, carbohydrate content, growth rate, viability, or the ability to develop normally into a soybean plant.

### Embryo Desiccation and Germination

Matured individual embryos were desiccated by placing them into an empty, small petri dish (35 x 10 mm) for approximately 4-7 days. The plates were sealed with fiber tape (creating a small humidity chamber). Desiccated embryos were planted into SB71-4 medium where they were left to germinate under the same culture conditions described above. Germinated plantlets were removed from germination medium and rinsed thoroughly with water and then planted in Redi-Earth in 24-cell pack tray, covered with clear plastic dome. After 2 weeks the dome was removed and plants hardened off for a further week. If plantlets looked hardy they were transplanted to 10" pot of Redi-Earth with up to 3 plantlets per pot. After 10 to 16 weeks, mature seeds were harvested, chipped and analyzed for fatty acids as described in Examples 10 and 11.

### Media Recipes

### SB 196 - FN Lite liquid proliferation medium (per liter) -

| | |
|---|---|
| MS FeEDTA - 100x Stock 1 | 10 ml |
| MS Sulfate - 100x Stock 2 | 10 ml |
| FN Lite Halides - 1 00x Stock 3 | 10 ml |
| FN Lite P,B,Mo - 100x Stock 4 | 10 ml |
| B5 vitamins (1 ml/L) | 1.0 ml |
| 2,4-D (10mg/L final concentration) | 1.0 ml |
| KNO3 | 2.83 gm |
| (NH4 )2 SO 4 | 0.463 gm |
| Asparagine | 1.0 gm |
| Sucrose (1%) | 10 gm |
| pH 5.8 | |

### FN Lite Stock Solutions

| **Stock #** | | | 1000ml | 500ml | |
|---|---|---|---|---|---|
| **1** | **MS Fe EDTA 100x Stock** | | | | |
| | | Na₂ EDTA* | 3.724 g | | 1.862 g |
| | | FeSO₄ - 7H₂O | 2.784 g | | 1.392 g |
| | * Add first, dissolve in dark bottle while stirring | | | | |

| **2** | **MS Sulfate 100x stock** | | | | |
|---|---|---|---|---|---|
| | | MgSO₄ - 7H₂O | 37.0 g | 18.5 g | |
| | | MnSO₄ - H₂O | 1.69 g | 0.845 g | |
| | | ZnSO₄ 7H₂O | 0.86 g | 0.43 g | |
| | | CuSO₄ - 5H₂O | 0.0025 g | 0.00125 g | |

| **3** | **FN Lite Halides 100x Stock** | | | | |
|---|---|---|---|---|---|
| - | | CaCl₂ - 2H₂O | 30.0 g | 15.0 g | |
| | | KI | 0.083 g | 0.0715 g | |
| | | CoCl₂ - 6H₂O | 0.0025 g | 0.00125 g | |

| **4** | **FN Life P,B,Mo 100x Stock** | | | | |
|---|---|---|---|---|---|
| | | KH₂PO₄ | 18.5 g | 9.25 g | |
| | | H₃BO₃ | 0.62 g | 0.31 g | |
| | | Na₂MoO₄ - 2H₂O | 0.025 g | 0.0125 g | |

### SB1 solid medium (per liter)-

1 pkg. MS salts (Gibco/ BRL - Cat# 11117-066)
1 ml B5 vitamins 1000X stock
31.5 g sucrose
2 ml 2,4-D (20mg/L final concentration)
pH 5.7
8 g TC agar

### SB 166 solid medium (per liter) -

1 pkg. MS salts (Gibco/ BRL - Cat# 11117-066)
1 ml B5 vitamins 1000X stock
60 g maltose
750 mg MgCl2 hexahydrate
5 g activated charcoal
pH 5.7
2 g gelrite

### SB 103 solid medium (per liter) -

1 pkg. MS salts (Gibco/BRL - Cat# 11117-066)
1 ml B5 vitamins 1000X stock
60 g maltose
750 mg MgCl2 hexahydrate
pH 5.7
2 g gelrite

### SB 71-4 solid medium (per liter)-

1 bottle Gamborg's B5 salts w/ sucrose (Gibco/BRL - Cat# 21153-036)
pH5.7
5 g TC agar

### 2,4-D stock

- obtained premade from Phytotech cat# D 295 - concentration is 1 mg/ml

### B5 Vitamins Stock (per 100 ml) - store aliquots at -20C

10g myo-inositol
100 mg nicotinic acid
100 mg pyridoxine HCl
1 g thiamine
If the solution does not dissolve quickly enough, apply a low level of heat via the hot stir plate.

### Chlorsulfuron Stock

-1mg / ml in 0.01 N Ammonium Hydroxide

### EXAMPLE 10

### Analysis of Somatic Soy Embryos containing various promoters driving M. alpina delta-6 desaturase

Mature somatic soybean embryos are a good model for zygotic embryos. While in the globular embryo state in liquid culture, somatic soybean embryos contain very low amounts of triacylglycerol or storage proteins typical of maturing, zygotic soybean embryos. At this developmental stage, the ratio of total triacylglyceride to total polar lipid (phospholipids and glycolipid) is about 1:4, as is typical of zygotic soybean embryos at the developmental stage from which the somatic embryo culture was initiated. At the globular stage as well, the mRNAs for the prominent seed proteins, α'-subunit of β-conglycinin, kunitz trypsin inhibitor 3, and seed lectin are essentially absent. Upon transfer to hormone-free media to allow differentiation to the maturing somatic embryo state, triacylglycerol becomes the most abundant lipid class. As well, mRNAs for α'-subunit of β-conglycinin, kunitz trypsin inhibitor 3 and seed lectin become very abundant messages in the total mRNA population. On this basis somatic soybean embryo system behaves very similarly to maturing zygotic soybean embryos *in vivo,* and is therefore a good and rapid model system for analyzing the phenotypic effects of modifying the expression of genes in the fatty acid biosynthesis pathway. Most importantly, the model system is also predictive of the fatty acid composition of seeds from plants derived from transgenic embryos.

Transgenic somatic soybean embryos containing the *M. alpina* delta-6 desaturase expression vectors described in Example 2 were prepared using the methods described In Example 9. Fatty acid methyl esters were prepared from single, matured, somatic soy embryos by transesterification. Embryos were placed in a vial containing 50 µL of trimethylsulfonium hydroxide (TMSH) and 0.5 mL of hexane and were incubated for 30 minutes at room temperature while shaking. Fatty acid methyl esters (5 µL injected from hexane layer) were separated and quantified using a Hewlett-Packard 6890 Gas Chromatograph fitted with an Omegawax 320 fused silica capillary column (Supelco Inc., Cat#24152). The oven temperature was programmed to hold at 220°C for 2.7 min, increase to 240°C at 20°C /min and then hold for an additional 2.3 min. Carrier gas was supplied by a Whatman hydrogen generator. Retention times were compared to those for methyl esters of standards commercially available (Nu-Chek Prep, Inc. catalog #U-99-A). The amount of GLA accumulated in embryo tissue was used as an indicator of the strength of each individual promoter. As indicated in Table 6, all of the promoters tested were capable of driving expression of the *M*. *alpina* delta-6 desaturase.

**TABLE 6**

| GLA Accumulation in Soybean Somatic Embryos: *M. alpina* delta-6 desaturase gene linked to various promoters | |
|---|---|
| Promoter | GLA (% fatty acid) |
| Soy α'-subunit β-conglycinin | 40+ |
| Soy KTi 3 | 40+ |
| Soy Annexin | 40 |
| Soy Glycinin 1 | 35 |
| Soy 2S albumin | 22 |
| Pea Legumin A1 | 10 |
| Soy β'-subunit β-conglycinin | 9 |
| Soy BD30 | 8 |
| Pea Legumin A2 | 3 |

### EXAMPLE 11

### Analysis of transgenic Somatic Soy Embryos and Seed Chips containing EPA Biosynthetic Genes

Transgenic somatic soybean embryos containing the expression vector pKR275 (Example 7) and either pKR274 (Example 4) or pKKE2 (Example 5) were prepared using the methods described in Example 9.

A portion of the somatic soy embryos from each line generated was harvested and analyzed for fatty acid composition by GC as described in Example 10. Approximately 10 embryos were analyzed for each individual transformation event. Fatty acids were identified by comparison of retention times to those for authentic standards. In this way, 471 events were analyzed for pKR274/pKR275 and 215 events were analyzed for pKKE/pKR275. From the 471 lines analyzed for pKR274/pKR275, 10 were identified that produced EPA (average of 10 individual embryos) at a relative abundance greater than 7% of the total fatty acids. The best line analyzed averaged 9% EPA with the best embryo of this line having 13% EPA. From the 215 lines analyzed for KKE/KR275, 11 lines were identified that produced EPA (average of 10 individual embryos) at a relative abundance greater than 9% of the total fatty acids. The best line analyzed averaged 13% EPA with the best embryo of this line having 16% EPA. The best EPA-producing events from each construct set are shown in Table 7. In Table 7, clones 3306-2-3 to 3324-1-3 are pKR274/pKR275 events and 3338-6-3 to 3338-6-24 are pKKE2 events. Fatty acids in Table 7 ar defined as X:Y where X is the fatty acid chain length and Y is the number of double bonds. In addition, fatty acids from Table 7 are further defined as follows where the number in parentheses corresponds to the position of the double bonds from the carboxyl end of the fatty acid: 18:1=18:1(9), 18:2=18:2(9,12), GLA=18:3(6,9,12), 18:3=18:3(9,12,15), STA=18:4(6,9,12,15), HGLA=20:3(8,11,14), ARA=20:4(5,8,11,14), ETA=20:4(8,11,14,17), EPA=20:5(5,8,11,14,17) and DPA=22:5(7,10,13,16, 19). Fatty acids listed as "others" include: 20:0, 20:1(5), 20:2(11,14), 20:3 (5,11,14), 20:3 (11,14,17), 20:4 (5,11,14,17), and 22:0. For KKE2 events each of these fatty acids is present at relative abundance of less than 1% of the total fatty acids. For KR274/275 each of these fatty acids is present at relative abundance of less than 1% of total fatty acids except for events 3306-5-2, 3319-6-1, 3319-2-13 in which 20:3 (11,14,17) and 20:4 (5,11,14,17) are both in the range of 1.1 to 2.2% of total fatty acids.

**TABLE 7**

| Fatty acid analyses of transgenic soybean somatic embryos producing C20 PUFAs | | | | | | | | | | | | | |
|---|---|---|---|---|---|---|---|---|---|---|---|---|---|
| ***Clone ID*** | ***16:0*** | ***18:0*** | ***18:1*** | ***18:2*** | ***GLA*** | **18:3** | ***STA*** | ***HGLA*** | ***ARA*** | ***ETA*** | ***EPA*** | ***DPA*** | ***Others*** |
| 3306-2-3 | 14.9 | 2.3 | 6.3 | 15.8 | 21.7 | 11.5 | 4.5 | 4.8 | 0.8 | 2.7 | 8.4 | 1.2 | 2 |
| 3306-5-2 | 14.2 | 4.4 | 11.7 | 19.4 | 4.6 | 20.8 | 1.5 | 1.5 | 0.2 | 1.5 | 7.7 | 4.2 | 5.3 |
| 3319-3-1 | 18.2 | 2.9 | 11.0 | 19.1 | 15.6 | 14.5 | 3.4 | 1.8 | 1.3 | 0.6 | 8.4 | 0.6 | 1.2 |
| 3319-6-1 | 11.1 | 3.7 | 16.6 | 12.9 | 10.7 | 12.1 | 3.3 | 5.0 | 0.8 | 2.8 | 9.3 | 2.0 | 4 |
| 3319-2-13 | 12.7 | 3.3 | 17.5 | 14.2 | 10.8 | 15.9 | 3.1 | 2.4 | 0.1 | 2.8 | 8.0 | 1.1 | 3.3 |
| 3319-2-16 | 12.7 | 2.5 | 8.5 | 18.1 | 10.3 | 12.1 | 2.3 | 3.4 | 4.0 | 1.0 | 7.3 | 2.5 | 2.3 |
| 3319-3-6 | 11.7 | 2.0 | 10.1 | 13.2 | 11.5 | 7.7 | 1.9 | 2.8 | 0.7 | 1.8 | 9.3 | 1.8 | 3.3 |
| 3320-6-1 | 15.3 | 3.7 | 13.5 | 10.7 | 14.8 | 12.4 | 4.5 | 6.6 | 1.4 | 2.4 | 8.0 | 1.2 | 2.4 |
| 3322-5-2 | 13.9 | 2.9 | 14.4 | 15.8 | 17.4 | 13.8 | 3.5 | 2.9 | 0.2 | 1.8 | 8.1 | 0.9 | 2.2 |
| 3324-1-3 | 12.0 | 4.4 | 18.6 | 17.6 | 13.9 | 7.8 | 1.8 | 4.8 | 0.3 | 3.4 | 8.1 | 0.8 | 2.9 |
| | | | | | | | | | | | | | |
| 3338-6-3 | 14.3 | 3.2 | 18.1 | 11.0 | 13.7 | 8.8 | 3.0 | 5.1 | 0.2 | 5.3 | 9.6 | 1.2 | 2.1 |
| 3338-7-11 | 20.5 | 2.9 | 9.9 | 10.6 | 8.9 | 17.3 | 3.8 | 2.0 | 0.4 | 3.0 | 12.8 | 1.8 | 1.9 |
| 3338-7-12 | 16.5 | 2.1 | 15.2 | 15.4 | 16.1 | 11.5 | 2.5 | 1.7 | 0.2 | 2.0 | 10.0 | 0.8 | 1.2 |
| 3338-3-4 | 20.2 | 3.9 | 6.7 | 11.9 | 9.9 | 10.5 | 3.9 | 4.6 | 1.8 | 3.1 | 12.0 | 3.2 | 2.1 |
| 3338-3-5 | 14.7 | 2.2 | 12.4 | 12.4 | 17.6 | 10.8 | 4.7 | 2.9 | 1.3 | 1.4 | 10.0 | 0.9 | 1.8 |
| 3338.6.10 | 13.7 | 1.8 | 12.4 | 8.3 | 16.4 | 14.0 | 5.8 | 3.2 | 0.3 | 4.0 | 12.1 | 1.2 | 2.2 |
| 3338-6-12 | 13.9 | 2.4 | 13.1 | 9.4 | 22.7 | 5.7 | 3.1 | 4.0 | 0.4 | 3.3 | 13.3 | 0.9 | 1.5 |
| 3338-7-21 | 14.8 | 1.7 | 8.4 | 13.1 | 20.2 | 12.5 | 4.8 | 3.9 | 0.4 | 3.6 | 11.6 | 0.6 | 2 |
| 3338-7-30 | 15.4 | 2.8 | 18.9 | 12.9 | 9.6 | 10.1 | 2.4 | 2.3 | 0.5 | 2.3 | 13.0 | 2.6 | 2.4 |
| 3338-1-4 | 14.1 | 2.1 | 10.8 | 26.3 | 13.8 | 9.6 | 1.9 | 3.3 | 1.1 | 1.9 | 10.1 | 1.0 | 1.3 |
| 3338-6-24 | 25.1 | 4.5 | 13.3 | 4.0 | 15.5 | 3.1 | 2.6 | 5.3 | 0.7 | 4.0 | 13.0 | 0.9 | 1.7 |

Mature plants were regenerated from the highest EPA-producing embryos as described in Example 10, and the fatty acid analyses were performed on chips of the seeds from the regenerated plants. The results for six seeds from three plants are presented in Table 8. Seeds of control plants possessed fatty aid profiles typical of normal soybean, in which linolenic acid (18:3) was the most highly unsaturated fatty acid that was detectable. Seeds produced from plants that had a reconstituted pathway for C20 PUFAs had as much as 25% of their total fatty acid in the form of C20 material. Combined levels of EPA and DPA were frequently greater than 15%, and were as high as 23.5% of the total.

**TABLE 8**

| **Event** | **16:0** | **18:0** | **18:1** | **18:2** | **GLA** | **18:3** | **STA** | **HGLA** | **ARA** | **ETA** | **EPA** | **DPA** | **Other** | **EPA+ DPA** |
|---|---|---|---|---|---|---|---|---|---|---|---|---|---|---|
| 3338-3-4-7 | 14.4 | 8.5 | 19.7 | 9.1 | 9.1 | 3.1 | 1.2 | 6.6 | 1.0 | 2.4 | 18.8 | 4.1 | 2.0 | 22.9 |
| | 13.2 | 5.5 | 18.6 | 10.4 | 11.7 | 3.3 | 1.1 | 10.1 | 2.2 | 2.4 | 19.6 | 0.8 | 1.2 | 20.4 |
| | 15.6 | 9.0 | 13.9 | 16.6 | 6.6 | 7.1 | 0.0 | 3.9 | 0.0 | 1.8 | 15.5 | 4.2 | 5.8 | 19.7 |
| | 22.4 | 8.8 | 20.8 | 14.2 | 5.0 | 3.8 | 0.6 | 3.0 | 1.0 | 1.1 | 14.0 | 3.1 | 2.2 | 17.1 |
| | 13.2 | 7.5 | 27.0 | 12.8 | 9.0 | 2.8 | 0.9 | 5.7 | 1.8 | 1.2 | 11.2 | 4.0 | 2.9 | 15.2 |
| | 15.2 | 4.9 | 18.3 | 12.3 | 13.3 | 3.5 | 1.3 | 10.5 | 5.3 | 2.4 | 12.9 | 0.0 | 0.0 | 12.9 |
| | | | | | | | | | | | | | | |
| 3338-7-11-11 | 13.0 | 7.1 | 13.6 | 13.1 | 13.0 | 5.9 | 1.7 | 5.2 | 0.5 | 0.4 | 16.4 | 4.3 | 5.8 | 20.7 |
| | 12.9 | 7.3 | 13.1 | 14.9 | 9.6 | 7.2 | 1.7 | 5.9 | 0.8 | 0.6 | 14.3 | 4.7 | 7.0 | 18.9 |
| | 12.4 | 7.6 | 15.9 | 12.6 | 13.6 | 5.4 | 1.8 | 6.0 | 0.5 | 0.0 | 15.2 | 3.7 | 5.2 | 18.9 |
| | 15.0 | 5.9 | 18.4 | 16.0 | 10.2 | 8.4 | 1.7 | 4.0 | 0.6 | 0.0 | 13.9 | 2.4 | 3.5 | 16.3 |
| | 13.8 | 5.9 | 19.6 | 18.0 | 7.2 | 10.8 | 1.5 | 3.4 | 0.4 | 0.0 | 10.8 | 3.2 | 5.5 | 14.0 |
| | 16.2 | 6.2 | 15.2 | 22.4 | 6.9 | 9.2 | 1.1 | 3.4 | 0.8 | 0.0 | 11.7 | 2.2 | 4.6 | 13.9 |
| 3339-5-3-7 | 13.7 | 8.1 | 6.9 | 8.1 | 16.5 | 4.7 | 1.8 | 7.1 | 0.7 | 2.2 | 19.5 | 4.0 | 6.7 | 23.5 |
| | 15.4 | 6.9 | 11.8 | 16.4 | 10.0 | 4.3 | 0.8 | 4.7 | 1.2 | 1.4 | 16.3 | 3.5 | 7.3 | 19.8 |
| | 14.7 | 6.3 | 13.6 | 18.1 | 8.1 | 3.1 | 0.9 | 4.3 | 2.1 | 0.1 | 14.9 | 4.2 | 9.6 | 19.1 |
| | 12.3 | 6.5 | 20.9 | 13.1 | 15.1 | 3.0 | 1.0 | 6.1 | 1.2 | 1.4 | 10.6 | 1.4 | 7.3 | 12.1 |
| | 12.2 | 6.4 | 22.9 | 13.7 | 12.0 | 2.9 | 0.9 | 5.7 | 1.3 | 1.3 | 9.9 | 1.7 | 9.1 | 11.7 |
| | 13.5 | 7.2 | 22.9 | 11.8 | 8.9 | 3.6 | 0.8 | 6.5 | 2.2 | 1.7 | 9.6 | 1.6 | 9.8 | 11.2 |
| | | | | | | | | | | | | | | |
| Control | 17.3 | 4.3 | 13.4 | 51.6 | 0.0 | 12.9 | 0.0 | 0.0 | 0.0 | 0.0 | 0.0 | 0.0 | 0.0 | 0.0 |
| | 17.1 | 4.8 | 12.1 | 50.5 | 0.0 | 14.5 | 0.0 | 0.0 | 0.0 | 0.0 | 0.0 | 0.0 | 0.0 | 0.0 |
| Others= sum of 20:0, 20:1 (d5), 20:1 (d11), 20:2 (d8,11), 20:2 (d11,14), 20:3 (d5,11,14), 20:3 (d11,14,17), 20:4 (d5,11,14,17) each of which is present at less than 2% of TFA | | | | | | | | | | | | | | |

### EXAMPLE 12

### Isolation of a Novel Elongase Gene from the Algae Pavlova sp. (CCMP459)

The fatty acid composition of the algae *Pavlova sp.* (CCMP 459) (Pav459) was investigated to determine the polyunsaturated fatty acids (PUFAs) produced by this organism. This algae showed a substantial amount of long chain PUFAs including eicosapentaenoic acid (EPA, 20:5n-3) and docosahexaenoic acid (DHA, 22:6n-3). Thus, Pav459 was predicted to possess an elongase capable of converting EPA to ω3-docosapentaenoic acid (DPA, 22:5n-3), which a delta-4 desaturase can convert to DHA. The goal was therefore to isolate the predicted elongase gene from Pav459, and to verify the functionality of the enzyme by expression in an alternate host.

Frozen pellets of Pav459 were obtained from Provasoli-Guillard National Center for Culture of Marine Phytoplankton (CCMP, West Boothbay Harbor, ME). These pellets were crushed in liquid nitrogen and total RNA was extracted from Pav459 by using the Qiagen RNeasy Maxi Kit (Qiagen, Valencia, CA), per manufacturers instructions. From this total RNA, mRNA was isolated using oligo dT cellulose resin, which was then used for the construction of a cDNA library using the pSport 1 vector (Invitrogen, Carlsbad, CA). The cDNA thus produced was directionally cloned (5'*SaIII*/3'*NotI*) into pSport1 vector. The Pav459 library contained approximately 6.1 x 10⁵ clones per ml, each with an average insert size of approximately 1200 bp. Two thousand five hundred primary clones from this library were sequenced from the 5' end using the T7 promoter primer (SEQ ID NO:93).
TAATACGACTCACTATTAGG SEQ ID NO:93

Sequencing was carried out using the ABI BigDye sequencing kit (Applied Biosystems, CA) and the MegaBase Capillary DNA sequencer (Amersham biosciences, Piscataway, NJ). Two clones, designated 'pav06-C06' and pav07-G01,' which aligned to give a 500 bp sequence containing the 5' end of this novel elongase, were obtained from sequencing of the 2,500 library clones. This fragment shared 33.3% amino acid sequence identity with the mouse elongase MELO4 and 32.7% amino acid sequence identity with *T. aureum* elongase TELO1 (WO 02/08401). To isolate the full-length gene, the EST clone pav06-C06 was used as a template for PCR reaction with 10 pmol of the 5' primer RO1327 (SEQ ID NO:94) and 10 pmol vector primer RO898 (SEQ ID NO:83).
TGCCCATGATGTTGGCCGCAGGCTATCTTCTAGTG SEQ ID NO:94

PCR amplification was carried out using Platinum Taq DNA polymerase (Invitrogen, Carlsbad, CA) in a 50 µl total volume containing: 1 µl of the cDNA clone pav06-C06, PCR buffer containing 20 mM Tris-Cl, pH 8.4, 50 mM KCI (final concentration), 200 µM each deoxyribonucleotide triphosphate, 10 pmole of each primer, 1.5 mM MgSO₄, and 0.5 µl of Platinum Taq (HF) DNA polymerase. Amplification was carried out as follows using the Perkin Elmer 9700 machine: initial denaturation at 94°C for 3 minute, followed by 35 cycles of the following: 94°C for 45 sec, 55°C for 30 sec, 68°C for 2 min. The reaction was terminated at 4°C. The PCR amplified mixture was run on a gel, an amplified fragment of approximately 1.3 Kb was gel purified, and the isolated fragment was cloned into the pCR-blunt vector (Invitrogen, Carlsbad, CA). The recombinant plasmid was transformed into TOP10 supercompetent cells (Invitrogen, Carlsbad, CA), and prepared. The prepared recombinant plasmid was digested with EcoRI, run on a gel, and the digested fragment of approximately 1.2 Kb was gel purified, and cloned into pYX242 (EcoRI) vector (Novagen, Madison, WI). The new plasmid was designated as pRPL-6-1.

The plasmid pRPL-6-1 was prepared and sequenced using ABI 373A Stretch DNA Sequencer (Perkin Elmer, Foster City, CA). The translated amino acid sequence of the cDNA in pRPL-6-1 had 33.7% identity in 261 amino acids with MELO4, 33.8% identity in 240 amino acids with GLELO, 28.1 % identity in 274 amino acids with HSELO1, and 32.5% identity in 246 amino acids with TELO1 (WO 02/08401).

The construct pRPL-6-1 was transformed into S. *cerevisiae* 334 (Hoveland et al. (1989) Gene 83:57-64) and screened for elongase activity. *S. cerevisiae* 334 containing the unaltered pYX242 vector was used as a negative control. The cultures were grown for 44 hours at 24°C, in selective media (Ausubel et al., (1992) Short Protocols in Molecular Biology, Ch. 13, p. 3-5), in the presence of 25 µM of GLA or EPA. In this study, DGLA or ω3-docosapentaenoic acid (DPA, 22:5n-3), respectively, was the predicted product of the elongase activity. The lipid profiles of these yeast cultures indicated that while no conversion of GLA to DGLA was seen, EPA was elongated to DPA at a very low level (DPA was 0.34% of total fatty acids, while EPA was 32.28% of total fatty acids). This indicated that the expressed enzyme in this culture preferred the elongation of 20 carbon chain long PUFA, and not the 18 carbon chain long PUFA, GLA. It also indicated that a mutation might be present in the DNA sequence, which is inhibiting the full activity of the expressed enzyme.

To isolate the full-length gene without mutations, RACE (rapid amplification of cDNA ends) ready cDNA was used as a target for the reaction. To prepare this material, approximately 5 µg of total RNA was used according to the manufacturer's direction with the GeneRacer™ kit (Invitrogen, Carlsbad, CA) and Superscript II™ enzyme (Invitrogen, Carlsbad, CA) for reverse transcription to produce cDNA target. This cDNA was then used as a template for a PCR reaction with 50 pmols of the 5' primer RO1327 and 30 pmol GeneRacer™ 3' primer (SEQ. ID NO:95).
GCTGTCAACGATACGCTACGTAACG SEQ ID NO:95

PCR amplification was carried out using Platinum Taq DNA polymerase (Invitrogen, Carlsbad, CA) in a 50 µl total volume containing: 2 µl of the RACE ready cDNA, PCR buffer containing 20 mM Tris-Cl, pH 8.4, 50 mM KCl (final concentration), 200 µM each deoxyribonucleotide triphosphate, 10 pmole of each primer, 1.5 mM MgSO₄, and 0.5 µl of Platinum Taq (HF) DNA polymerase. Amplification was carried out as follows using the Perkin Elmer 9600 machine: initial denaturation at 94°C for 3 minute, followed by 35 cycles of the following: 94°C for 45 sec, 55°C for 30 sec, 68°C for 2 min. The reaction was terminated at 4°C.

The PCR amplified mixture was run on a gel, an amplified fragment of approximately 1.2 Kb was gel purified, and the isolated fragment was cloned into the PCR-blunt vector (Invitrogen, Carlsbad, CA). The recombinant plasmids were transformed into TOP10 supercompetent cells (Invitrogen, Carlsbad, CA), and prepared. The prepared recombinant plasmid was digested with EcoRI, run on a gel, and the digested fragment of approximately 1.2 Kb was gel purified, and cloned into pYX242 (EcoRI) vector (Novagen, Madison, WI). The new plasmids were designated as pRPL-6-B2 and pRPL-6-A3.

The plasmids pRPL-6-B2 and pRPL-6-A3 were prepared and sequenced using ABI 373A Stretch DNA Sequencer (Perkin Elmer, Foster City, CA). The translated amino acid sequence of the cDNA in pRPL-6-B2 had 34.1 % identity in 261 amino acids with MELO4, 33.8% identity in 240 amino acids with GLELO, 28.5% identity in 274 amino acids with HSELO1, and 32.5% identity in 246 amino acids with TELO1. (Plasmid pRPL-6-B2 was deposited with the American Type Culture Collection, 10801 Manassas, VA 20110-2209 under the terms of the Budapest Treaty and was accorded accession number PTA-4350.)

The constructs pRPL-6-B2 and pRPL-6-A3 were transformed into *S. cerevisiae* 334 (Hoveland et al., *supra)* and screened for elongase activity. *S. cerevisiae* 334 containing the unaltered pYX242 vector was used as a negative control. The cultures were grown for 44 hours at 24°C, in selective media (Ausubel et al., supra), in the presence of 25 µM of GLA or EPA. In this study, DGLA or ω3-docosapentaenoic acid (DPA, 22:5n-3), respectively, was the predicted product of the elongase activity. The lipid profiles of these yeast cultures indicated that GLA was not elongated to DGLA in any of the samples (data not shown). The cultures of 334(pRPL-6-B2) and 334(pRAT-6-A3) had significant levels of conversion of the substrate EPA to DPA, indicating that the expressed enzymes in these cultures preferred the elongation of 20-carbon chain long PUFA, and not the 1 8-chain long PUFA, GLA.

The amino acid sequences of the 3 clones were compared to determine if the substrate conversion levels were dictated by the translated sequences. The cDNA sequence of pRPL-6-1 is different from pRPL-6-B2 at A512G. This single mutation substantially reduced the conversion of the C20 substrate fatty acid to its elongated product. It appears that this is an important region of the enzyme for 20-carbon chain elongation. The cDNA sequence of pRPL-6-A3 is different from pRPL-6-B2 at D169N and C745R. These mutations reduced the conversion of the C20 substrate fatty acid to its elongated product, but the expressed enzyme was able to maintain some activity. The elongase gene in pRPL-6-B2, has the sequence set forth in SEQ ID NO:49 and the amino acid sequence set forth in SEQ ID NO:50.

To further confirm the substrate specificity of the algal elongation enzyme, described above and referred to herein as PELO1p, the recombinant yeast strain 334(pRPL-6-B2) was grown in minimal media containing n-6 fatty acids LA, GLA, DGLA, AA, or n-3 fatty acids ALA, STA, ETA, EPA, or 20:0, or 20:1. The lipid profiles of these yeast cultures, when examined by GC and GC-MS, indicated that there were accumulations of adrenic acid (ADA, 22:4-6) and EPA, respectively. The levels of these fatty acids were 1.40% ADA and 2.54% EPA, respectively, of the total fatty acids in the strains containing the PELO1 sequence. These represented 14.0% and 14.1 % conversions of the substrate fatty acids, respectively, to the products elongated by two carbon atoms. No elongation of the saturated fatty acid 20:0, or monounsaturated fatty acid 20:1 was seen. Also, no elongation of the C18 substrates LA, GLA, ALA, or STA was seen. These results indicated that the expressed enzyme activity in strain 334(pRPL-6-B2) was specific for the elongation of 20-carbon chain long PUFAs, and not the 18-chain long PUFA, or the 20-carbon chain long saturated or monounsaturated fatty acids.

### EXAMPLE 13

### Assembling DHA Biosynthetic Pathway Genes for Expression in Somatic Soybean Embryos (pKR365; pKR364, and pKR357)

### Construction of plasmid pKR365

The *S. diclina* delta-6 desaturase, *M. alpina* delta-5 desaturase and *S. diclina* delta-17 desaturase were cloned into plasmid pKR365 behind strong, seed-specific promoters allowing for high expression of these genes in somatic soybean embryos and soybean seeds. The delta6 desaturase was cloned behind the KTi promoter followed by the KTi 3' termination region (Kti/Sdd6/Kti3' cassette). The delta-5 desaturase was cloned behind the GlycininGy1 promoter followed by the pea leguminA2 3' termination region (Gy1/Mad5/legA2 cassette). The *S. diclina* delta-17 desaturase was cloned behind the soybean Annexin promoter followed by the soy BD30 3' termination region (Ann/Sdd17/BD30 cassette). Plasmid pKR365 also contains the T7prom/HPT/T7term cassette for bacterial selection of the plasmid on hygromycin B and a bacterial origin of replication *(ori)* from the vector pSP72 (Stratagene).

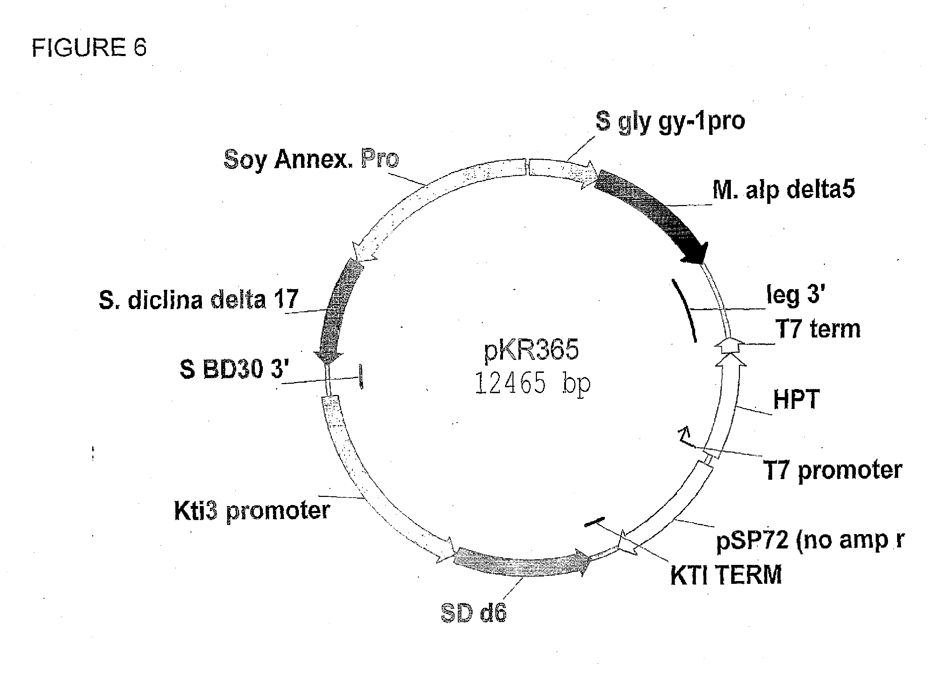

Plasmid pKR365 was constructed from a number of different intermediate cloning vectors as follows: The Gy1/Mad5/legA2 cassette was released from plasmid pKR287 by digestion with SbfI and BsiWI. This cassette was cloned into the *SbFI*/*BsiWI* site of plasmid pKR359, containing the Kti/Sdd6/Kti3' cassette, the T7prom/hpt/T7term cassette and the bacterial ori to give pKR362. The Ann/Sdd17/BD30 cassette, released from pKR271 (described in Example 7) by digestion with *Pst*I*,* was then cloned into the SbfI site of pKR362 to give pKR365.. A schematic representation of pKR365 is shown in Figure 6. A detailed description for plasmid construction for pKR287 and pKR359 is provided below.

Plasmid pKR287 was constructed by digesting pKR136 (described in Example 4) with *Not*I, to release the *M. alpina* delta-5 desaturase, and cloning this fragment into the *Not*I site of pKR263 (described in Example 4).

Plasmid pKR359 was constructed by cloning the *Not*I fragment of pKR295, containing the delta-6 desaturase, into the *Not*I site of the Kti/*Not*I/Kti3' cassette in pKR353. Vector pKR353 was constructed by cloning the *Hind*III fragment, containing the Kti/*Not*I/Kti3' cassette, from pKR124 (described in Example 2) into the *Hind*III site of pKR277. Plasmid pKR277 was constructed by digesting pKR197 (described in Example 4) with *Hind*III to remove the Bcon/*Not*I/phas3' cassette. To construct pKR295, the gene for the S. *diclina* delta-6 desaturase was removed from pRSP1 (Table 1) by digestion with EcoRI and EcoRV and cloned into the *Mfe*I/*Eco*RV site of pKR288. Vector pKR288 was an intermediate cloning vector containing a DNA stuffer fragment flanked by *Not*I/*Mfe*I sites at the 5' end and *Eco*RV/*Not*I sites at the 3' end of the fragment. The DNA stuffer fragment was amplified with Vent polymerase (NEB) from plasmid CalFad2-2 (described in WO 01/12800) using primer oCal-26 (SEQ ID NO:96), designed to introduce an *Mfe*I site at the 5' end of the fragment, and oCal-27 (SEQ ID NO:97), designed to introduce an EcoRV site at the 3' end of the fragment.
GCCAATTGGAGCGAGTTCCAATCTC (SEQ ID NO:96)
GCGATATCCGTTTGTTCTGACCTTCATG (SEQ ID NO:97),

The primers also introduced partial *Not*I sites at both ends of the fragment such that subsequent cloning into a filled *Not*I site added *Not*I sites to the end.

### Construction of plasmid pKR364

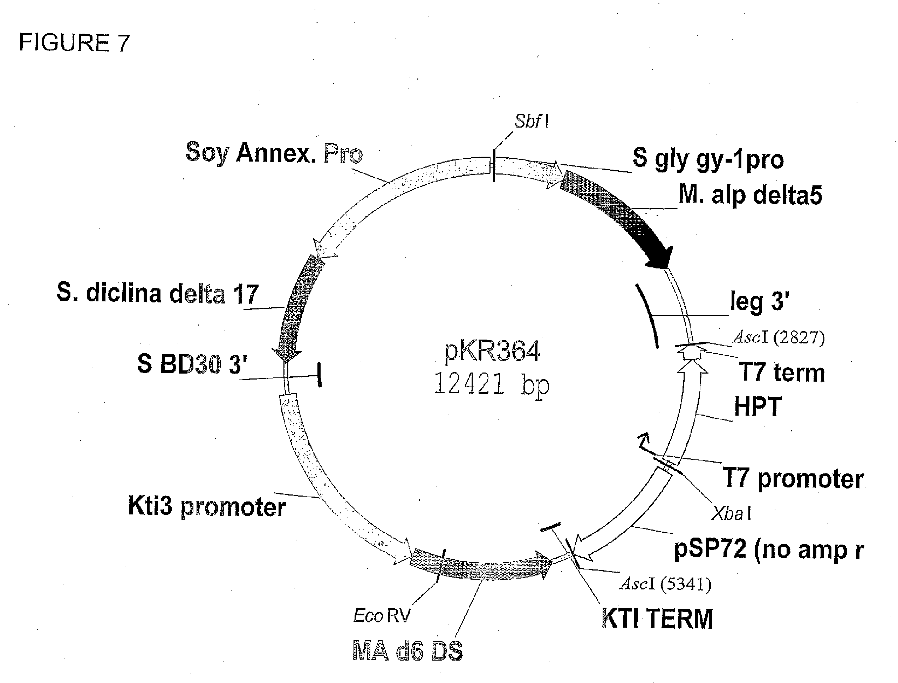

The *M.alpina* delta-6 desaturase, *M. alpina* delta-5 desaturase and *S. diclina* delta-17 desaturase were cloned into plasmid pKR364 behind strong, seed-specific promoters allowing for high expression of these genes in somatic soybean embryos and soybean seeds. Plasmid pKR364 is identical to pKR365 except that the *Not*I fragment that contains the S. *diclina* delta-6 desaturase in pKR365 was replaced with the *Not*I fragment containing the *M. alpina* delta-6 desaturase as found in pKR274. A schematic representation of pKR364 is shown in Figure 7.

### Construction of plasmid pKR357

The *S. aggregatum* delta-4 desaturase, *M. alpina* elongase and *Pavlova* elongase (Table1) were cloned into plasmid pKR357 behind strong, seed-specific promoters allowing for high expression of these genes in somatic soybean embryos and soybean seeds. The delta-4 desaturase (SEQ ID NO:51, and its protein translation product shown in SEQ ID NO:52) was cloned behind the KTi promoter followed by the KTi 3' termination region (Kti/Sad4/Kti3' cassette). The *Pavlova* elongase (SEQ ID NO:49) was cloned behind the GlycininGy1 promoter followed by the pea leguminA2 3' termination region (Gy1/Pavelo/legA2 cassette). The *M. alpina* elongase was cloned behind the promoter for the α'-subunit of β-conglycinin followed by the 3' transcription termination region of the phaseolin gene (βcon/Maelo/Phas3' cassette). Plasmid pKR357 also contains the T7prom/HPT/T7term cassette for bacterial selection of the plasmid on hygromycin B, a 35S/hpt/NOS3' cassette for selection in soy and a bacterial origin of replication (ori).

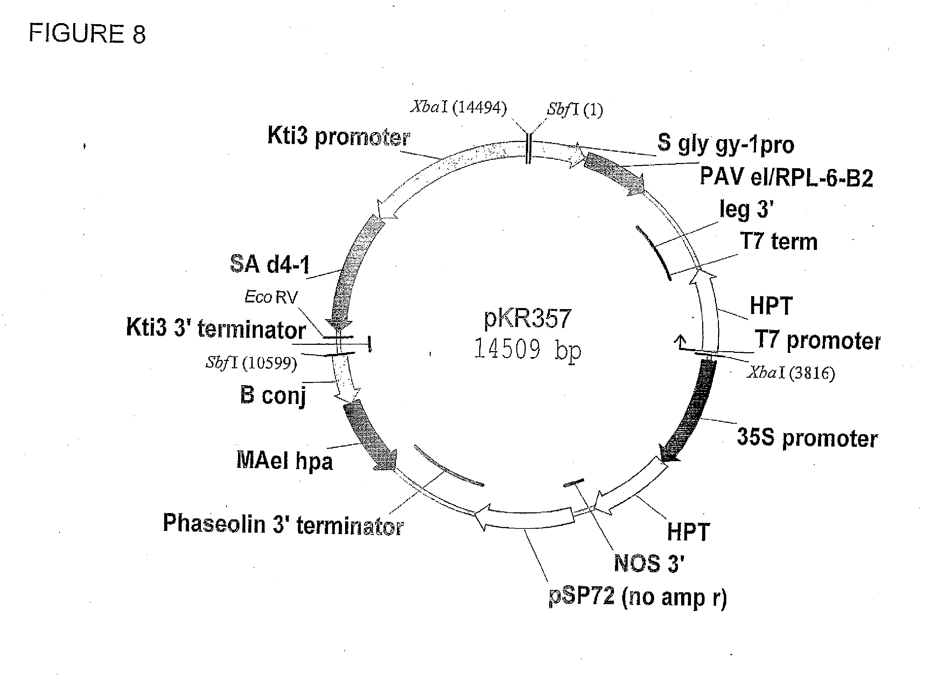

Plasmid pKR357 was constructed from a number of different intermediate cloning vectors as follows: The Gy1/Pavelo/legA2 cassette was released from plasmid pKR336 by digestion with *Pst*I and *Bsi*WI. The Gy1/Pavelo/legA2 cassette was then cloned into the *Sbf*I/*Bsi*WI site of plasmid pKR324, containing the βcon/Maelo/Phas3' cassette, the T7prom/hpt/T7term cassette, the 35S/hpt/Nos3' cassette and the bacterial *ori* to give pKR342. The KTi/Sad4/KTi3' cassette, released from pKR348 by digestion with *Pst*I*,* was then cloned into the *Sbf*I site of pKR342 to give pKR357. A schematic representation of pKR357 is shown in Figure 8. A detailed description for plasmid construction for pKR336, pKR324 and pKR348 is provided below.

Plasmid pKR336 was constructed by digesting pKR335 with *Not*I*,* to release the *Pavlova* elongase, and cloning this fragment into the *Not*I site of pKR263 (described in Example 4), which contained the Gy1/*NotI*/legA2 cassette. To construct pKR335, pRPL-6-B2 (described in Table 1) was digested with *Pst*I and the 3' overhang removed by treatment with VENT polymerase (NEB). The plasmid was then digested with *Eco*RI to fully release the *Pavlova* elongase as an *Eco*RI/*Pst*I blunt fragment. This fragment was cloned into the *Mfe*I/*Eco*RV site of intermediate cloning vector pKR333 to give pKR335. Vector pKR333 was identical to pKR288 (Example 3 and 13) in that it contained the same *Mfe*I and *Eco*RV sites falnked by *Not*I sites and was generated in a similar way as pKR288.

Plasmid pKR324 was constructed by cloning the *Not*I fragment of pKS134 (described in Example 3), containing the *M. alpina* elongase, into the *Not*I site of the βcon/*Not*I/Phas3' cassette of vector pKR72 (described in Example 4).

Plasmid pKR348 was constructed by cloning the *Not*I fragment of pKR300, containing the *S. aggregatum* delta-4 desaturase, into the *Not*I site of the KTi/*Not*I/KTi3' cassette in pKR123R. To construct pKR300, the gene for the delta-4 desaturase was removed from pRSA1 (Table 1) by digestion with EcoRI and EcoRV and cloned into the *Mfe*I/*Eco*RV site of pKR288 (described in Example 3 and 13). Plasmid pKR123R contains a *Not*I site flanked by the KTi promoter and the KTi transcription termination region (KTi/*Not*I/KTi3' cassette). In addition, the KTi/*Not*I/KTi3' cassette was flanked by Pstl sites. The KTi/*Not*I/KTi3' cassette was amplified from pKS126 (described in Example 2) using primers oKTi5 (SEQ ID NO:23) and oKTi7 (SEQ ID NO:98) designed to introduce an *Xba*I and *Bsi*WI site at the 5' end, and a *Pst*I/*Sbf*I and *Xba*I site at the 3' end, of the cassette.
TTCTAGACCTGCAGGATATAATGAGCCG (SEQ ID NO:98)

The resulting PCR fragment was subcloned into the *Xba*I site of the cloning vector pUC19 to give plasmid pKR123R with the KTi/*Not*I/KTi3' cassette flanked by *Pst*I sites.

### Production of DHA in somatic embryos

Plasmids pKR357, pKR365 and pKR364 were prepared as described in Example 9. Fragments of pKR365 and pKR364 were also obtained and purified as described for pKR274, pKR275 and pKKE2 in Example 9. Plasmids pKR357and either pKR365 or pKR364 were cotransformed into soybean embryogenic suspension cultures (cv. Jack) as described in Example 9. Hygromycin-resistant embryos containing pKR365 and pKR357, or pKR364 and pKR357 were selected and clonally propagated also as described in Example 9. Embryos were matured by culture for 4-6 weeks at 26°C in SB196 under cool white fluorescent (Phillips cool white Econowatt F40/CW/RS/EW) and Agro (Phillips F40 Agro) bulbs (40 watt) on a 16:8 hr photoperiod with light intensity of 90-120 µE/m2s. After this time embryo clusters were removed to a solid agar media, SB166, for 1-2 weeks. Clusters were then subcultured to medium SB103 for 3 weeks. During this period, individual embryos were removed from the clusters and screened for alterations in their fatty acid compositions as follows.

Fatty acid methyl esters were prepared from single, matured, somatic soy embryos by transesterification as described in Example 10. Retention times were compared to those for methyl esters of standards commercially available (Nu-Chek Prep, Inc. catalog #U-99-A). Six embryos from each event were analyzed in this way. Fatty acid methyl esters from embryos transformed with pKR357 and pKR365 containing the highest levels of DHA are shown in Table 9.

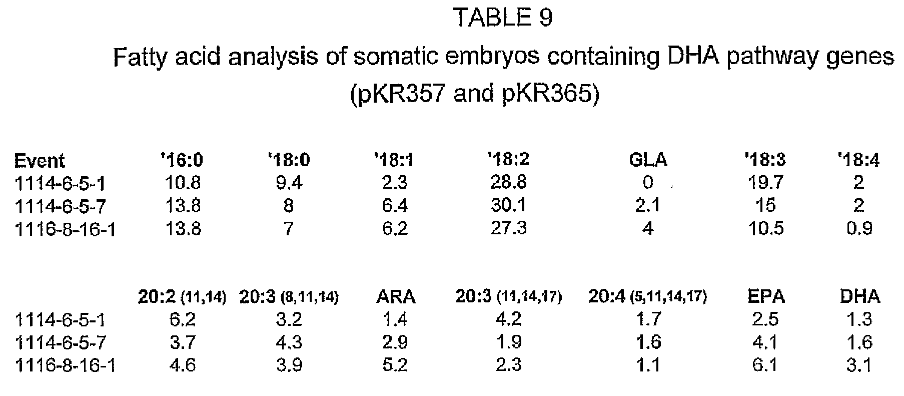

In addition to those fatty acids shown, 20:0, 20:1, 20:3 (5,11,14), DPA and ETA are also present in the extracts, each less than 1% of total fatty acids.

DHA is defined as 22:6(4,7,10,13,16,19) by the nomenclature described in Example 11.

Fatty acid methyl esters for embryos transformed with pKR357 and pKR364 containing the higest levels of DHA are shown in Table 10.

**TABLE 10**

| **Fatty acid analysis of somatic embryos containing DHA pathway genes (pKR357 & pKR364)** | | | | | | | | | | | | | | | | | |
|---|---|---|---|---|---|---|---|---|---|---|---|---|---|---|---|---|---|
| **Event** | **16:0** | **18:0** | **18:1** | **18:2** | **GLA** | **18:3** | **STA** | **20:2** | **HGLA** | **ARA** | **20:3** | **20:4(5,11,14,17)** | **ETA** | **EPA** | **DPA** | **DHA** | **Others** |
| 1141-4-2-1 | 17.4 | 2.8 | 1.8 | 41.2 | 0.0 | 33.7 | 2.7 | 0.0 | 0.0 | 0.0 | 0.0 | 0.0 | 0.0 | 0.0 | 0.0 | 0.0 | 0.2 |
| 1141-4-2-2 | 11.8 | 7.4 | 3.9 | 23.7 | 2.7 | 22.0 | 3.6 | 2.3 | 3.1 | 0.0 | 4.4 | 2.5 | 2.1 | 5.2 | 1.0 | 3.3 | 1.0 |
| 1141-4-2-3 | 16.6 | 5.5 | 4.8 | 26.3 | 3.0 | 23.7 | 3.1 | 1.4 | 2.6 | 0.3 | 3.1 | 1.3 | 2.8 | 3.8 | 0.0 | 1.4 | 0.4 |
| 1141-4-2-4 | 16.5 | 5.8 | 3.8 | 28.5 | 4.1 | 27.7 | 2.9 | 1.0 | 1.4 | 0.0 | 2.5 | 1.1 | 1.9 | 1.9 | 0.0 | 1.0 | 0.0 |
| 1141-4-2-5 | 15.3 | 3.6 | 3.3 | 27.3 | 3.4 | 28.9 | 3.2 | 0.8 | 2.3 | 0.0 | 2.8 | 0.9 | 2.6 | 4.0 | 0.0 | 1.6 | 0.0 |
| 1141-4-2-6 | 16.5 | 3.1 | 3.7 | 41.5 | 2.0 | 25.6 | 1.7 | 0.2 | 1.0 | 0.0 | 1.1 | 0.3 | 1.3 | 1.2 | 0.0 | 0.7 | 0.0 |
| 1141-5-2-1 | 14.1 | 3.9 | 4.7 | 24.1 | 7.4 | 26.2 | 1.8 | 1.1 | 3.7 | 1.8 | 1.1 | 0.7 | 0.7 | 6.5 | 0.0 | 2.2 | 0.0 |
| 1141-5-2-2 | 12.6 | 5.0 | 1.9 | 29.8 | 1.1 | 28.9 | 2.9 | 3.4 | 4.2 | 1.1 | 3.7 | 1.1 | 0.6 | 1.8 | 0.0 | 2.0 | 0.0 |
| 1141-5-2-3 | 10.8 | 3.5 | 7.8 | 34.5 | 5.0 | 22.9 | 1.1 | 2.2 | 2.4 | 0.8 | 2.0 | 1.7 | 0.0 | 3.4 | 0.0 | 1.8 | 0.0 |
| 1141-5-2-4 | 12.0 | 3.8 | 3.8 | 30.9 | 3.5 | 27.1 | 1.5 | 2.3 | 4.1 | 1.3 | 2.4 | 1.0 | 0.0 | 3.7 | 0.0 | 2.6 | 0.0 |
| 1141-5-2-5 | 11.2 | 3.8 | 8.4 | 33.9 | 6.1 | 19.4 | 0.0 | 2.1 | 2.0 | 0.7 | 2.0 | 1.7 | 0.6 | 5.7 | 0.0 | 2.1 | 0.3 |
| 1141-5-2-6 | 14.1 | 7.4 | 3.9 | 28.8 | 2.2 | 20.2 | 2.4 | 3.7 | 5.7 | 1.5 | 2.7 | 1.0 | 0.0 | 3.0 | 0.0 | 2.1 | 1.3 |
| 1142-9-4-1 | 13.6 | 2.7 | 5.7 | 39.7 | 4.1 | 18.1 | 0.0 | 1.5 | 2.0 | 0.8 | 1.3 | 1.8 | 0.6 | 6.1 | 0.0 | 1.8 | 0.0 |
| 1142-9-4-2 | 13.8 | 3.9 | 8.2 | 35.7 | 3.2 | 18.3 | 1.0 | 2.1 | 1.7 | 0.7 | 2.0 | 1.7 | 0.6 | 4.3 | 0.3 | 1.4 | 0.8 |
| 1142-9-4-3 | 15.4 | 5.2 | 6.6 | 31.0 | 5.0 | 14.7 | 1.1 | 1.8 | 2.9 | 0.6 | 2.1 | 2.5 | 0.8 | 7.6 | 0.0 | 1.9 | 0.5 |
| 1142-9-4-4 | 14.4 | 3.4 | 6.4 | 37.8 | 4.5 | 18.2 | 0.9 | 1.4 | 2.5 | 0.7 | 1.4 | 1.3 | 0.6 | 4.4 | 0.0 | 1.2 | 0.8 |
| 1142-9-4-5 | 13.5 | 3.4 | 3.7 | 35.8 | 4.1 | 24.0 | 1.3 | 1.3 | 1.6 | 0.4 | 1.9 | 2.3 | 0 8 | 4.7 | 0.0 | 1.3 | 0.0 |
| 1142-9-4-6 | 12.9 | 3.6 | 7.6 | 37.6 | 2.4 | 18.7 | 0.0 | 2.1 | 0.9 | 0.6 | 2.3 | 2.4 | 0.6 | 5.5 | 0.0 | 2.5 | 0.3 |
| 1142-10-6-1 | 9.7 | 5.1 | 6.1 | 41.7 | 2.2 | 16.7 | 0.5 | 4.4 | 1.7 | 0.2 | 3.3 | 3.4 | 0 4 | 1.8 | 0.4 | 0.8 | 1.7 |
| 1142-10-6-2 | 11.4 | 3.1 | 6.5 | 39.3 | 4.3 | 21.4 | 0.0 | 1.2 | 0.8 | 0.0 | 2.4 | 3.4 | 0.0 | 4.9 | 0.0 | 1.1 | 0.0 |
| 1142-10-6-3 | 15.5 | 3.1 | 7.5 | 46.6 | 1.3 | 19.2 | 0.4 | 0.8 | 0.5 | 0.0 | 2.0 | 1.1 | 0.6 | 1.0 | 0.0 | 0.0 | 0.3 |
| 1142-10-6-4 | 11.8 | 4.1 | 8.0 | 38.8 | 3.0 | 17.2 | 0.0 | 2.2 | 1.3 | 0.0 | 2.9 | 5.2 | 0.8 | 3.6 | 0.0 | 1.1 | 0.0 |
| 1142-10-6-5 | 12.1 | 4.5 | 7.1 | 34.6 | 2.5 | 21.5 | 1.5 | 1.8 | 1.9 | 0.0 | 3.4 | 2.2 | 2.0 | 2.8 | 0.5 | 1.4 | 0.3 |
| 1142-10-6-6 | 11.7 | 3.0 | 6.2 | 39.2 | 4.3 | 20.9 | 1.0 | 1.5 | 1.6 | 0.0 | 2.5 | 3.1 | 1.3 | 2.9 | 0.0 | 0.9 | 0.0 |
| 1142-10-8-1 | 14.6 | 6.5 | 5.4 | 26.4 | 8.7 | 11.1 | 1.4 | 4.3 | 3.3 | 2.5 | 1.9 | 1.6 | 0.8 | 6.1 | 0.5 | 2.6 | 2.3 |
| 1142-10-8-2 | 14.3 | 3.3 | 3.9 | 28.4 | 4.0 | 28.2 | 1.7 | 1.0 | 2.3 | 0.2 | 2.5 | 1.3 | 2.6 | 4.6 | 0.4 | 1.3 | 0.0 |
| 1142-10-8-3 | 16.7 | 3.7 | 15.2 | 13.8 | 27.9 | 10.6 | 1.7 | 0.4 | 3.3 | 0.4 | 0.3 | 0.0 | 1.6 | 2.9 | 0.0 | 0.4 | 1.2 |
| 1142-10-8-4 | 20.5 | 4.2 | 10.0 | 12.1 | 21.8 | 12.0 | 2.6 | 0.4 | 6.4 | 1.0 | 0.5 | 0.0 | 2.4 | 4.3 | 0.3 | 0.6 | 1.1 |
| 1142-10-8-5' | 13.4 | 5.1 | 3.9 | 31.5 | 2.2 | 24.1 | 2.1 | 2.5 | 2.5 | 0.0 | 4.5 | 1.5 | 2 3 | 2.3 | 0.4 | 1.2 | 0.5 |
| 1142-10-8-6 | 11.2 | 3.9 | 17.0 | 21.0 | 15.3 | 13.0 | 0.0 | 2.4 | 2.6 | 2.1 | 1.1 | 1.3 | 0.9 | 4.8 | 0.0 | 1.3 | 2.1 |

For Table 10, fatty acids listed as "others" include,: 20:0, 20:1(11), 20:3 (5,11,14) and 22:0. Each of these fatty acids is present at relative abundance of less than 1% of the total fatty acids.

### Further embodiments of the invention

1. An oilseed plant that produces mature seeds in which the total seed fatty acid profile comprises at least 1.0 % of at least one polyunsaturated fatty acid having at least twenty carbon atoms and five or more carbon-carbon double bonds.
2. An oilseed plant that produces mature seeds in which the total seed fatty acid profile comprises at least 5.0 % of at least one polyunsaturated fatty acid having at least twenty carbon atoms and five or more carbon-carbon double bonds.
3. An oilseed plant that produces mature seeds in which the total seed fatty acid profile comprises at least 10.0 % of at least one polyunsaturated fatty acid having at least twenty carbon atoms and five or more carbon-carbon double bonds.
4. An oilseed plant that produces mature seeds in which the total seed fatty acid profile comprises at least 15.0 % of at least one polyunsaturated fatty acid having at least twenty carbon atoms and five or more carbon-carbon double bonds.
5. An oilseed plant that produces mature seeds in which the total seed fatty acid profile comprises at least 20.0 % of at least one polyunsaturated fatty acid having at least twenty carbon atoms and five or more carbon-carbon double bonds.
6. An oilseed plant that produces mature seeds in which the total seed fatty acid profile comprises at least 25.0 % of at least one polyunsaturated fatty acid having at least twenty carbon atoms and five or more carbon-carbon double bonds.
7. An oilseed plant that produces mature seeds in which the total seed fatty acid profile comprises at least 30.0 % of at least one polyunsaturated fatty acid having at least twenty carbon atoms and five or more carbon-carbon double bonds.
8. An oilseed plant that produces mature seeds in which the total seed fatty 5 acid profile comprises at least 40.0 % of at least one polyunsaturated fatty acid having at least twenty carbon atoms and five or more carbon-carbon double bonds.
9. An oilseed plant that produces mature seeds in which the total seed fatty acid profile comprises at least 50.0 % of at least one polyunsaturated fatty acid having at least twenty carbon atoms and five or more carbon-carbon double bonds.
10. An oilseed plant that produces mature seeds in which the total seed fatty acid profile comprises at least 60.0 % of at least one polyunsaturated fatty acid having at least twenty carbon atoms and five or more carbon-carbon double bonds.
11. The oilseed plant of any of Embodiments 1-10 wherein the polyunsaturated fatty acid is an omega-3 fatty acid.
12. The oilseed plant of any of Embodiments 1-10 wherein the polyunsaturated fatty acid is an omega-3 fatty acid selected from the group consisting of EPA, DPA, and DHA.
13. The oilseed plant of any of Embodiments 3-10 wherein the total seed fatty acid profile further comprises less than 2.0% arachidonic acid.
14. The oilseed plant of any of Embodiments 3-10 wherein the polyunsaturated fatty acid is an omega-3 fatty acid and the total seed fatty acid profile further comprises less than 2.0% arachidonic acid.
15. The oilseed plant of any of Embodiments 3-10 wherein the polyunsaturated fatty acid is an omega-3 fatty acid selected from the group consisting of EPA, DPA, and DHA, and the total seed fatty acid profile further comprises less than 2.0% arachidonic acid.
16. Seeds obtained from the plant of any of Embodiments 1-10.
17. Seeds obtained from the plant of any of Embodiments 1 -10 wherein the polyunsaturated fatty acid is an omega-3 fatty acid.
18. Seeds obtained from the plant of any of Embodiments 1-10 wherein the polyunsaturated fatty acid is an omega-3 fatty acid selected from the group consisting of EPA, DPA, and DHA.
19. Seeds obtained from the plant of any of Embodiments 3-10 wherein the polyunsaturated fatty acid is an omega-3 fatty acid and the total seed fatty acid profile further comprises less than 2.0% arachidonic acid.
20. Seeds obtained from the plant of any of Embodiments 3-10 wherein the polyunsaturated fatty acid is an omega-3 fatty acid selected from the group consisting of EPA, DPA, and DHA, and the total seed fatty acid profile further comprises less than 2.0% arachidonic acid.
21. Oil obtained from the seeds of the plants of any of Embodiments 1-10.
22. Oil obtained from the seeds of the plants of any of Embodiments 1-10 wherein the polyunsaturated fatty acid an omega-3 fatty acid.
23. Oil obtained from the seeds of the plants of any of Embodiments 1-10 wherein the polyunsaturated fatty acid is an omega-3 fatty acid selected from the group consisting of EPA, DPA, and DHA.
24. Oil obtained from the seeds of the plants of any of Embodiments 3-10 wherein the polyunsaturated fatty acid is an omega-3 fatty acid and the total seed fatty acid profile further comprises less than 2.0% arachidonic acid.
25. Oil obtained from the seeds of the plants of any of Embodiments 3-10 wherein the polyunsaturated fatty acid is an omega-3 fatty acid selected from the group consisting of EPA, DPA, and DHA, and the total seed fatty acid profile further comprises less than 2.0% arachidonic acid.
26. The plant of any of Embodiments 1-10 wherein the oilseed plant is selected from the group consisting of soybean, Brassica species, sunflower, maize, cotton, flax, and safflower.
27. The plant of any of Embodiments 1-10 wherein the oilseed plant is selected from the group consisting of soybean, Brassica species, sunflower, maize, cotton, flax, and safflower, and further wherein the polyunsaturated fatty acid is an omega-3 fatty acid.
28. The plant of any of Embodiments 1 -10 wherein the oilseed plant is selected from the group consisting of soybean, Brassica species, sunflower, maize, cotton, flax, and safflower, and further wherein the polyunsaturated fatty acid is an omega-3 fatty acid selected from the group consisting of EPA, DPA, and DHA.
29. The plant of any of Embodiments 3-10 wherein the oilseed plant is selected from the group consisting of soybean, Brassica species, sunflower, maize, cotton, flax, and safflower, and further wherein the polyunsaturated fatty acid is an omega-3 fatty acid and the total seed fatty acid profile further comprises less than 2.0% arachidonic acid.
30. The plant of any of Embodiments 3-10 wherein the oilseed plant is selected from the group consisting of soybean, Brassica species, sunflower, maize, cotton, flax, and safflower, and further wherein the polyunsaturated fatty acid is an omega-3 fatty acid selected from the group consisting of EPA, DPA, and DHA, and the total seed fatty acid profile further comprises less than 2.0% arachidonic acid.
31. A recombinant construct for altering the total fatty acid profile of mature seeds of an oilseed plant, said construct comprising at least two promoters wherein each promoter is operably linked to a nucleic acid sequence encoding a polypeptide required for making at least one polyunsaturated fatty acid having at least twenty carbon atoms and four or more carbon-carbon double bonds and further wherein the total fatty acid profile comprises at least 2% of at least one polyunsaturated fatty acid having at least twenty carbon atoms and four or more carbon-carbon double bonds and further wherein said polypeptide is an enzyme selected from the group consisting of a Δ4 desaturase, a Δ5 desaturase, Δ6 desaturase, a Δ15 desaturase, a Δ17 desaturase, a C18 to C22 elongase and a C20 to C24 elongase.
32. The recombinant construct of Embodiment 31 wherein the promoter is selected from the group consisting of the alpha prime subunit of beta conglycinin promoter, Kunitz trypsin inhibitor 3 promoter, annexin promoter, Gly1 promoter, beta subunit of beta conglycinin promoter, P34/Gly Bd m 30K promoter, albumin promoter, Leg A1 promoter and Leg A2 promoter.
33. An oilseed plant comprising in its genome the recombinant construct of Embodiment 31.
34. An oilseed plant comprising in its genome the recombinant construct of Embodiment 32.
35. The oilseed plant of Embodiment 33 wherein the oilseed plant is selected from the group consisting of soybean, Brassica species, sunflower, maize, cotton, flax, safflower.
36. The oilseed plant of Embodiment 34 wherein the oilseed plant is selected from the group consisting of soybean, Brassica species, sunflower, maize, cotton, flax, safflower.
37. Seeds obtained from the plant of Embodiment 33.
38. Seeds obtained from the plant of Embodiment 34.
39. Seeds obtained from the plant of Embodiment 35.
40. Seeds obtained from the plant of Embodiment 36.
41. Oil obtained from the seeds of Embodiment 37.
42. Oil obtained from the seeds of Embodiment 38.
43. Oil obtained from the seeds of Embodiment 49.
44. Oil obtained from the seeds of Embodiment 40.
45. A method for making an oilseed plant having an altered fatty acid profile which comprises:
   a) transforming a plant with the recombinant construct of Embodiment 31;
   b) growing the transformed plant of step (a); and
   c) selecting those plants wherein the total fatty acid profile comprises at least 1.0 % of at least one polyunsaturated fatty acid having at least twenty carbon atoms and five or more carbon-carbon double bonds.
46. An oilseed plant made by the method of Embodiment 30.
47. Seeds obtained from the plant of Embodiment 31.
48. Oil obtained from the seeds of Embodiment 32.
49. A method for making an oilseed plant having an altered fatty acid profile which comprises:
   a) transforming a plant with the recombinant construct of Embodiment 32,
   b) growing the transformed plant of step (a); and
   c) selecting those plants wherein the total fatty acid profile comprises at least 1.0 % of at least one polyunsaturated fatty acid having at least twenty carbon atoms and five or more carbon-carbon double bonds.
50. An oilseed plant made by the method of Embodiment 34.
51. Seeds obtained from the plant of Embodiment 35.
52. Oil obtained from the seeds of Embodiment 36.
53. A food product or food analog which has incorporated therein the oil of Embodiment 21.
54. A food product or food analog which has incorporated therein the oil of Embodiment 41.
55. A food product or food analog which has incorporated therein the oil of Embodiment 42.
56. A food product or food analog which has incorporated therein the oil of Embodiment 43.
57. A food product or food analog which has incorporated therein the oil of Embodiment 44.
58. A food product or food analog which has incorporated therein the oil of Embodiment 48.
59. A food product or food analog which has incorporated therein the oil of Embodiment 52.
60. The food product of Embodiment 53 wherein said product is selected from the group consisting of a spray-dried food particle, a freeze-dried food particle, meat products, a cereal food, a snack food, a baked good, an extruded food, a fried food, a health food, a dairy food, meat analogs, cheese analogs, milk analogs, a pet food, animal feed or aquaculture feed.
61. The food product of Embodiment 54 wherein said product is selected from the group consisting of a spray-dried food particle, a freeze-dried food particle, meat products, a cereal food, a snack food, a baked good, an extruded food, a fried food, a health food, a dairy food, meat analogs, cheese analogs, milk analogs, a pet food, animal feed or aquaculture feed.
62. The food product of Embodiment 55 wherein said product is selected from the group consisting of a spray-dried food particle, a freeze-dried food particle, meat products, a cereal food, a snack food, a baked good, an extruded food, a fried food, a health food, a dairy food, meat analogs, cheese analogs, milk analogs, a pet food, animal feed or aquaculture feed.
63. The food product of Embodiment 56 wherein said product is selected from the group consisting of a spray-dried food particle, a freeze-dried food particle, meat products, a cereal food, a snack food, a baked good, an extruded food, a fried food, a health food, a dairy food, meat analogs, cheese analogs, milk analogs, a pet food, animal feed or aquaculture feed.
64. The food product of Embodiment 57 wherein said product is selected from the group consisting of a spray-dried food particle, a freeze-dried food particle, meat products, a cereal food, a snack food, a baked good, an extruded food, a fried food, a health food, a dairy food, meat analogs, cheese analogs, milk analogs, a pet food, animal feed or aquaculture feed.
65. The food product of Embodiment 58 wherein said product is selected from the group consisting of a spray-dried food particle, a freeze-dried food particle, meat products, a cereal food, a snack food, a baked good, an extruded food, a fried food, a health food, a dairy food, meat analogs, cheese analogs, milk analogs, a pet food, animal feed or aquaculture feed.
66. The food product of Embodiment 59 wherein said product is selected from the group consisting of a spray-dried food particle, a freeze-dried food particle, meat products, a cereal food, a snack food, a baked good, an extruded food, a fried food, a health food, a dairy food, meat analogs, cheese analogs, milk analogs, a pet food, animal feed or aquaculture feed.
67. A beverage which has incorporated therein the oil of Embodiment 21.
68. A beverage which has incorporated therein the oil of Embodiment 41.
69. A beverage which has incorporated therein the oil of Embodiment 42.
70. A beverage which has incorporated therein the oil of Embodiment 43.
71. A beverage which has incorporated therein the oil of Embodiment 44.
72. A beverage which has incorporated therein the oil of Embodiment 48.
73. A beverage which has incorporated therein the oil of Embodiment 52.
74. Infant formula incorporated therein the oil of Embodiment 21.
75. Infant formula incorporated therein the oil of Embodiment 41.
76. Infant formula incorporated therein the oil of Embodiment 42.
77. Infant formula incorporated therein the oil of Embodiment 43.
78. Infant formula incorporated therein the oil of Embodiment 44.
79. Infant formula incorporated therein the oil of Embodiment 48.
80. Infant formula incorporated therein the oil of Embodiment 52.
81. A nutritional supplement which has incorporated therein the oil of Embodiment 21.
82. A nutritional supplement which has incorporated therein the oil of Embodiment 41.
83. A nutritional supplement which has incorporated therein the oil of Embodiment 42.
84. A nutritional supplement which has incorporated therein the oil of Embodiment 43.
85. A nutritional supplement which has incorporated therein the oil of Embodiment 44.
86. A nutritional supplement which has incorporated therein the oil of Embodiment 48.
87. A nutritional supplement which has incorporated therein the oil of Embodiment 52.
88. A food product or food analog which has incorporated therein the seed of Embodiment 13
89. A food product or food analog which has incorporated therein the seed of Embodiment 37
90. A food product or food analog which has incorporated therein the seed of Embodiment 38
91. A food product or food analog which has incorporated therein the seed of Embodiment 39
92. A food product or food analog which has incorporated therein the seed of Embodiment 40.
93. A pet food which has incorporated therein the seed of Embodiment 16.
94. A pet food which has incorporated therein the seed of Embodiment 37.
95. A pet food which has incorporated therein the seed of Embodiment 38.
96. A pet food which has incorporated therein the seed of Embodiment 39.
97. A pet food which has incorporated therein the seed of Embodiment 40.
98. Animal feed which has incorporated therein the seed of Embodiment 16.
99. Animal feed which has incorporated therein the seed of Embodiment 37.
100. Animal feed which has incorporated therein the seed of Embodiment 38.
101. Animal feed which has incorporated therein the seed of Embodiment 39.
102. Animal feed which has incorporated therein the seed of Embodiment 40.
103. A pet food incorporated therein the oil of Embodiment 21.
104. A pet food incorporated therein the oil of Embodiment 41.
105. A pet food incorporated therein the oil of Embodiment 42.
106. A pet food incorporated therein the oil of Embodiment 43.
107. A pet food incorporated therein the oil of Embodiment 44.
108. A pet food incorporated therein the oil of Embodiment 48.
109. A pet food incorporated therein the oil of Embodiment 52.
110. Animal feed which has incorporated therein the oil of Embodiment 21.
111. Animal feed which has incorporated therein the oil of Embodiment 41.
112. Animal feed which has incorporated therein the oil of Embodiment 42.
113. Animal feed which has incorporated therein the oil of Embodiment 43.
114. Animal feed which has incorporated therein the oil of Embodiment 44.
115. Animal feed which has incorporated therein the oil of Embodiment 48.
116. Animal feed which has incorporated therein the oil of Embodiment 52.
117. A whole bean soy product made from the seed of Embodiment 16.
118. A whole bean soy product made from the seed of Embodiment 37.
119. A whole bean soy product made from the seed of Embodiment 38.
120. A whole bean soy product made from the seed of Embodiment 39.
121. A whole bean soy product made from the seed of Embodiment 40.
122. An aquaculture food product which has incorporated therein the oil of Embodiment 21.
123. An aquaculture food product which has incorporated therein the oil of Embodiment 41.
124. An aquaculture food product which has incorporated therein the oil of Embodiment 42.
126. An aquaculture food product which has incorporated therein the oil of Embodiment 43.
126. An aquaculture food product which has incorporated therein the oil of Embodiment 44.
127. An aquaculture food product which has incorporated therein the oil of Embodiment 48.
128. An aquaculture food product which has incorporated therein the oil of Embodiment 62.
129. An aquaculture food product which has incorporated therein the seed of Embodiment 16.
130. An aquaculture food product which has incorporated therein the seed of Embodiment 37.
131. An aquaculture food product which has incorporated therein the seed of Embodiment 38.
132. An aquaculture food product which has incorporated therein the seed of Embodiment 39.
133. An aquaculture food product which has incorporated therein the seed of Embodiment 40.
134. An oilseed plant that produces mature seeds in which the total seed fatty acid profile comprises polyunsaturated fatty acids having at least twenty carbon atoms and five or more carbon-carbon double bonds wherein the ratio of EPA:DHA is in the range from 1:100 to 860:100.
135. An oilseed plant that produces mature seeds in which the total seed fatty acid profile comprises polyunsaturated fatty acids having at least twenty carbon atoms and five or more. carbon-carbon double bonds wherein the ratio of EPA:DHA is in the range from 1:100 to 860:100 and further wherein the total seed fatty acid profile further comprises less than 2.0% arachidonic acid.
136. An oilseed plant that produces mature seeds in which the total seed fatty acid profile comprises polyunsaturated fatty acids having at least twenty carbon atoms and five or more carbon-carbon double bonds wherein the ratio of DHA:EPA is in the range from 1 :100 to 110:100.
137. An oilseed plant that produces mature seeds in which the total seed fatty acid profile comprises polyunsaturated fatty acids having at least twenty carbon atoms and five or more carbon-carbon double bonds wherein the ratio of DHA: EPA is in the range from 1:100 to 110:100 and further wherein the total seed fatty acid profile further comprises less than 2.0% arachidonic acid.
138. Seeds obtained from the plant of any of Embodiments 134-137. 139. Oil obtained from the seeds of the plants of any of Embodiments 134-137.

## Claims

1. Oil obtained from the seeds of a transgenic oilseed plant that produces mature seeds in which the total seed fatty acid profile comprises at least 2% of at least one polyunsaturated fatty acid having at least 20 carbon atoms and four or more double bonds, wherein the transgenic oilseed plant is selected from the group consisting of soybean, Brassica species, sunflower, maize, cotton, flax and safflower and comprises a recombinant construct comprising at least two promoters wherein each promoter is operably linked to a nucleic acid sequence encoding an enzyme selected from the group consisting of a Δ4 desaturase, a Δ5 desaturase, a Δ6 desaturase, a Δ15 desaturase, a Δ17 desaturase, a C18 to C22 elongase and a C20 to C24 elongase.

2. The oil of Claim 1 wherein the polyunsaturated fatty acid is arachidonic acid.

3. The oil of Claim 1 or 2 wherein the transgenic oilseed plant comprises a recombinant construct encoding a Δ5 desaturase, a Δ6 desaturase, and an elongase.

4. The oil of any one of Claims 1 to 3 wherein the promoter is selected from the group consisting of the alpha prime subunit of beta conglycinin promoter, Kunitz trypsin inhibitor 3 promoter, annexin promoter, Gly1 promoter, beta subunit of beta conglycinin promoter, P34/Gly Bd m 30K promoter, albumin promoter, Leg A1 promoter and Leg A2 promoter.

5. Use of the oil as defined in claims 1 to 4 in the production of a nutritional supplement, food products or food analogs, infant formula, animal feed, pet feed, aquaculture food product or a beverage.

6. The use of claim 5 wherein the food product or food analog is selected from the group consisting of a meat product, a cereal food, a snack food, a baked good, a fried food, an extruded cereal food product, a health food, a dairy food, meat analogs, cheese analogs, and milk analogs.

7. A food product or food analog which has incorporated therein the oil of any one of Claims 1 to 4.

8. The food product of Claim 7, wherein said product is selected from the group consisting of meat products, a cereal food, a snack food, a baked good, an extruded cereal food product , a fried food, a health food, a dairy food, meat analogs, cheese analogs, milk analogs, a pet food, animal feed or aquaculture feed.

9. The food product of Claim 7, wherein said product is a beverage, infant formula or a nutritional supplement.
